# EUROPEAN PATENT SPECIFICATION

(11) **EP 4 291 000 B1**
(45) Date of publication and mention of the grant of the patent: **04.02.2026**
(21) Application number: 22897190.9
(22) Date of filing: 22.07.2022
(51) Int. Cl.: C09K 11/06, C07D 471/06, C07F 5/02, C07F 7/08, H10K 85/60

(54) **ORGANIC ELECTROLUMINESCENT DEVICE AND DISPLAY APPARATUS**
ORGANISCHE ELEKTROLUMINESZENTE VORRICHTUNG UND ANZEIGEVORRICHTUNG
DISPOSITIF ÉLECTROLUMINESCENT ORGANIQUE ET DISPOSITIF D'AFFICHAGE

(30) Priority: 26.11.2021 CN 202111423658
(43) Date of publication of application: 13.12.2023
(73) Proprietor: Kunshan Go-Visionox Opto-Electronics Co., Ltd., KunShan, Jiangsu 215300 (CN); Tsinghua University, Beijing 100084 (CN)
(72) Inventor: LI, Guomeng, Kunshan, Jiangsu 215300 (CN); DUAN, Lian, Kunshan, Jiangsu 215300 (CN); ZHANG, Yuewei, Kunshan, Jiangsu 215300 (CN); ZHANG, Dongdong, Kunshan, Jiangsu 215300 (CN); CAI, Minghan, Kunshan, Jiangsu 215300 (CN); LI, Baoyu, Kunshan, Jiangsu 215300 (CN); LI, Mengzhen, Kunshan, Jiangsu 215300 (CN)
(74) Representative: Arnold & Siedsma
(86) International application number: PCT/CN2022/107504
(87) International publication number: WO 2023/093094

(56) References cited:
- EP-A1- 3 876 296
- WO-A1-2019/164340
- WO-A1-2021/210501
- CN-A- 111 440 122
- CN-A- 111 463 352
- CN-A- 111 755 615
- CN-A- 112 898 322
- CN-A- 112 996 796
- CN-A- 114 171 692
- JIANG PENGCHENG ET AL: "Quenching-Resistant Multiresonance TADF Emitter Realizes 40% External Quantum Efficiency in Narrowband Electroluminescence at High Doping Level", ADVANCED MATERIALS, vol. 34, no. 3, 21 November 2021 (2021-11-21), DE, XP093192774, ISSN: 0935-9648, DOI: 10.1002/adma.202106954

## Description

### TECHNICAL FIELD

The present application relates to an organic electroluminescent device and a display apparatus, belonging to the field of organic electroluminescent technologies.

### BACKGROUND

Organic Light Emitting Diode (OLED) is a device that achieves a purpose of emitting light by current driving. Its main characteristics are derived from an organic light-emitting layer. When applying an appropriate voltage, electrons and holes are combined in the organic light-emitting layer to produce excitons and emit light with different wavelengths based on the characteristics of the organic light-emitting layer.

Currently, the light-emitting layer is composed of a host material and a dye, and the dye is mostly selected from a traditional fluorescent material and a traditional phosphorescent material. Among them, although the traditional phosphorescent material has high efficiency, it is expensive and has poor stability; while the traditional fluorescent material has extremely low efficiency although it is cheap. The existing display apparatuses still have problems of low efficiency, high driving voltage, etc.

In recent years, the Multi-Resonance (MR) material with the high efficiency and narrow-band emission has attracted widespread attention from the scientific and industrial communities. Although this type of material has a certain degree of promoting effect on the device performance relative to the traditional fluorescent material and the traditional phosphorescent material. However, this type of material is difficult to fully utilize the energy between the host and guest under low concentration conditions, and decreased efficiency and other issues will be caused by further increasing the concentration. The evaporation window is relatively narrow, and the process requirements are complex.

WO2021/210501A1 provides a compound represented by formula (I) shown in the specification, wherein X, Y, and Z are each independently a hydrogen atom or an (un)substituted nitrogen-containing heteroaryl group but Y and/or Z is an (un)substituted nitrogen-containing heteroaryl group.

CN111440122A relates to a thermally activated delayed fluorescence material and an organic light-emitting device. The thermally activated delayed fluorescence material comprises a compound represented by the general formula (I) shown in the specification, wherein R₀, R₁, R₂ and R₃ are each independently selected from any one of hydrogen, deuterium, halogen group, or a substituted or unsubstituted C6-C30 aryl group or C1-C20 aliphatic group; R is selected from any one of a substituted or unsubstituted C1-C10 aliphatic group, C6-C30 aryl group or C4-C30 heteroaryl group, and G is selected from any one of a direct bond, or a substituted or unsubstituted C6-C30 arylene group or C4-C30 heteroarylene group; and A is a group having an electron-deficient property.

WO2019/164340A1 relates to a heterocyclic compound represented by chemical Formula I shown in the specification, and an organic light-emitting device comprising same.

EP3876296A1 provides an organic light-emitting device including: a first electrode; a second electrode; and an organic layer between the first electrode and the second electrode and including an emission layer, wherein the emission layer may include a polycyclic compound represented by specific Formula and a host, and a content of the polycyclic compound may be smaller than a content of the host.

JIANG PENGCHENG ET AL ("Quenching-Resistant Multiresonance TADF Emitter Realizes 40% External Quantum Efficiency in Narrowband Electroluminescence at High Doping Level", ADVANCED MATERIALS, vo1.34, no.3, 21 November 2021 (2021-11-21), XP093192774, DE ISSN:0935-9648, DOI: 10.1002/adma.202106954) provides a highly emissive molecule with enhanced quenching resistance. The steric effect largely removes the formation of detrimental excimers / aggregates, and boosts the performance of the corresponding devices with a maximum external quantum efficiency (EQEₘₐₓ) up to 40.0% and full width at half maximum (FWHM) of 25 nm, representative of the only example of single OLED that can concurrently achieve narrow bandwidth and high EL efficiency surpassing 40% to date. Even at doping ratio of 30 wt%, the EQEₘₐₓ is retained to be 33.3% with nearly unchanged emission spectrum.

CN112898322A provides a compound and application thereof, and an organic electroluminescent device comprising the same, the compound has a structure of formula (I) shown in the specification, and the compound is used as a material of a luminescent layer in the organic electroluminescent device.

### SUMMARY

The present application is set out in the appended set of claims.

The present application provides an organic electroluminescent device and a display apparatus with high luminescence efficiency and high stability of driving voltage.

The present application provides an organic electroluminescent device including a light-emitting layer, the light-emitting layer includes a triplet-triplet annihilation type host and a fluorescent dye, where the fluorescent dye has a structure represented by the following Formula (1-2), (1-3), (1-6) or (2-3): 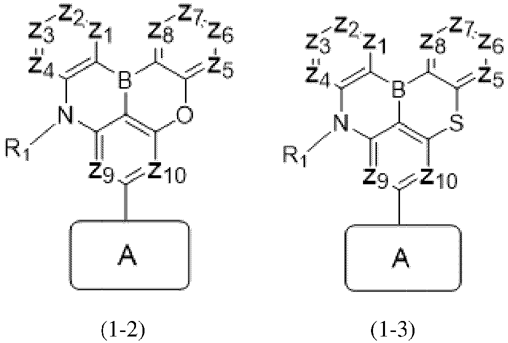 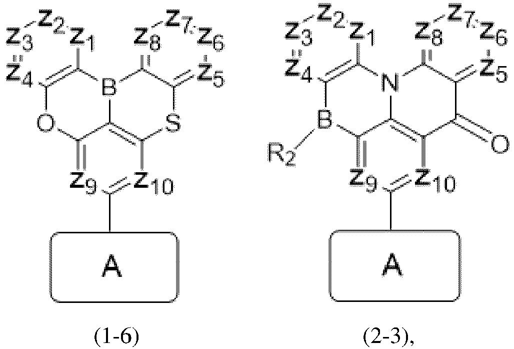
in Formula (1-2), (1-3), (1-6) or (2-3), A represents one of a substituted or unsubstituted C₆-C₆₀ carbocyclic group, and a substituted or unsubstituted C₃-C₆₀ heterocyclyl; a substituted group in A is one or a combination of at least two selected from deuterium, tritium, cyano, halogen, C₁-C₁₀ alkyl, C₃-C₁₀ cycloalkyl, silyl, C₆-C₃₀ arylamino, C₆-C₃₀ aryl, and C₂-C₃₀ heteroaryl, and the substituted group is independently connected to a connected aromatic ring or heteroaromatic ring to form a ring, or the substituted group is independently connected to a connected aromatic ring or heteroaromatic ring to not form a ring;
in Formula (1-2), (1-3), (1-6) or (2-3), Z₁-Z₁₀ are each independently represented as N or CR, with R being the same or different each time, two adjacent R can bond to each other to form a ring;
R₁ represents one of a substituted or unsubstituted C₁-C₁₀ alkyl, a substituted or unsubstituted C₃-C₁₀ cycloalkyl, a substituted or unsubstituted C₆-C₃₀ aryl, and a substituted or unsubstituted C₂-C₃₀ heteroaryl;
R₂ represents one of a substituted or unsubstituted C₆-C₃₀ aryl, and a substituted or unsubstituted C₂-C₃₀ heteroaryl;
R represents one of hydrogen, deuterium, tritium, cyano, halogen, a substituted or unsubstituted C₁-C₁₀ alkyl, a substituted or unsubstituted C₃-C₁₀ cycloalkyl, a substituted or unsubstituted C₁-C₁₀ alkoxy, a substituted or unsubstituted C₆-C₃₀ aryloxy, a substituted or unsubstituted C₆-C₃₀ arylamino, a substituted or unsubstituted C₆-C₃₀ aryl, and a substituted or unsubstituted C₂-C₃₀ heteroaryl;
a substituted group in the each above substituted R₁, R₂, and R is one or a combination of at least two selected from deuterium, tritium, cyano, halogen, C₁-C₁₀ alkyl, C₃-C₁₀ cycloalkyl, silyl, C₆-C₃₀ arylamino, C₆-C₃₀ aryl, and C₂-C₃₀ heteroaryl; and the substituted group is independently connected to the connected aromatic ring or heteroaromatic ring to form a ring, or the substituted group is independently connected to the connected aromatic ring or heteroaromatic ring to not form a ring;
where the triplet-triplet annihilation type host material is selected from at least one of compounds represented by BFH-18 to BFH-25:

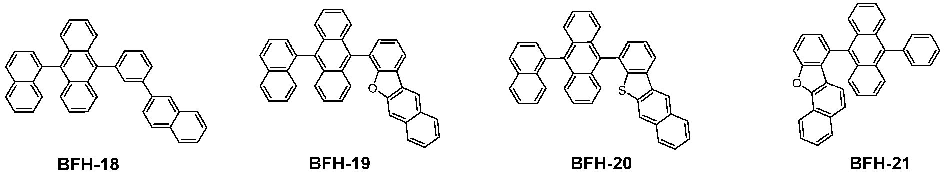

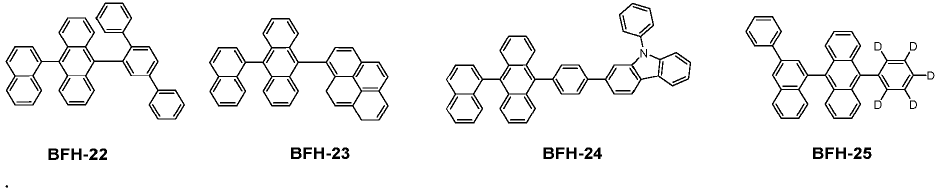

The present application further provides a display apparatus, including the above organic electroluminescent device.

In the organic electroluminescent device of the present application, the light-emitting layer includes a triplet-triplet annihilation material and a fluorescent dye with a structure represented by Formula (1-2), (1-3), (1-6) or (2-3). Among them, the triplet-triplet annihilation material with a triplet state annihilation effect and a lower triplet state energy level combined with the fluorescent dye with a reverse intersystem crossing property, can realize a highly efficient utilization of excitons in the system and reduce the concentration of triplet excitons in the system, achieve the improvement of luminescence efficiency and driving voltage of the device, and inhibit the roll-off of efficiency. In addition, the fluorescent dye of the present application can effectively suppress an intermolecular interaction of a planar multi-resonance compound, and inhibit the Dexter energy transfer between host and guest materials in the light-emitting layer and the influence of intermolecular interactions, further achieving the improvement of luminescence efficiency and driving voltage of the device.

### DESCRIPTION of EMBODIMENTS

In order to make the purpose, technical solution and advantages of the present application clearer, the following will provide a clear and complete description of the technical solution in the embodiments of the present application, with reference to the embodiments of the present application. Obviously, the described embodiments are a part of the embodiments of the present application, not all of them. Based on the embodiments in the present application, all other embodiments obtained by ordinary technical personnel in the art without creative work fall within the protection scope of the present application.

The present application provides an organic electroluminescent device including a light-emitting layer, the light-emitting layer includes a triplet-triplet annihilation type host and a fluorescent dye, where the fluorescent dye has a structure represented by the following Formula (1-2), (1-3), (1-6) or (2-3): 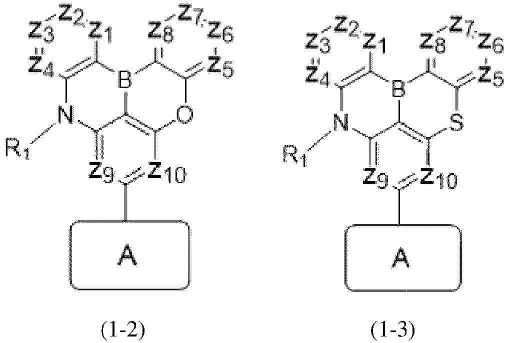 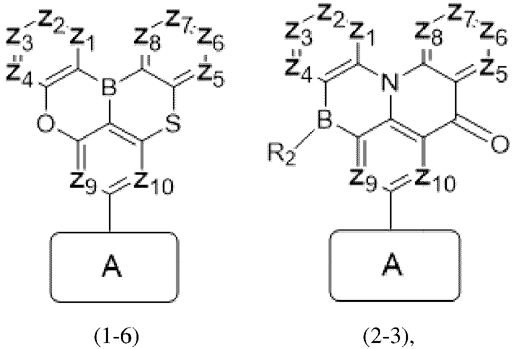
in Formula (1-2), (1-3), (1-6) or (2-3), A represents one of a substituted or unsubstituted C₆-C₆₀ carbocyclic group, and a substituted or unsubstituted C₃-C₆₀ heterocyclyl; a substituted group in A is one or a combination of at least two selected from deuterium, tritium, cyano, halogen, C₁-C₁₀ alkyl, C₃-C₁₀ cycloalkyl, silyl, C₆-C₃₀ arylamino, C₆-C₃₀ aryl, and C₂-C₃₀ heteroaryl, and the substituted group is independently connected to a connected aromatic ring or heteroaromatic ring to form a ring, or the substituted group is independently connected to a connected aromatic ring or heteroaromatic ring to not form a ring;
in Formula (1-2), (1-3), (1-6) or (2-3), Z₁-Z₁₀ are each independently represented as N or CR, with R being the same or different each time, two adjacent R can bond to each other to form a ring;
preferably, R₁ is connected to adjacent R through a single bond, while R₂ is connected to adjacent R through a single bond;
R₁ represents one of a substituted or unsubstituted C₁-C₁₀ alkyl, a substituted or unsubstituted C₃-C₁₀ cycloalkyl, a substituted or unsubstituted C₆-C₃₀ aryl, and a substituted or unsubstituted C₂-C₃₀ heteroaryl;
R₂ represents one of a substituted or unsubstituted C₆-C₃₀ aryl, and a substituted or unsubstituted C₂-C₃₀ heteroaryl;
the expression "R being the same or different each time" means that when at least two of Z₁-Z₁₀ are selected from CR, R in any two CRs are the same or different. Exemplarily, R represents one of hydrogen, deuterium, tritium, cyano, halogen, a substituted or unsubstituted C₁-C₁₀ alkyl, a substituted or unsubstituted C₃-C₁₀ cycloalkyl, a substituted or unsubstituted C₁-C₁₀ alkoxy, a substituted or unsubstituted C₆-C₃₀ aryloxy, a substituted or unsubstituted C₆-C₃₀ arylamino, a substituted or unsubstituted C₆-C₃₀ aryl, and a substituted or unsubstituted C₂-C₃₀ heteroaryl;
a substituted group in the each above substituted R₁, R₂, and R is one or a combination of at least two selected from deuterium, tritium, cyano, halogen, C₁-C₁₀ alkyl, C₃-C₁₀ cycloalkyl, silyl, C₆-C₃₀ arylamino, C₆-C₃₀ aryl, and C₂-C₃₀ heteroaryl; and the substituted group is independently connected to the connected aromatic ring or heteroaromatic ring to form a ring, or the substituted group is independently connected to the connected aromatic ring or heteroaromatic ring to not form a ring.

It should be noted that in the present application, the expression of Ca-Cb represents that the carbon atom number of the group is a-b. Unless otherwise specified, the carbon atom number generally does not include the carbon atom number of the substituted group. In the present application, the expression of chemical elements, unless otherwise specified, generally includes the concept of isotopes with same chemical properties. For example, the expression of "hydrogen" also includes the concepts of "deuterium" and "tritium" with same chemical properties; and carbon (C) includes ¹²C, ¹³C, etc., which will not be repeated.

In the disclosed Formulas of the present application, the expression of a ring structure streaked by "-" represents any position on the ring structure where a connectable site can form a bond.

The term " heteroaryl" in the present application refers to an aromatic cyclic group containing a heteroatom. The so-called heteroatom usually refers to N, O, S, P, Si, and Se, preferably N, O, and S.

The above C₆-C₆₀ aryl and C₃-C₆₀ heteroaryl in the present application, unless otherwise specified, are the aromatic group that meets π conjugated system, and the C₆-C₆₀ aryl and C₃-C₆₀ heteroaryl both include cases of a single ring and a fused ring. The so-called single ring means that there is at least one phenyl in the molecule; when there are at least two phenyls in the molecule, the phenyls are independent from each other and connected through a single bond, for example, phenyl, diphenyl, terphenyl, etc. A fused ring refers to a molecule that contains at least two benzene rings, which are not independent from each other, but rather share a common ring edge and are fused with each other, for example, naphthyl, anthryl, and phenanthryl, etc. A monocyclic heteroaryl refers to a molecule containing at least one heteroaryl, and when the molecule contains one heteroaryl and other groups (such as aryl, heteroaryl, alkyl, etc.), the heteroaryl and other groups are independent from each other and connected through a single bond, exemplarily, such as pyridine, furan, thiophene, etc. A fused ring heteroaryl means that it is formed by fusing at least one phenyl and at least one heteroaryl, or it is formed by fusing at least two heteroaryl, exemplarily, such as quinoline, isoquinoline, benzofuran, dibenzofuran, benzothiophene, dibenzothiophene, etc.

In the present application, the substituted or unsubstituted C₆-C₆₀ aryl is preferably C6-C30 aryl, and the carbon number of the aryl includes but is not limited to C6, C8, C10, C12, C14, C16, C18, C20, C22, C24, C26, C28, etc. Exemplarily, the aryl is preferably selected from a group consisting of phenyl, naphthyl, anthryl, benzoanthryl, phenanthryl, benzophenanthryl, pyrenyl, chrysenyl, perylenyl, fluoranthenyl, tetraphenyl, pentaphenyl, benzopyrenyl, biphenylyl, diphenyl, terphenyl, trimericphenyl, tetraphenyl, fluorenyl, spirodifluorenyl, dihydrophenanthryl, dihydropyrenyl, tetrahydropyrenyl, cis or trans indenofluorenyl, trimericindenyl, isotrimericindenyl, spiro-trimericindenyl, and spiro-isotrimericindenyl. Specifically, the biphenylyl is selected from 2-biphenylyl, 3-biphenylyl, and 4-biphenylyl; the terphenyl includes p-terphenyl-4-yl, p-terphenyl-3-yl, p-terphenyl-2-yl, m-terphenyl-4-yl, m-terphenyl-3-yl, and m-terphenyl-2-yl; the naphthyl includes 1-naphthyl or 2-naphthyl; the anthryl is selected from 1-anthryl, 2-anthryl, and 9-anthryl; the fluorenyl is selected from 1-fluorenyl, 2-fluorenyl, 3-fluorenyl, 4-fluorenyl, and 9-fluorenyl; the pyrenyl is selected from 1-pyrenyl, 2-pyrenyl, and 4-pyrenyl; the tetraphenyl is selected from 1-tetraphenyl, 2-tetraphenyl, and 9-tetraphenyl. As a preferred example of the aromatic ring in the present application, it is a group selected from a group consisting of phenyl, biphenylyl, terphenyl, naphthyl, anthryl, phenanthryl, indenyl, fluorenyl and derivatives thereof, fluoranthenyl, terphenylene, pyrenyl, pyrylo, chrysenyl, and tetraphenyl. The biphenylyl is selected from 2-biphenylyl, 3-biphenylyl, and 4-biphenylyl; the terphenyl includes p-terphenyl-4-yl, p-terphenyl-3-yl, p-terphenyl-2-yl, m-terphenyl-4-yl, m-terphenyl-3-yl, and m-terphenyl-2-yl; the naphthyl includes 1-naphthyl and 2-naphthyl; the anthryl is selected from a group consisting of 1-anthryl, 2-anthryl, and 9-anthryl; the fluorenyl is selected from a group consisting of 1-fluorenyl, 2-fluorenyl, 3-fluorenyl, 4-fluorenyl, and 9-fluorenyl; the fluorenyl derivative is selected from a group consisting of 9,9-dimethylfluorene, 9,9-spirodifluorene, and benzofluorene; the pyrenyl is selected from a group consisting of 1-pyrenyl, 2-pyrenyl, and 4-pyrenyl; the tetraphenyl is selected from a group consisting of 1-tetraphenyl, 2-tetraphenyl, and 9-tetraphenyl. The C6-C60 aryl in the present application may also be a group formed from the aforementioned groups via a single bond connection or/and by fusing.

In the presents application, the substituted or unsubstituted C3-C60 heteroaryl is preferably C3-C30 heteroaryl. In the present application, the carbon number of the heteroaryl includes but is not limited to C4, C5, C6, C8, C10, C12, C14, C16, C18, C20, C22, C24, C26, C28, etc., and the heteroaryl is a N-containing heteroaryl, a O-containing heteroaryl, a S-containing heteroaryl, etc. Specific examples are: furyl, thienyl, pyrryl, pyridinyl benzofuryl, benzothienyl, isobenzofuryl, isobenzothienyl, indolyl, isoindolyl, dibenzofuryl, dibenzothienyl, carbazolyl and derivatives thereof, quinolyl, isoquinolyl, acridinyl, phenanthridinyl, benzo-5,6-quinolyl, benzo-6,7-quinolyl, benzo-7,8-quinolyl, phenothiazinyl, phenazinyl, pyrazolyl, indazolyl, imidazolyl, benzimidazolyl, naphthoimidazolyl, phenanthroimidazolyl, pyridino imidazolyl, pyrazino imidazolyl, quinoxalino imidazolyl, oxazolyl, benzo-oxazolyl, naphtho-oxazolyl, anthro-oxazolyl, phenanthro-oxazolyl, 1,2-thiazolyl, 1,3-thiazolyl, benzothiazolyl, pyridazinyl, benzopyridazinyl, pyrimidinyl, benzopyrimidinyl, quinoxalinyl, 1,5-diaza anthryl, 2,7-diaza pyrenyl, 2,3-diaza pyrenyl, 1,6- diaza pyrenyl, 1,8-diaza pyrenyl, 4,5-diaza pyrenyl, 4,5,9,10-tetraaza perylenyl, pyrazinyl, phenazinyl, phenothiazinyl, naphthyridyl, azacarbazolyl, benzocarbolinyl, phenanthrolinyl, 1,2,3-triazolyl, 1,2,4-triazolyl, benzotriazolyl, 1,2,3-oxadiazolyl, 1,2,4-oxadiazolyl, 1,2,5-oxadiazolyl, 1,2,3-thiadiazolyl, 1,2,4-thiadiazolyl, 1,2,5-thiadiazolyl, 1,3,4-thiadiazolyl, 1,3,5-triazinyl, 1,2,4-triazinyl, 1,2,3-triazinyl, tetrazolyl, 1,2,4,5-tetrazinyl, 1,2,3,4-tetrazinyl, 1,2,3,5-tetrazinyl, purinyl, pteridyl, indozinyl, benzothiadiazolyl, etc. As a preferred example of the heterocyclyl in the present application, it is, for example, furyl, thienyl, pyrryl, benzofuryl, benzothienyl, isobenzofuryl, indolyl, dibenzofuryl, dibenzothienyl, carbazolyl and derivatives thereof, where the carbazolyl derivatives are preferably 9-phenyl carbazole, 9-naphthyl benzocarbazolyl, benzocarbazolyl, dibenzocarbazolyl or indolo carbazolyl. The C3-C60 heteroaryl in the present application may also be a group formed from the aforementioned groups via a single bond connection or/and by fusing.

In the present application, alkyl is not specified, and includes the concepts of linear alkyl and branched alkyl, as well as cycloalkyl. The carbon number of alkyl includes but is not limited to C1, C2, C3, C4, C5, C6, C7, C8, C9, C10, C11, C12, C13, C14, C15, C16, C17, C18, C19, C20, C22, C24, C26, C28, etc. As a C1-C30 alkyl, it is preferably C1-C20 alkyl, for example, methyl, ethyl, n-propyl, isopropyl, n-butyl, isobutyl, sec-butyl, tert-butyl, 2-methyl butyl, n-pentyl, sec-pentyl, cyclopentyl, neopentyl, n-hexyl, cyclohexyl, adamantyl, neo-hexyl, n-heptyl, cycloheptyl, n-octyl, cyclooctyl, 2-ethyl hexyl, trifluoromethyl, pentafluoroethyl, 2,2,2-trifluoroethyl, etc, more preferably, it is C1-C10 alkyl.

In the present application, the cycloalkyl includes monocyclic alkyl and polycyclic alkyl, and the carbon number of cycloalkyl includes but is not limited to C4, C5, C6, C7, C8, C9, etc. For example, it is cyclopropyl, cyclobutyl, cyclopentyl, cyclohexyl, cycloheptyl, etc.

In the present application, as an example of C1-C20 alkoxy, it is, methoxy, ethoxy, n-propoxy, isopropoxy, n-butoxy, sec-butoxy, isobutoxy, tert-butoxy, pentyloxy, isopentyloxy, hexyloxy, heptyloxy, octyloxy, nonyloxy, decyloxy, undecyloxy, dodecyloxy, and etc., preferably, methoxy, ethoxy, n-propoxy, isopropoxy, tert-butoxy, sec-butoxy, isobutoxy, isopentyloxy, more preferably, methoxy.

In the present application, for an example of C1-C20 silyl group, it is the silyl that is substituted by the group exemplified in the above C1-C20 alkyl. Specifically, for example, methyl silyl, dimethyl silyl, trimethyl silyl, ethyl silyl, diethyl silyl, triethyl silyl, tert-butyl dimethyl silyl, tert-butyl diphenyl silyl and other groups are enumerated.

In the present application, for a C6-C60 aryloxy, the groups that are formed by connecting the group listed in the substituted or unsubstituted C6-C60 aryl with oxygen can be enumerated. Specific examples may refer to the above examples and will not be repeated here.

In the present application, as an example of halogens, fluorine, chlorine, bromine, iodine, etc. can be enumerated.

In the present application, a C6-C60 arylamino or a C3-C60 heteroarylamino refers to a group obtained by substituting one or two H in amino group (-NH₂) with the above illustrative C6-C60 aryl or C3-C60 heteroaryl.

The light-emitting layer of the organic electroluminescent device of the present application includes a host material and a fluorescent dye, where the host material is a triplet-triplet annihilation, TTA, material, and the fluorescent dye is a planar multi-resonance type compound represented by Formula (1-2), (1-3), (1-6) or (2-3) and with an inverse intersystem crossing property. Specifically, the energy level of the first excited singlet state of the host material is greater than that of the first excited singlet state of the fluorescent dye, and the energy level of the first excited triplet state of the host material is less than that of the first excited triplet state of the fluorescent dye. Because the host material and the fluorescent dye have such relationship of energy level, after the organic electroluminescent device is electrically excited, the first excited singlet exciton of the host material will undergo Föster transition to the first excited singlet state of the low-energy level fluorescent dye. Although the fluorescent dye has the first excited triplet state with a higher energy level, the fluorescent dye will generate an up-conversion process due to its inverse intersystem crossing property. Therefore, the first excited triplet excitons and the first excited singlet excitons from the fluorescent dye and the first excited singlet excitons from the host material will jump to the ground state for emitting fluorescence. In addition, the excitons in the energy level of the first excited triplet state of the fluorescent dye that are too late for upconversion will also jump to the first excited triplet state of the low-energy host material, and then a phenomena that the triplet excitons are annihilated in pairs to generate singlet excitons occurs. Based on the energy transfer process, the organic electroluminescent device of the present application can not only effectively utilize the triplet excitons, but also have a low concentration of the triplet excitons in the system. Therefore, the organic electroluminescent device of the present application has excellent luminescence efficiency, roll-off of efficiency and low driving voltage.

In addition to the above reasons, the inventor believes that the improvement of device performance may also be related to the fluorescent dye used in the present application. On the one hand, the molecular structure of Formula (1-2), (1-3), (1-6) or (2-3) introduces a carbon ring or heterocyclic that is represented by A and coated by a group with large steric effect. The group with large steric effect not only does not have a significant impact on the emitting color and half-peak width of the parent nucleus, but also can effectively inhibit the interaction between planar type multi-resonance compounds, so as to effectively inhibit the reduction of the luminescence efficiency and spectral broadening of compounds at a high concentration. On the other hand, the molecular structures of Formula (1-2), (1-3), (1-6) or (2-3) can effectively inhibit the influence between host and guest materials in the light-emitting layer, including Dexter energy transfer and intermolecular interaction, thereby greatly improving the luminescence efficiency of the device and reducing the driving voltage, and realizing the optimization of the efficiency process window qualification, enhancing the stability of the luminescence efficiency and driving voltage. Moreover, the feasibility of chemical synthesis of the fluorescent dye represented by Formula (1-2), (1-3), (1-6) or (2-3) is higher, and it is easy to make various functional modifications, allowing for further structural adjustments according to different application requirements.

Furthermore, the fluorescent dye has a structure represented by any one of the following Formula (1-1), (1-4), (1-5), (2-1), or (2-2). The structures represented by Formula (1-1), (1-4), (1-5), (2-1), or (2-2) are not according to the invention and are present for illustration purposes only. 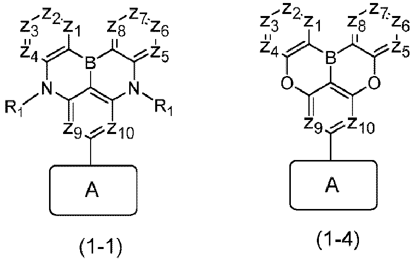 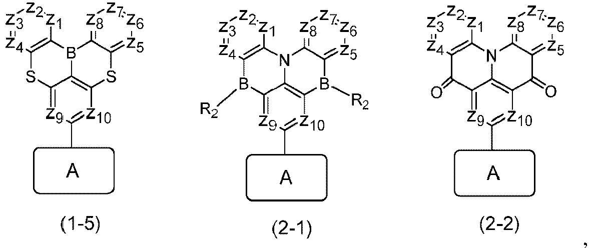 where, Z₁-Z₁₀ are each independently represented as CR, and the definitions of A, R, R₁, and R₂ are same as those in Formula (1-2), (1-3), (1-6) or (2-3); preferably, R₁ is connected to adjacent R through a single bond, and R₂ is connected to adjacent R through a single bond.

Further, in the aforementioned structure of the fluorescent dye, A represents a substituted group represented by any one of the following (3-1), (3-2), (3-3), (3-4), (3-5), (3-6), (3-7), (3-8) or (3-9): 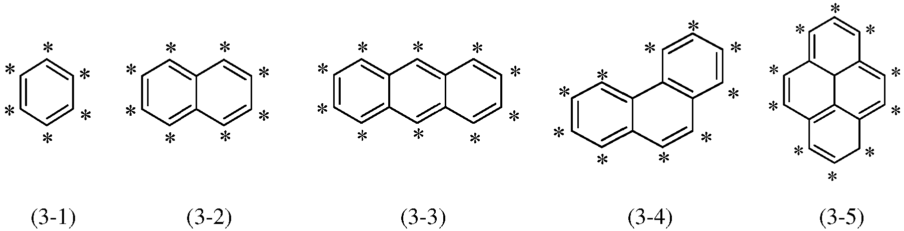 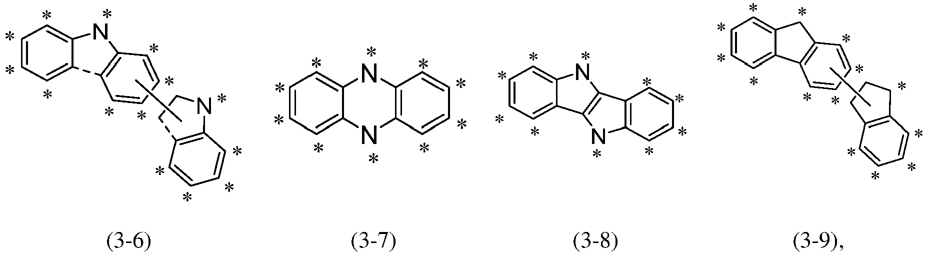 an asterisks in the above Formulas denotes a connectable site, such "connection" denotes connection to a parent nucleus and/or connection with a substituted group; an expression of a ring structure streaked by "-" denotes any position on the ring structure where the connectable site can form a bond; a dashed line in the above Formulas represents being connected or being unconnected; a substituted group in A is one or a combination of at least two selected from deuterium, tritium, cyano, halogen, C₁-C₁₀ alkyl, C₃-C₁₀ cycloalkyl, C₆-C₃₀ arylamino, C₆-C₃₀ aryl, C₂-C₃₀ heteroaryl, and the substituted group is independently connected to a connected aromatic ring or heteroaromatic ring to form a ring, or the substituted group is independently connected to a connected aromatic ring or heteroaromatic ring to not form a ring.

Furthermore, A is represented by any one of the following structural formulas: 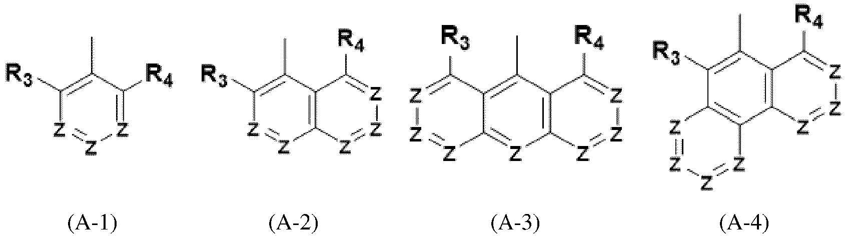 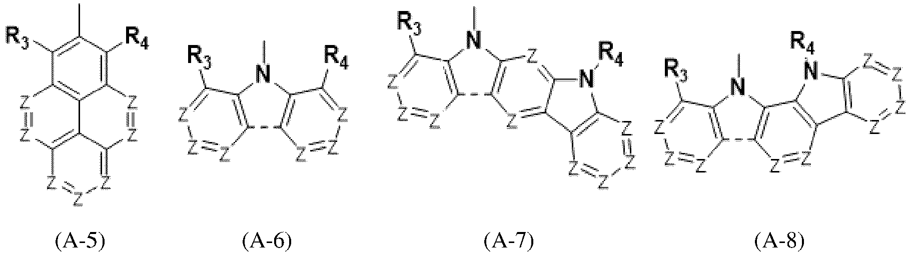 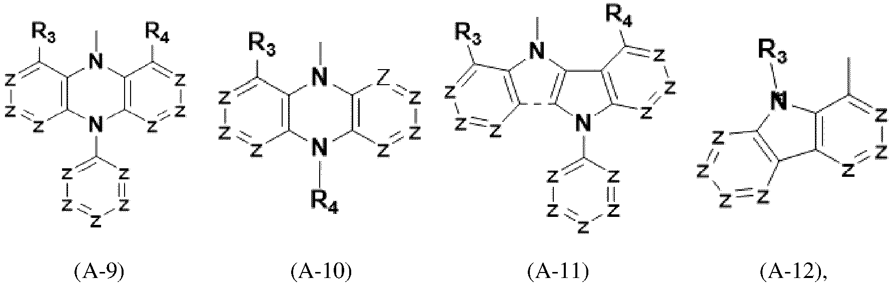 where, R₃ and R₄ are each independently represented as any one of hydrogen, deuterium, tritium, a substituted or unsubstituted C₁-C₃₀ alkyl, a substituted or unsubstituted C₃-C₃₀ cycloalkyl, a silyl, a substituted or unsubstituted C₁-C₃₀ alkoxy, a substituted or unsubstituted C₆-C₆₀ aryloxy, a substituted or unsubstituted C₆-C₆₀ arylamino, a substituted or unsubstituted C₆-C₆₀ aryl, and a substituted or unsubstituted C₂-C₆₀ heteroaryl; Z is independently represented as N or CR₅, with R₅ being the same or different each time, and preferably, two adjacent R₅ can bond with each other to form a ring; R₅ is represented as one of hydrogen, deuterium, tritium, cyano, halogen, a substituted or unsubstituted C₁-C₁₀ alkyl, a substituted or unsubstituted C₃-C₁₀ cycloalkyl, a substituted or unsubstituted C₁-C₁₀ alkoxy, a substituted or unsubstituted C₆-C₃₀ aryloxy, a substituted or unsubstituted C₆-C₃₀ arylamino, a substituted or unsubstituted C₆-C₃₀ aryl, or a substituted or unsubstituted C₂-C₃₀ heteroaryl; a substituted group in the each substituted R₃, R₄, and R₅ is one or a combination of at least two selected from deuterium, tritium, cyano, halogen, C₁-C₁₀ alkyl, C₃-C₁₀ cycloalkyl, silyl, C₆-C₃₀ arylamino, C₆-C₃₀ aryl, C₂-C₃₀ heteroaryl, where the substituted group is independently connected to the connected aromatic ring or heteroaromatic ring to form a ring, or the substituted group is independently connected to the connected aromatic ring or heteroaromatic ring to not form a ring.

Further, above R₃ and R₄ are each independently represented as any one of a substituted or unsubstituted C₁-C₃₀ alkyl, a substituted or unsubstituted C₃-C₃₀ cycloalkyl, a substituted or unsubstituted C₆-C₆₀ aryl, and a substituted or unsubstituted C₂-C₆₀ heteroaryl; preferably, at least one of the above R₃ and R₄ is selected from one of the following groups with large steric effect: terphenyl, trimericphenyl, tetraphenyl, fluorenyl, spirodifluorenyl, dihydrophenanthryl, dihydropyrenyl, tetrahydropyrenyl, cis or trans indenofluorenyl, trimericindenyl, isotrimericindenyl, spiro-trimericindenyl, spiro-isotrimericindenyl, furyl, benzofuryl, isobenzofuryl, dibenzofuryl, benzothienyl, isobenzothienyl, dibenzothienyl, isoindolyl, carbazolyl, indenocarbazolyl, isoquinolyl, acridinyl, phenanthridinyl, benzo-5,6-quinolyl, benzo-6,7-quinolyl, benzo-7,8-quinolyl, benzimidazolyl, naphthoimidazolyl, phenanthroimidazolyl, pyridino imidazolyl, pyrazino imidazolyl, quinoxalino imidazolyl, oxazolyl, benzo-oxazolyl, naphtho-oxazolyl, anthro-oxazolyl, phenanthro-oxazolyl, 1,2-thiazolyl, 1,3-thiazolyl, benzothiazolyl, pyridazinyl, benzopyridazinyl, 1,5-diaza anthryl, 2,7- diaza pyrenyl, 2,3-diaza pyrenyl, 1,6-diaza pyrenyl, 1,8-diaza pyrenyl, 4,5-diaza pyrenyl, 4,5,9,10-tetraaza perylenyl, phenazinyl, phenothiazinyl, azacarbazolyl, benzocarbolinyl, phenanthrolinyl, 1,2,3-triazolyl, 1,2,4-triazolyl, benzotriazolyl, 1,2,3-oxadiazolyl, 1,2,4-oxadiazolyl, 1,2,5-oxadiazolyl, 1,2,3-thiadiazolyl, 1,2,4-thiadiazolyl, 1,2,5-thiadiazolyl, 1,3,4-thiadiazolyl, 1,3,5-triazinyl, 1,2,4-triazinyl, 1,2,3-triazinyl, tetrazolyl, 1,2,4,5-tetrazinyl, 1,2,3,4-tetrazinyl, 1,2,3,5-tetrazinyl, purinyl, pteridyl, indozinyl, benzothiadiazolyl, 9,9-dimethylacridinyl, diphenylamindo, adamantyl, fluorophenyl, methylphenyl, trimethylphenyl, cyanophenyl, silyl; or at least one of R₃ or R₄ is a combination formed by at least two groups selected from the aforementioned groups with large steric effect.

In addition, in the fluorescent dye of the present application, R denotes one of hydrogen, deuterium, tritium, fluorine atom, cyano, methyl, deuterated methyl, tritiated methyl, ethyl, deuterated ethyl, tritiated ethyl, isopropyl, deuterated isopropyl, tritiated isopropyl, tert-butyl, deuterated tert-buty, tritiated tert-butyl, deuterated cyclopentyl, tritiated cyclopentyl, cyclohexyl, cyclopentyl, adamantyl, phenyl, deuterated phenyl, tritiated phenyl, biphenyl, deuterated biphenyl, tritiated biphenyl, deuterated terphenyl, tritiated terphenyl, terphenyl, naphthyl, anthryl, phenanthryl, pyridyl, quinolyl, furyl, thienyl, dibenzofuryl, dibenzothienyl, carbazolyl, N-phenylcarbazolyl, 9,9-dimethylfluorenyl, 9,9-diphenylfluorenyl, spirofluorenyl, methyl-substituted phenyl, ethyl-substituted phenyl, isopropyl-substituted phenyl, tert-butyl-substituted phenyl, methyl-substituted biphenyl, ethyl-substituted biphenyl, isopropyl-substituted biphenyl, tert-butyl-substituted biphenyl, deuterated methyl-substituted phenyl, deuterated ethyl-substituted phenyl, deuterated isopropyl-substituted phenyl, deuterated tert-butyl-substituted phenyl, deuterated methyl-substituted biphenyl, deuterated ethyl-substituted biphenyl, deuterated isopropyl-substituted biphenyl, deuterated tert-butyl-substituted diphenyl, tritiated methyl-substituted phenyl, tritiated ethyl-substituted phenyl, tritiated isopropyl-substituted phenyl, tritiated tert-butyl-substituted phenyl, tritiated methyl-substituted diphenyl, tritiated ethyl-substituted diphenyl, tritiated isopropyl-substituted diphenyl, tritiated tert-butyl-substituted diphenyl, diphenylamido, di-biphenylamido, and triphenylamino.

R₁ represents one of methyl, deuterated methyl, tritiated methyl, ethyl, deuterated ethyl, tritiated ethyl, isopropyl, deuterated isopropyl, tritiated isopropyl, tert-butyl, deuterated tert-butyl, tritiated tert-butyl, deuterated cyclopentyl, tritiated cyclopentyl, cyclopentyl, adamantyl, phenyl, deuterated phenyl, tritiated phenyl, biphenyl, deuterated biphenyl, tritiated biphenyl, deuterated terphenyl, tritiated terphenyl, terphenyl, naphthyl, anthryl, phenanthryl, pyridyl, quinolyl, furyl, thienyl, dibenzofuryl, dibenzothienyl, carbazolyl, N-phenylcarbazolyl, 9,9-dimethylfluorenyl, 9,9-diphenylfluorenyl, spirofluorenyl, methyl-substituted phenyl, ethyl-substituted phenyl, isopropyl-substituted phenyl, tert-butyl-substituted phenyl, methyl-substituted phenyl, ethyl-substituted diphenyl, isopropyl-substituted diphenyl, tert-butyl-substituted diphenyl, deuterated methyl-substituted phenyl, deuterated ethyl-substituted phenyl, deuterated isopropyl-substituted phenyl, deuterated tert-butyl-substituted phenyl, deuterated methyl-substituted diphenyl, deuterated ethyl-substituted diphenyl, deuterated isopropyl-substituted diphenyl, deuterated tert-butyl-substituted diphenyl, tritiated methyl-substituted phenyl, tritiated ethyl-substituted phenyl, tritiated isopropyl-substituted phenyl, tritiated tert-butyl-substituted phenyl, tritiated methyl-substituted diphenyl, tritiated ethyl-substituted diphenyl, tritiated isopropyl-substituted diphenyl, and tritiated tert-butyl-substituted diphenyl.

R₂ represents one of phenyl, deuterated phenyl, tritiated phenyl, biphenyl, deuterated biphenyl, tritiated biphenyl, deuterated terphenyl, tritiated terphenyl, terphenyl, naphthyl, anthryl, phenanthryl, pyridyl, quinolyl, dibenzofuryl, dibenzothienyl, N-phenylcarbazolyl, methyl-substituted phenyl, amidine, ethyl-substituted phenyl, isopropyl-substituted phenyl, tert-butyl-substituted phenyl, methyl-substituted biphenyl, ethyl-substituted biphenyl, isopropyl-substituted biphenyl, tert-butyl-substituted biphenyl, deuterated methyl-substituted phenyl, deuterated ethyl-substituted phenyl, deuterated isopropyl-substituted phenyl, deuterated tert-butyl-substituted phenyl, deuterated methyl-substituted biphenyl, deuterated ethyl-substituted biphenyl, deuterated isopropyl-substituted biphenyl, deuterated tert-butyl-substituted biphenyl, tritiated methyl-substituted phenyl, tritiated ethyl-substituted phenyl, tritiated isopropyl-substituted phenyl, tritiated tert-butyl-substituted phenyl, tritiated methyl-substituted diphenyl, tritiated ethyl-substituted diphenyl, tritiated isopropyl-substituted diphenyl, and tritiated tert-butyl-substituted diphenyl.

In a specific implementation, in Formula (1-1), (1-2), (1-3), (1-4), (1-5), (1-6), (2-1), (2-2), or (2-3), Z₉ and Z₁₀ are each CR, and R is hydrogen, while Z₁-Z₈ are each CR, the definition of R is the same as that in Formula (1-1), (1-2), (1-3), (1-4), (1-5), (1-6), (2-1), (2-2), or (2-3).

In another specific implementation, in Formula (1-1), (1-2), (1-3), (1-4), (1-5), (1-6), (2-1), (2-2), or (2-3), Z₂ and Z₇ are each CR, and R is tert-butyl, while Z₁, Z₃-Z₆, and Z₈-Z₁₀ are each CR, and R is hydrogen.

More specifically, the fluorescent dye in the present application is selected from a compound represented by the following structural formulas. The compounds represented by formulas S1-S93, S190-S336, and S340-S345 are not according to the invention and are present for illustration purposes only.

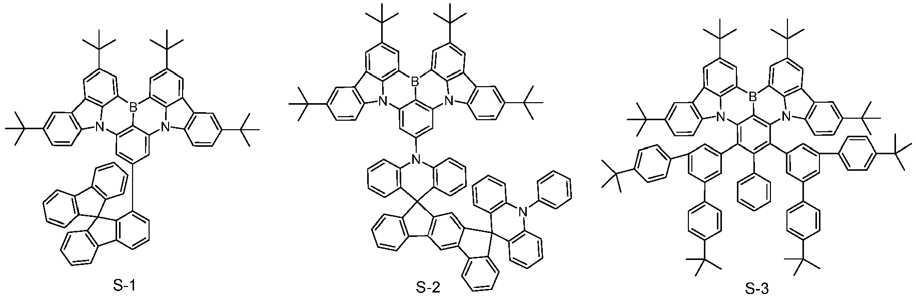

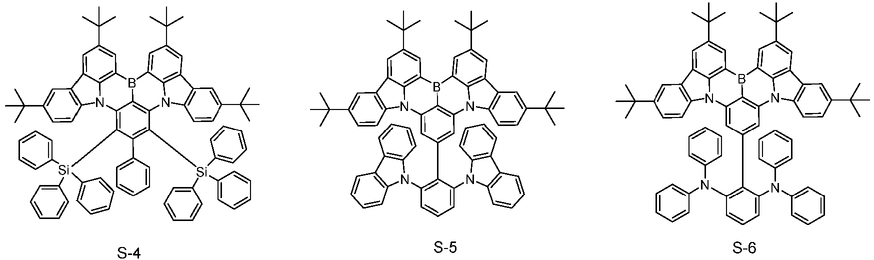

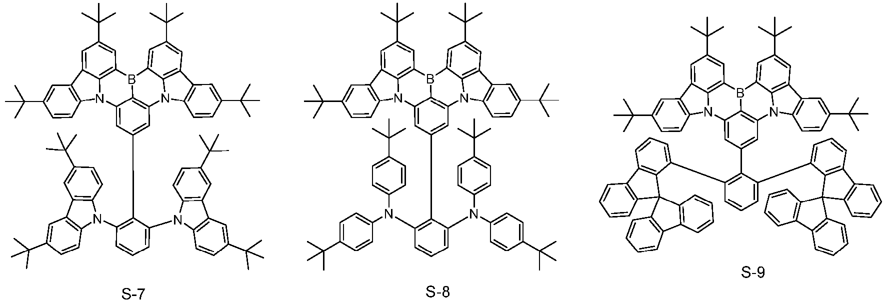

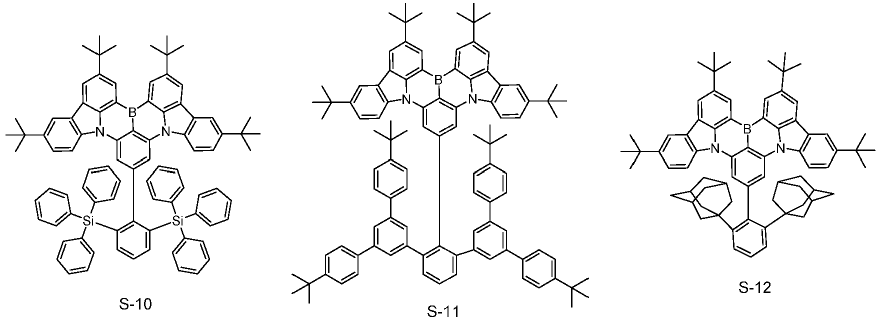

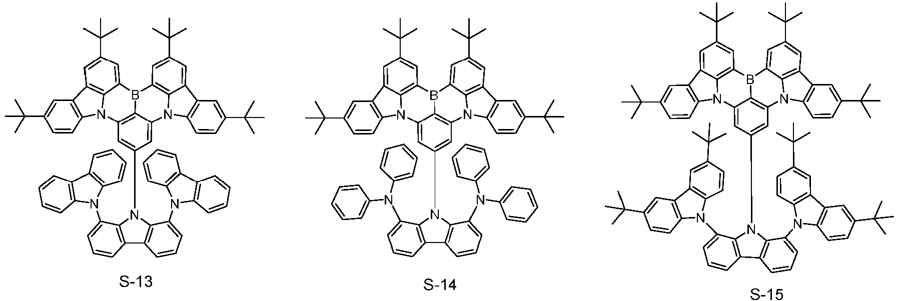

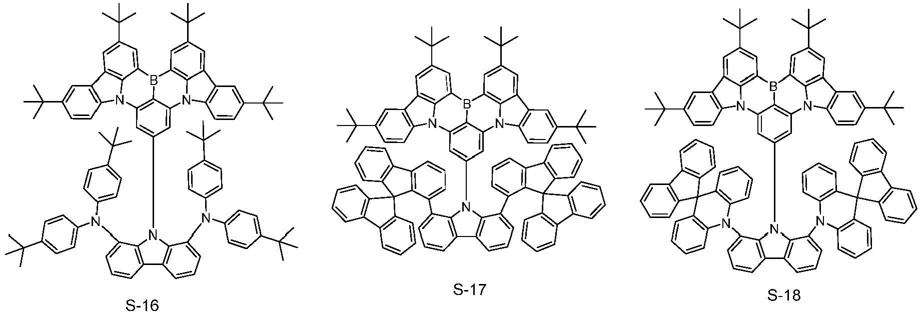

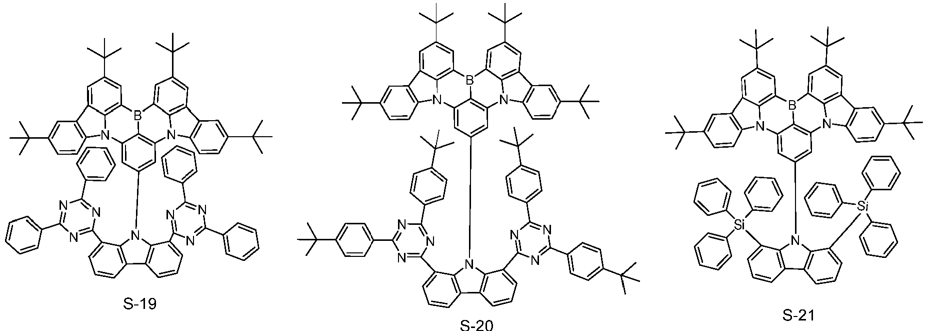

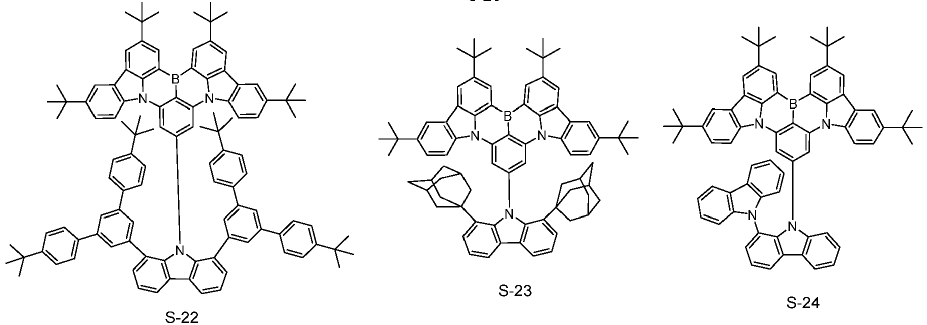

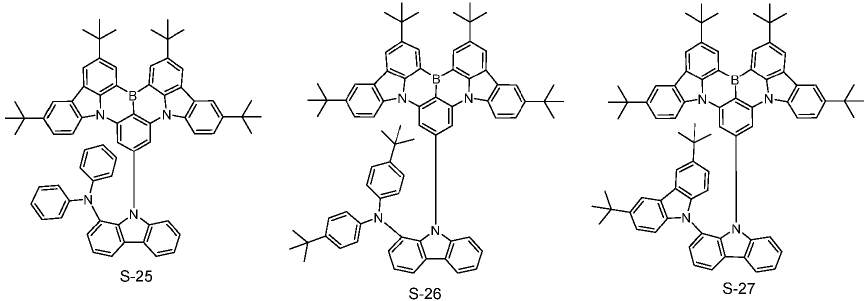

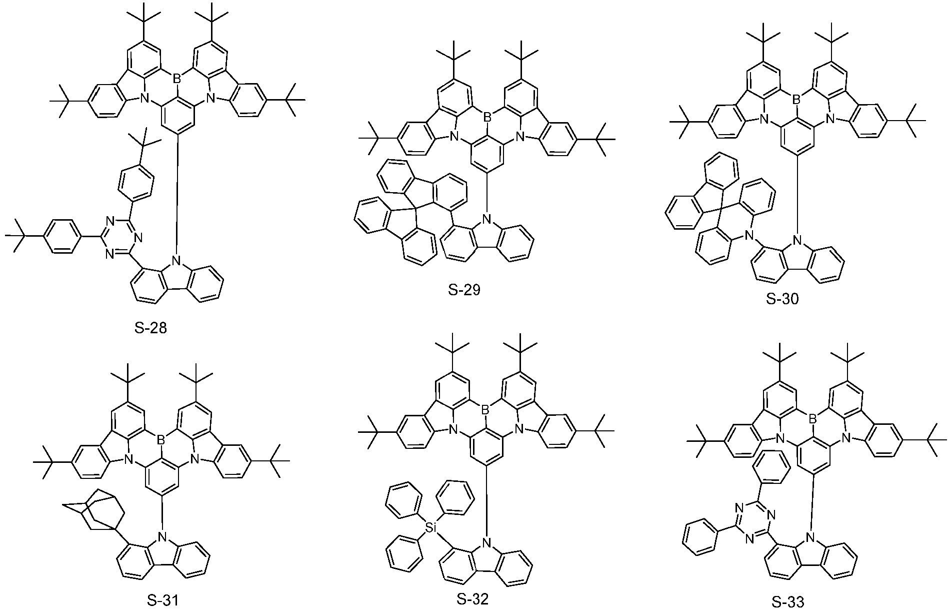

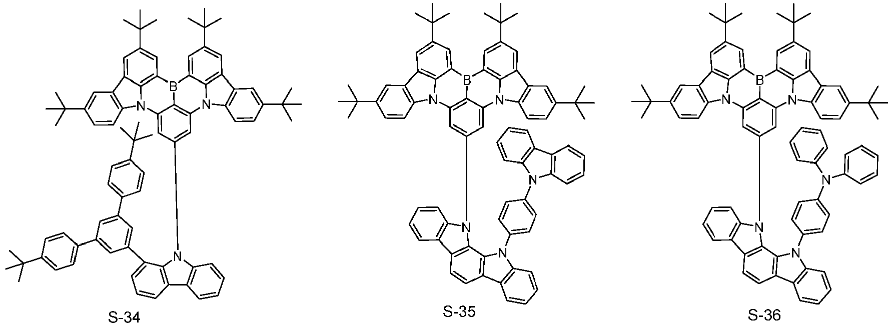

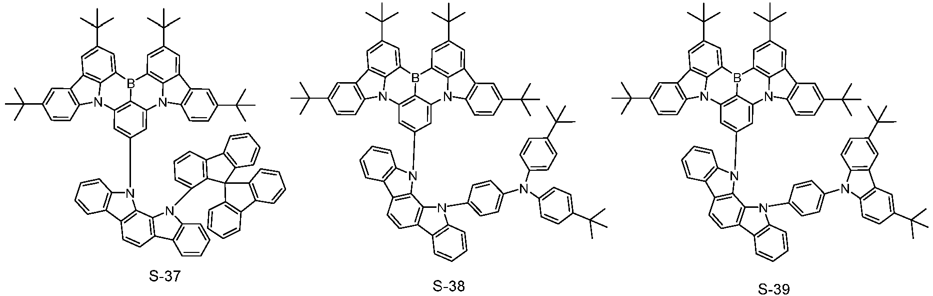

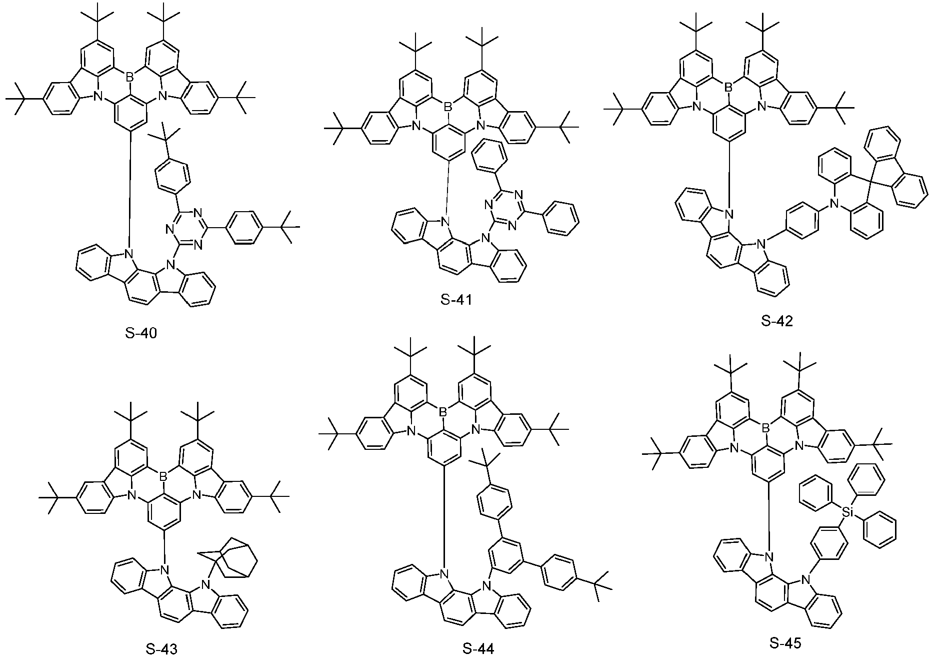

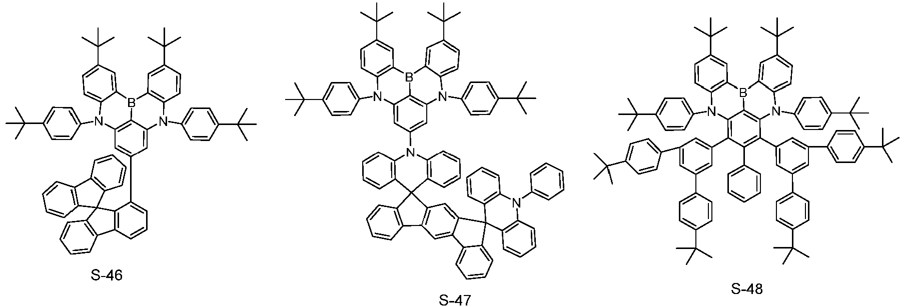

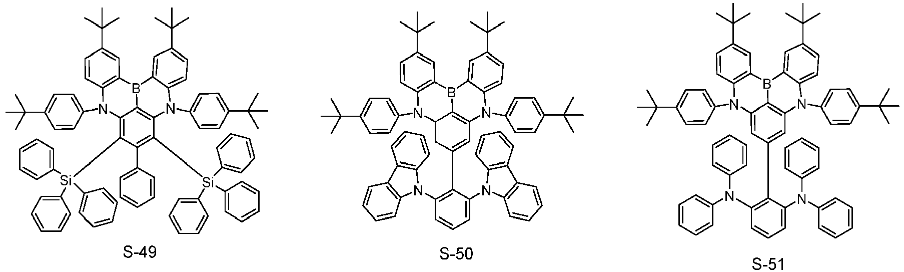

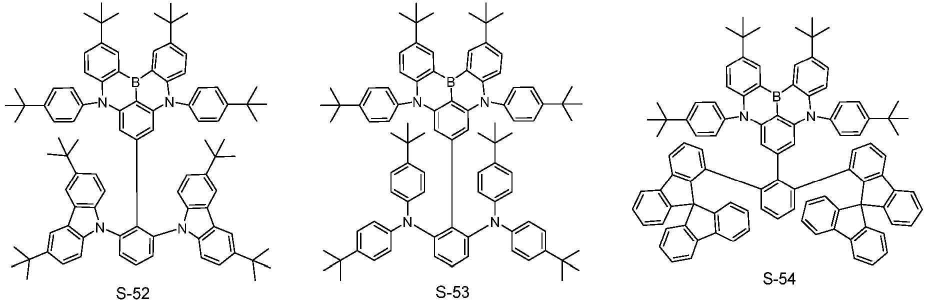

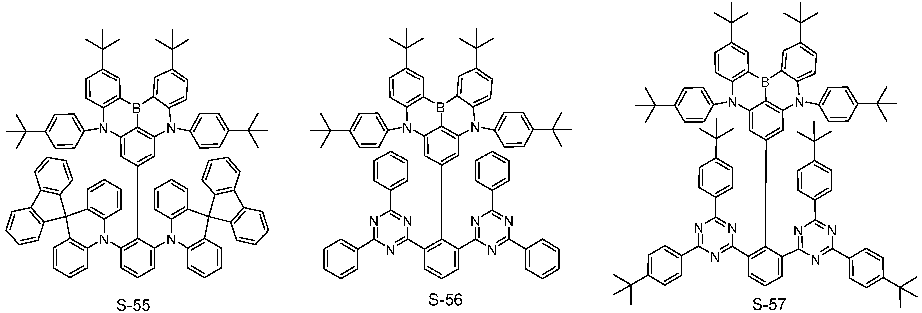

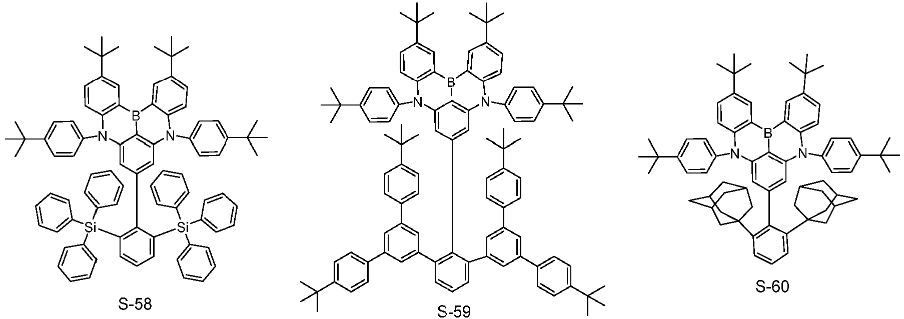

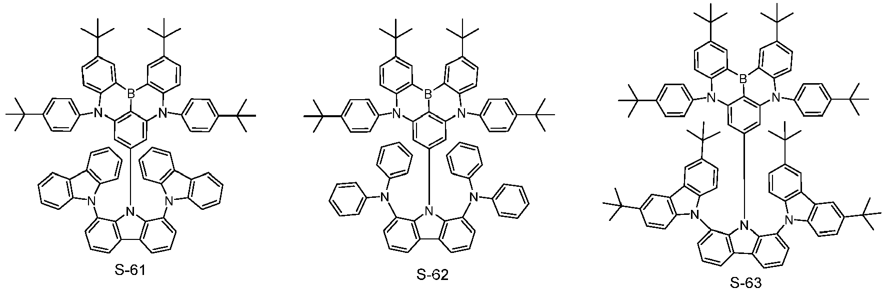

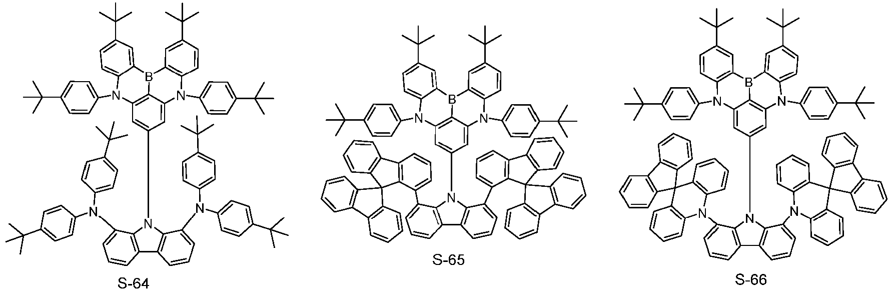

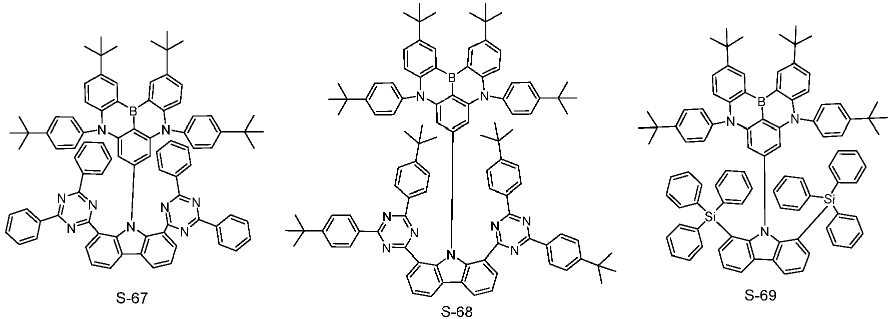

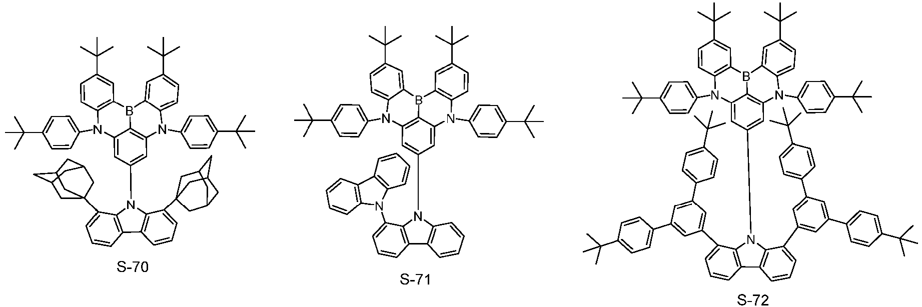

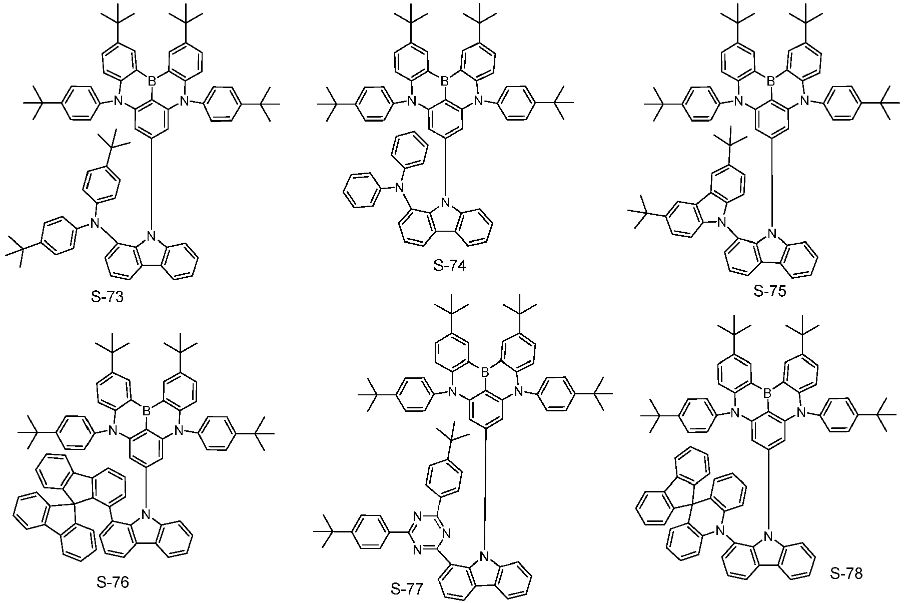

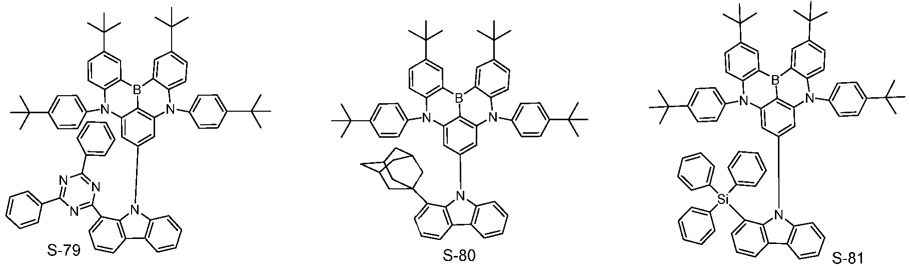

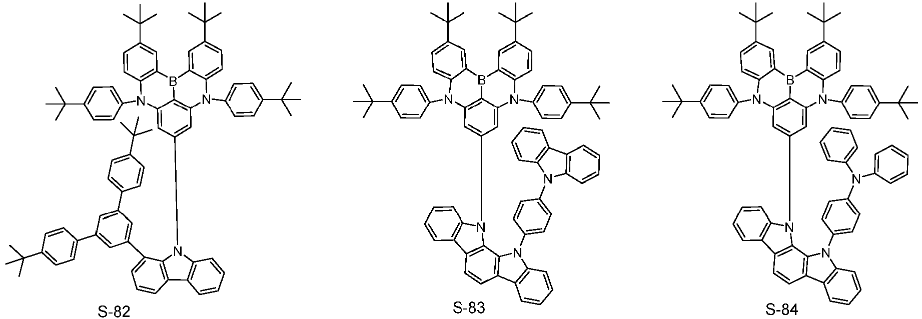

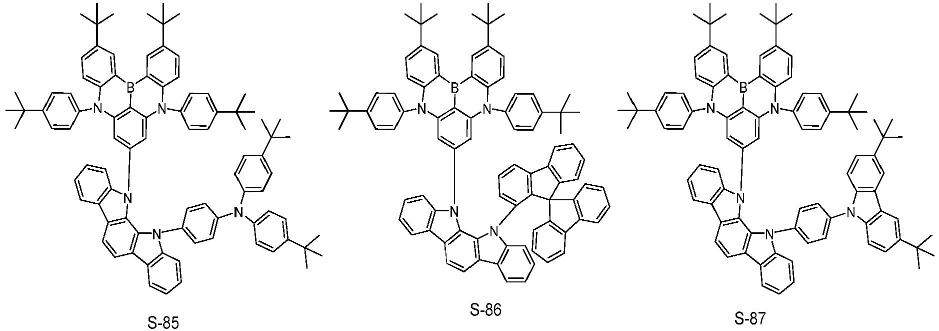

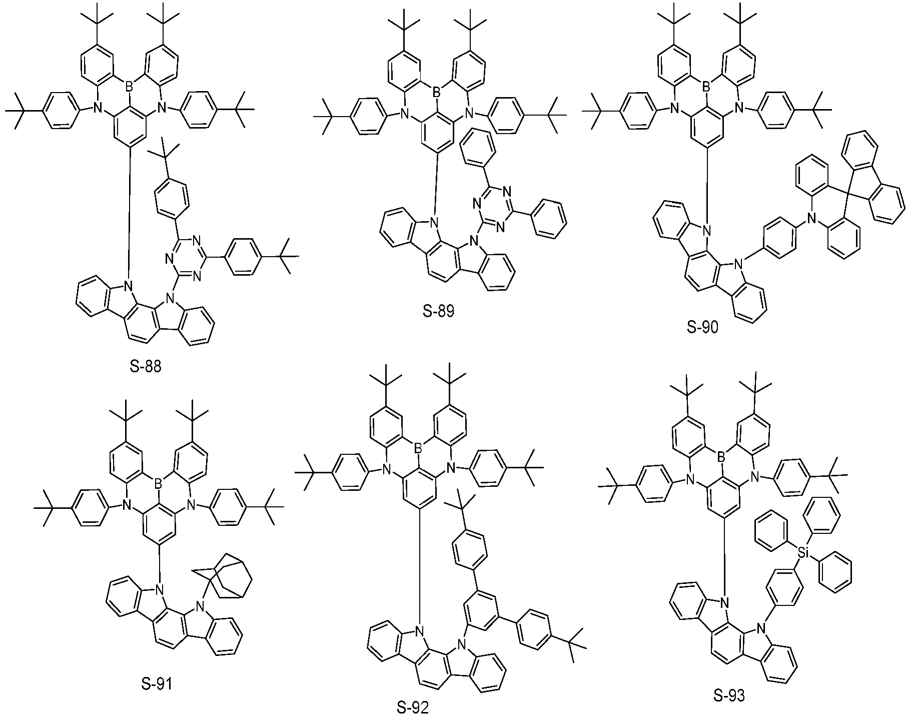

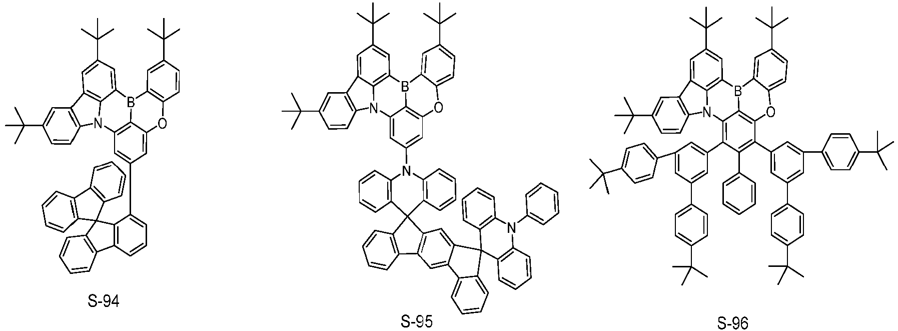

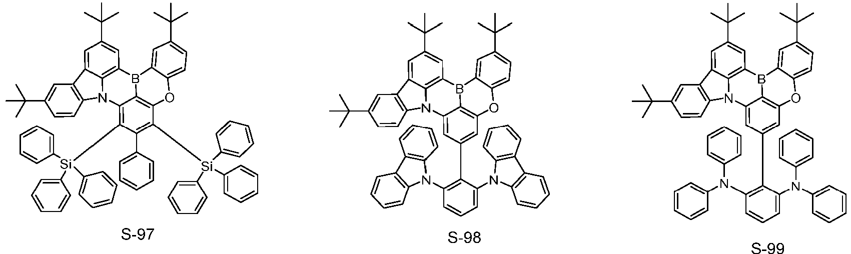

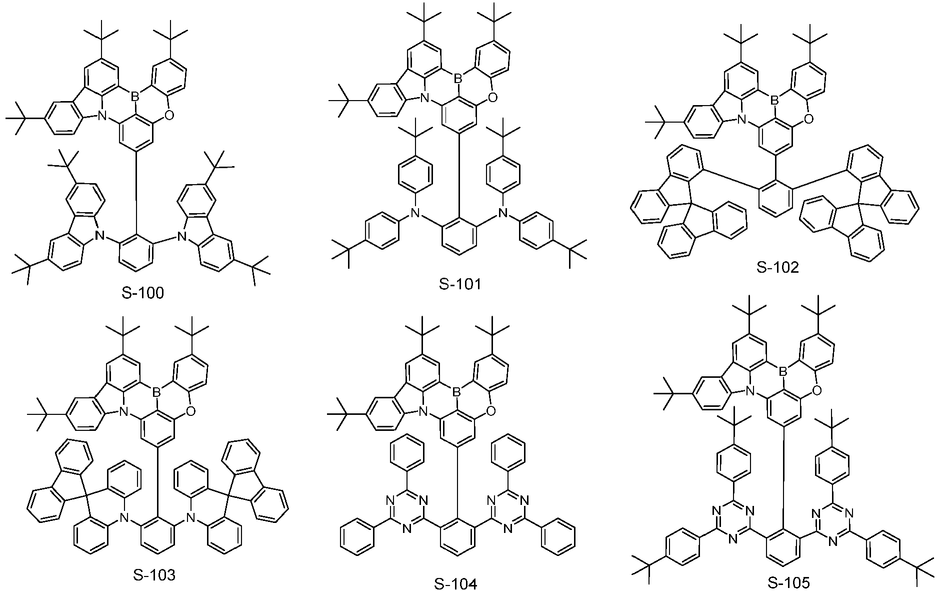

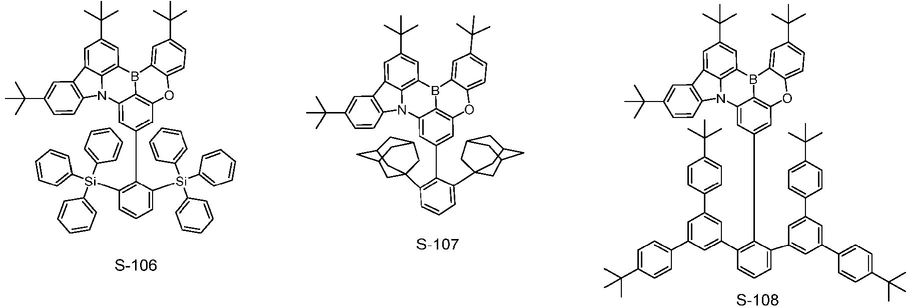

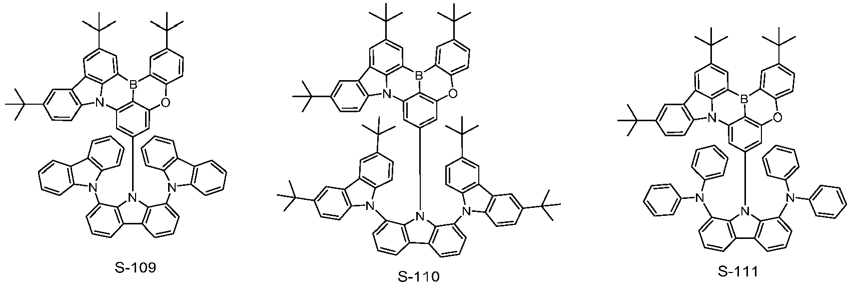

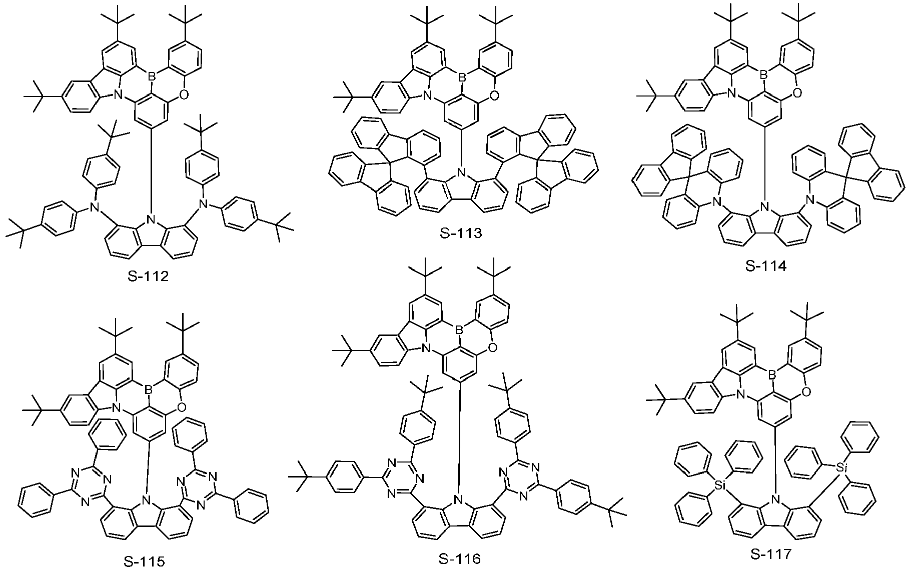

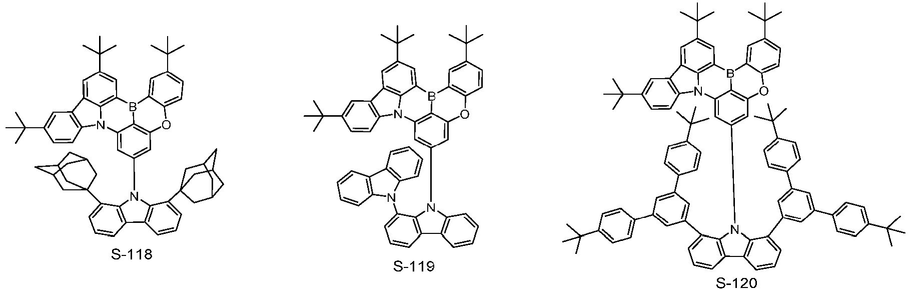

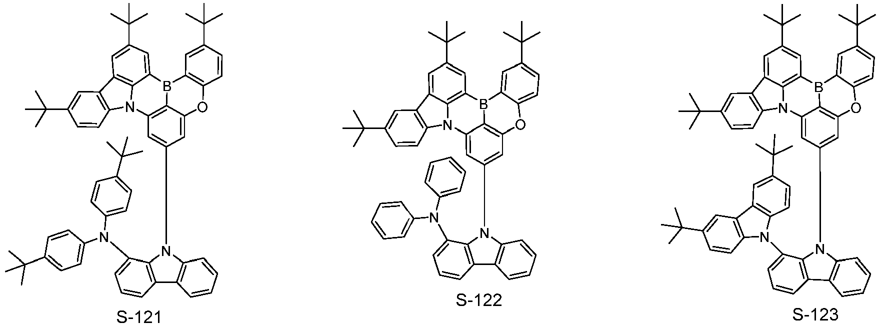

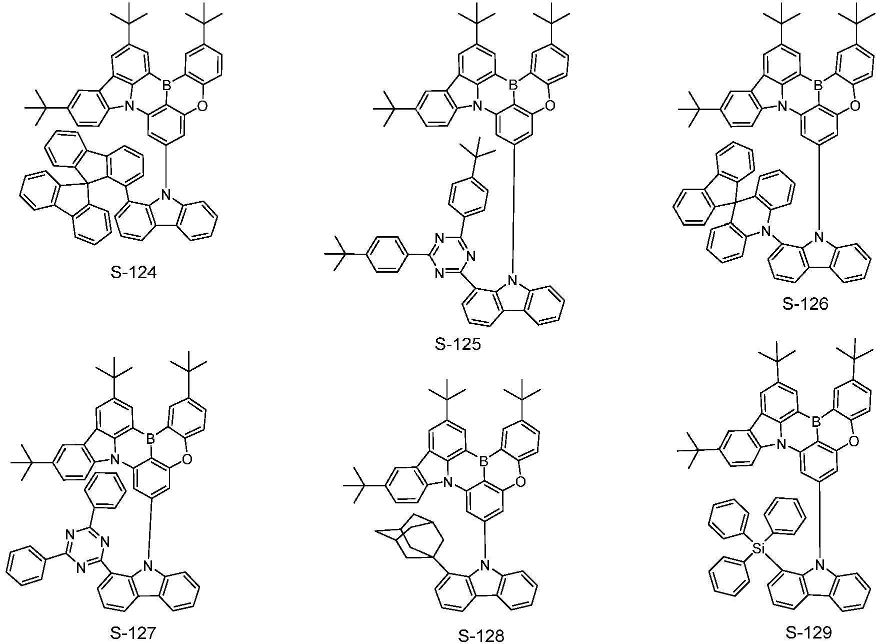

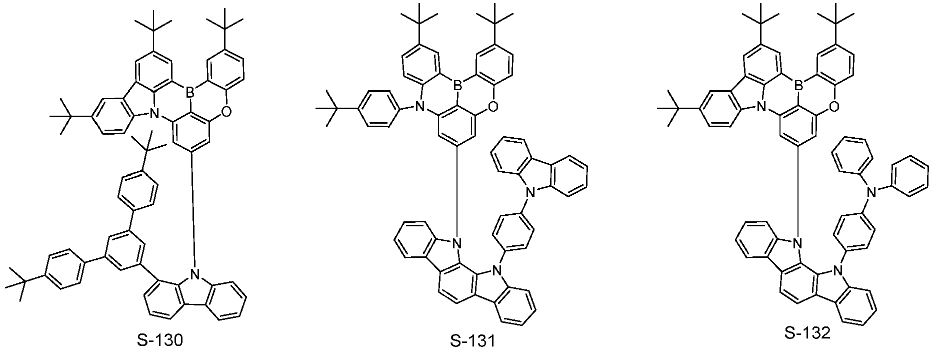

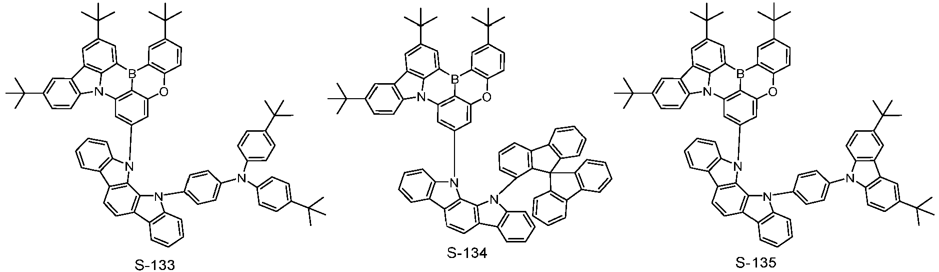

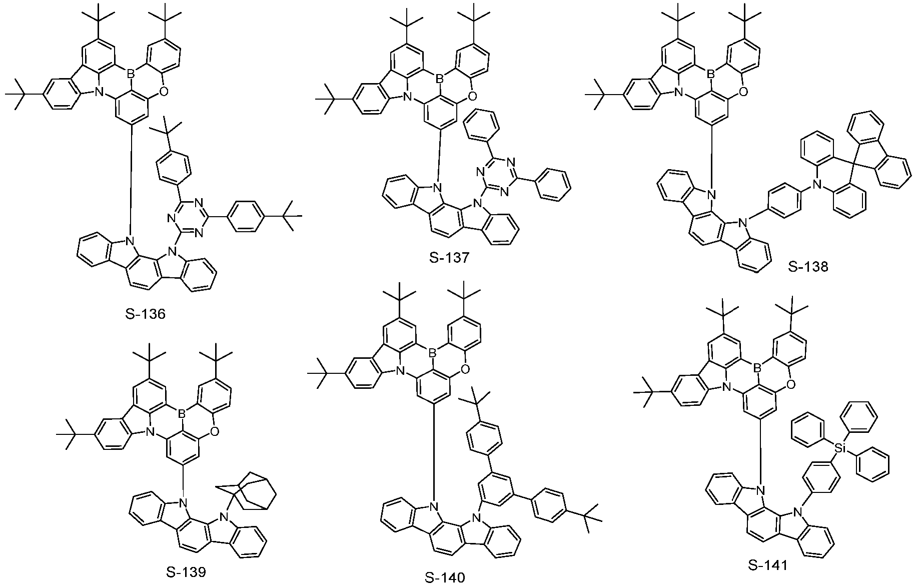

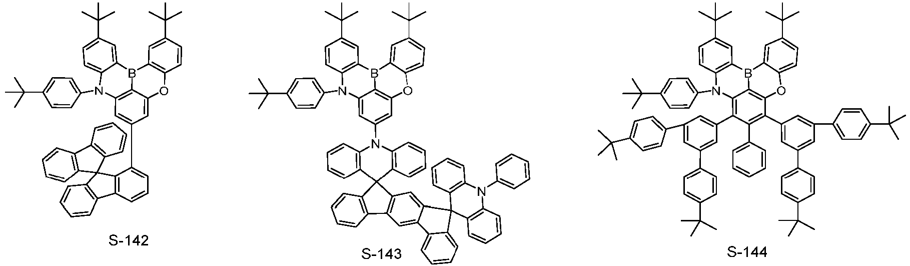

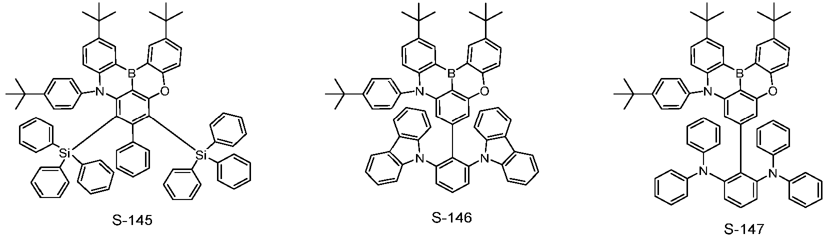

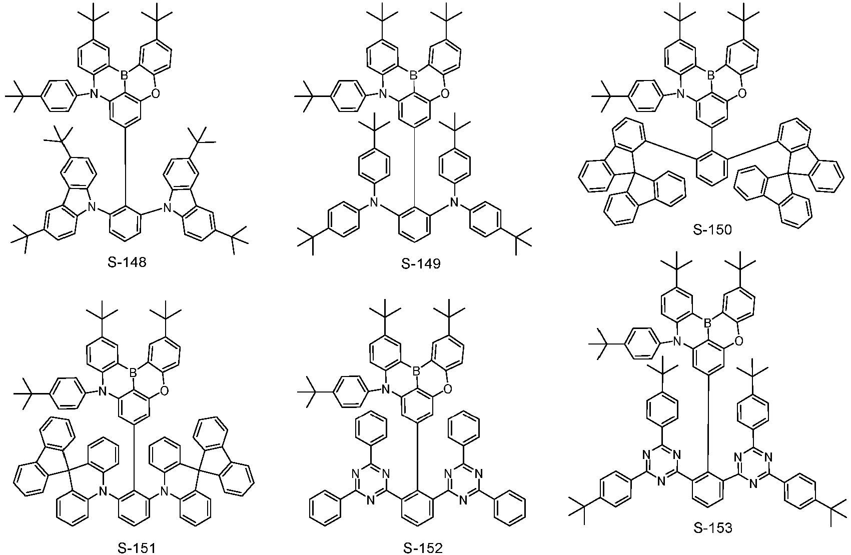

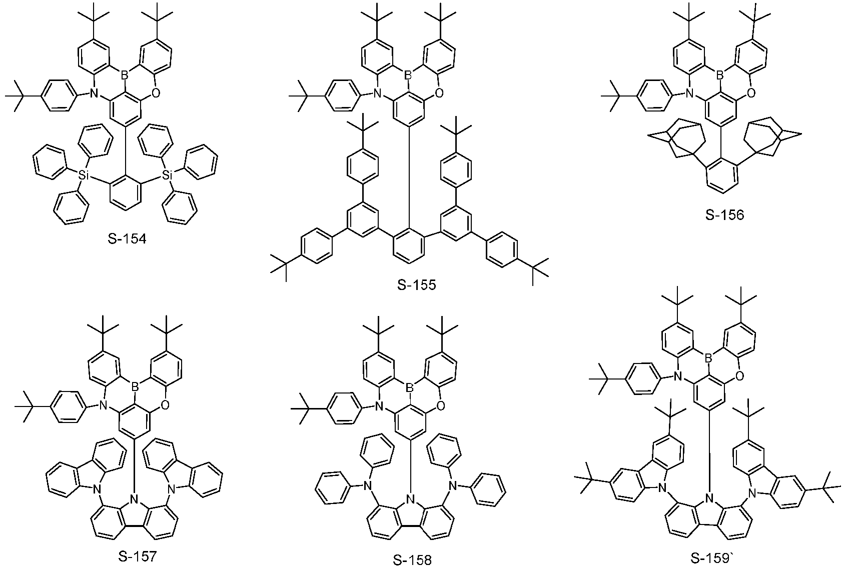

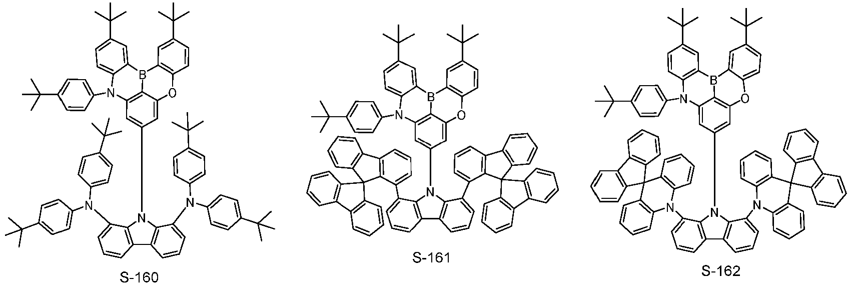

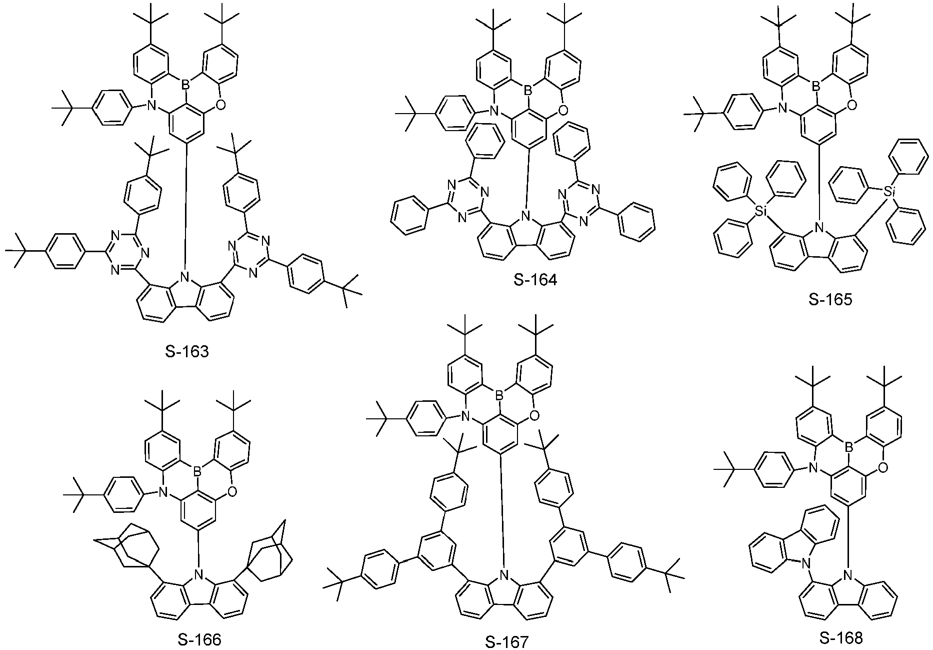

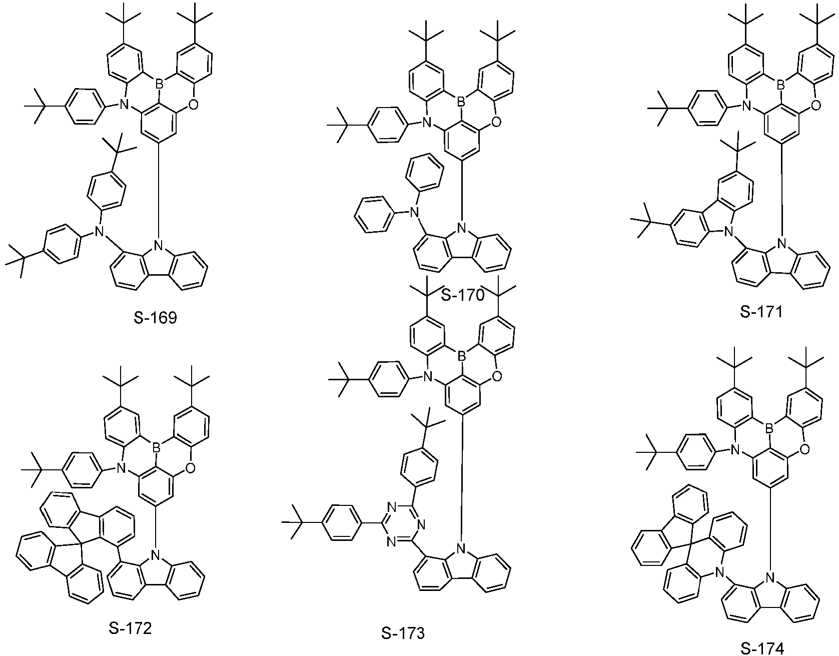

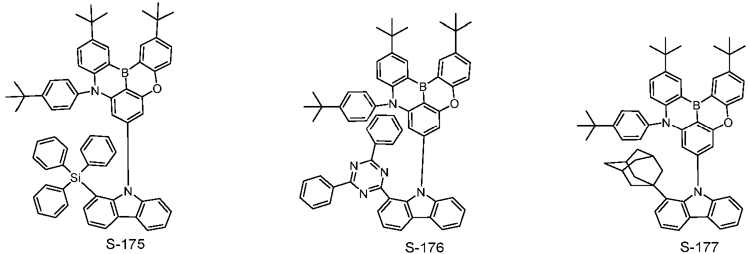

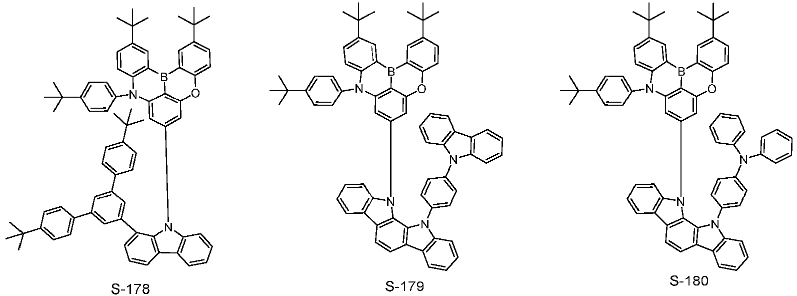

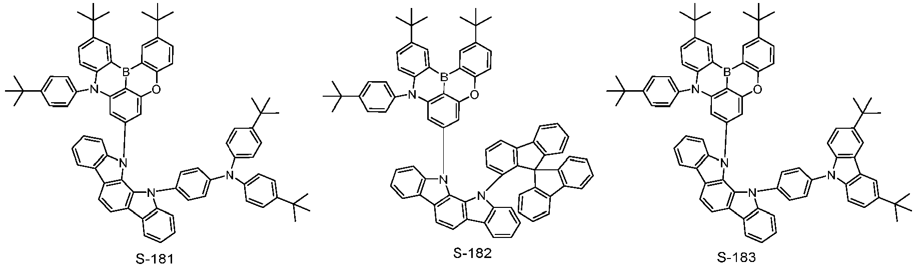

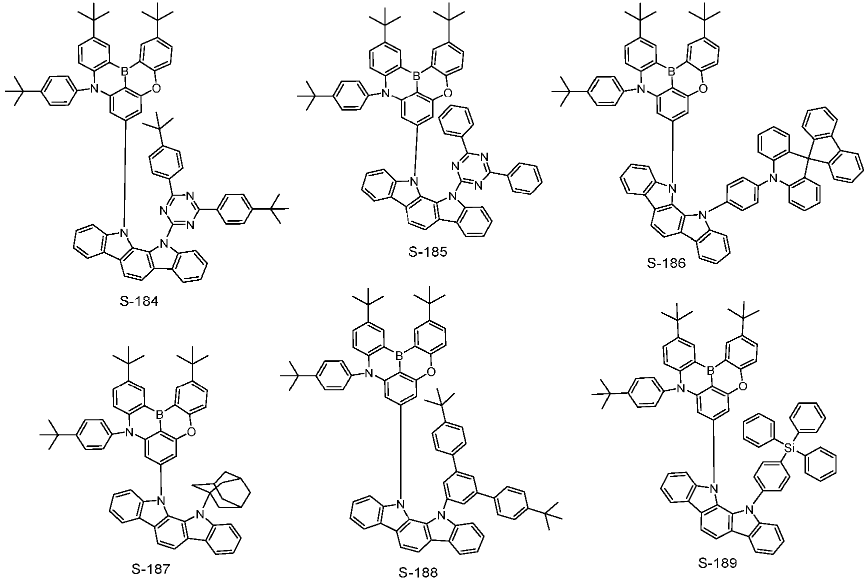

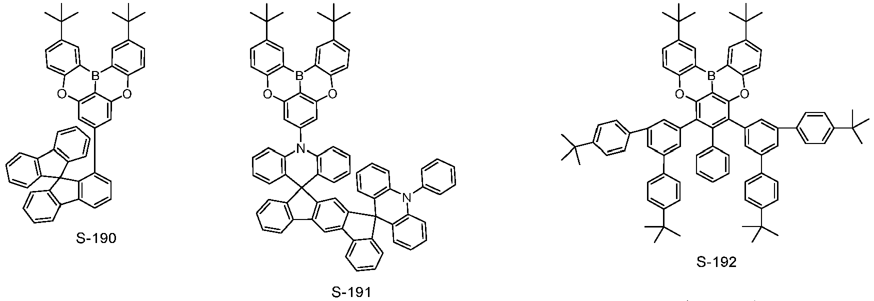

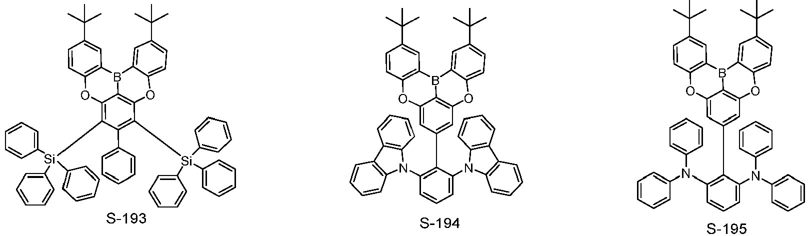

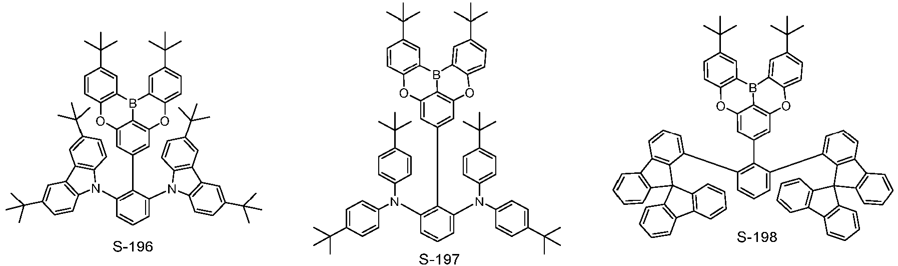

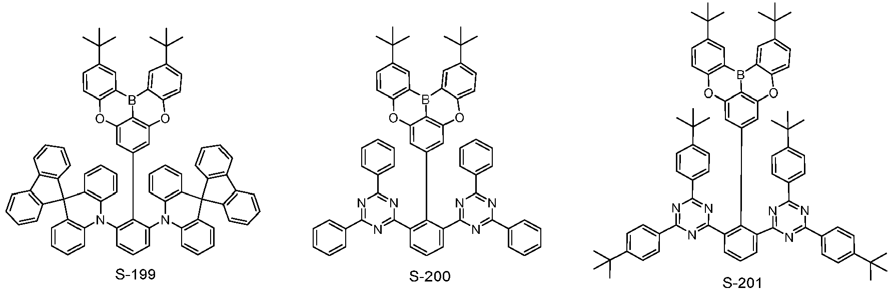

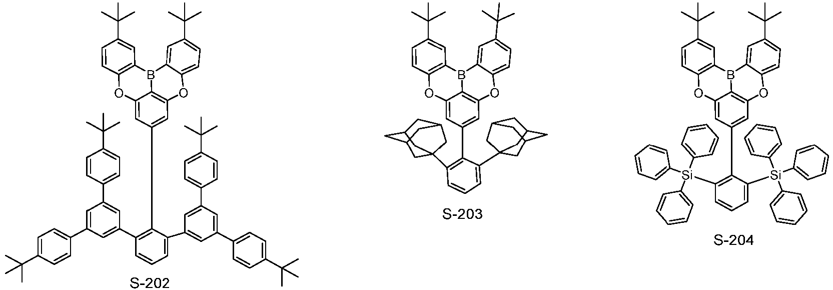

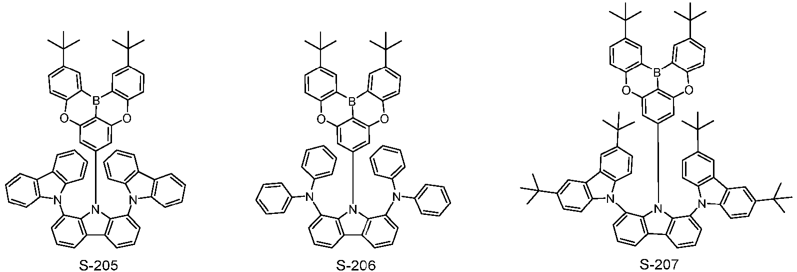

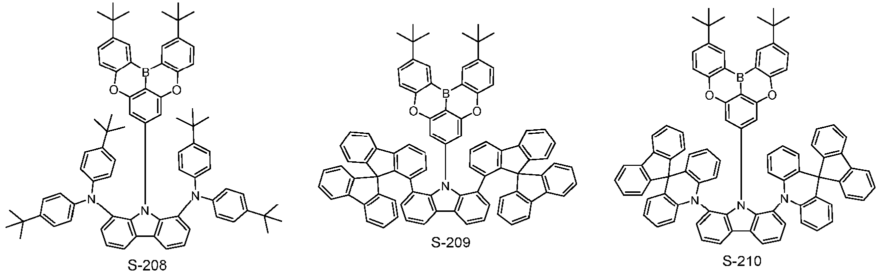

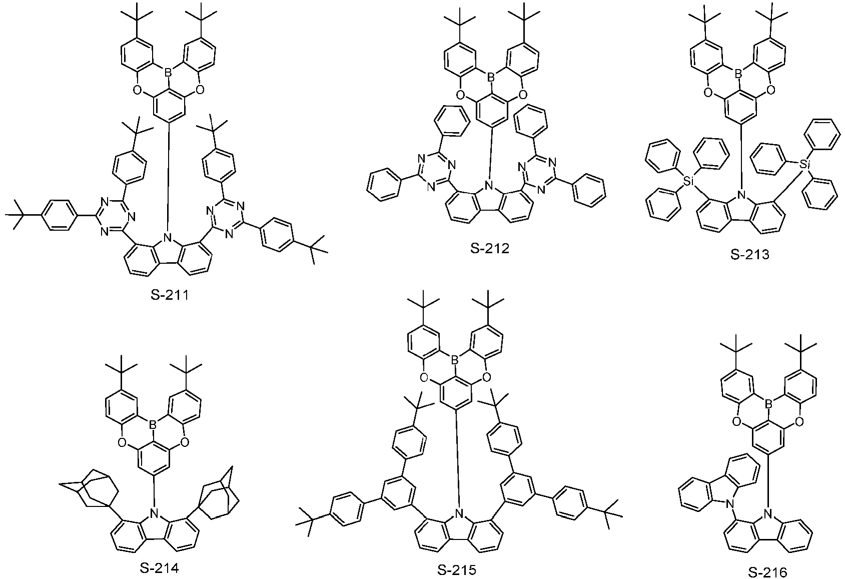

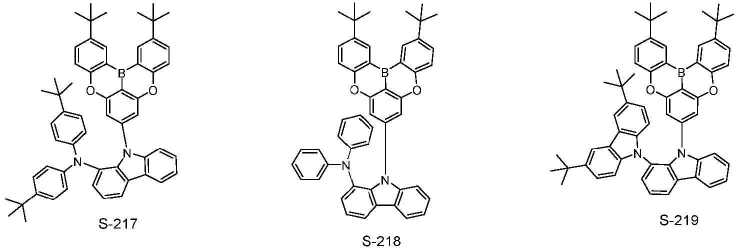

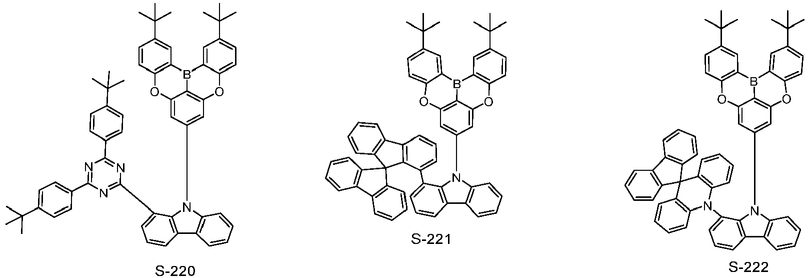

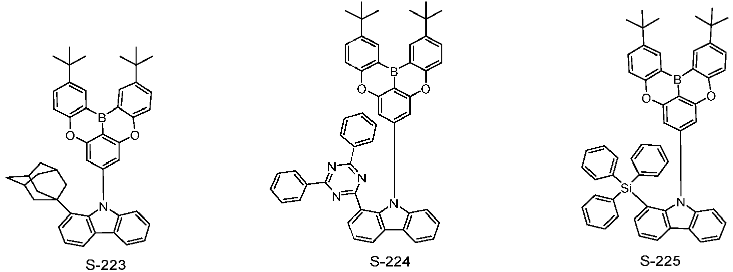

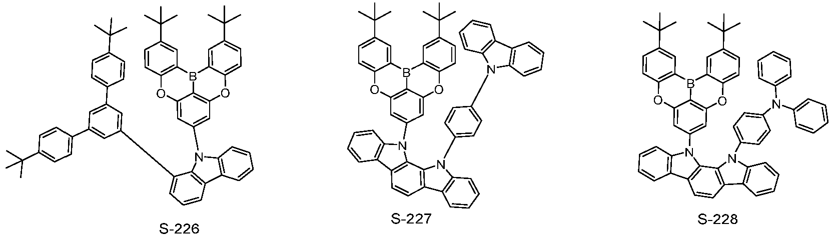

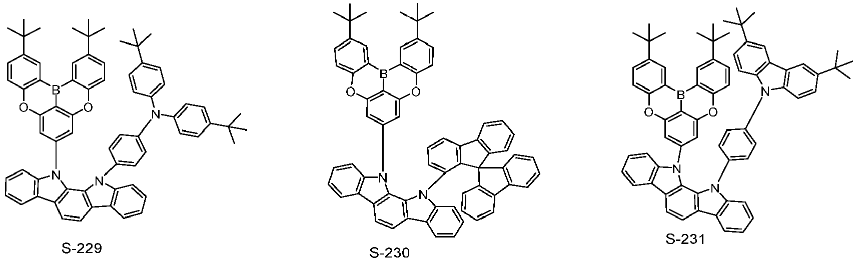

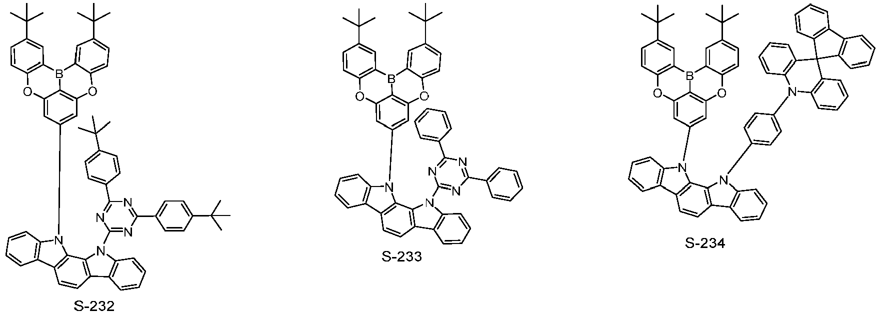

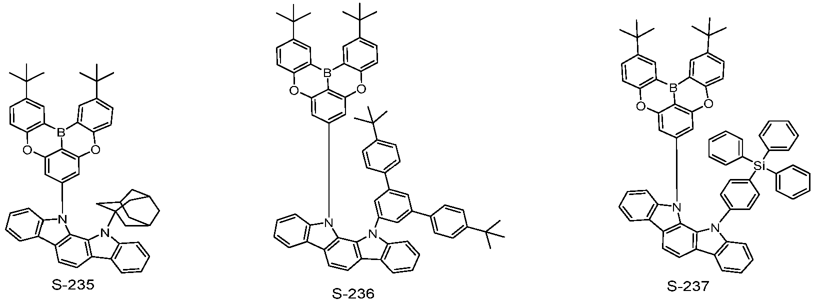

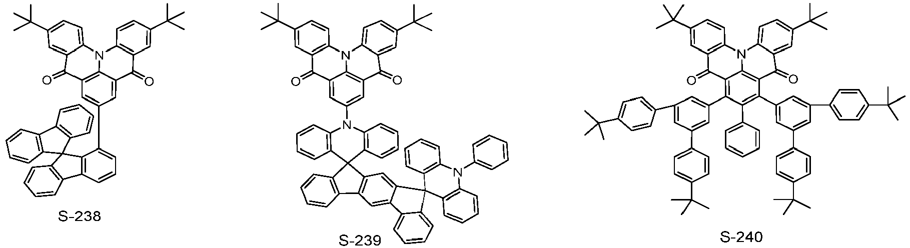

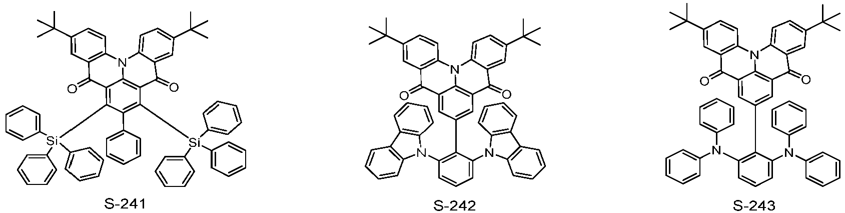

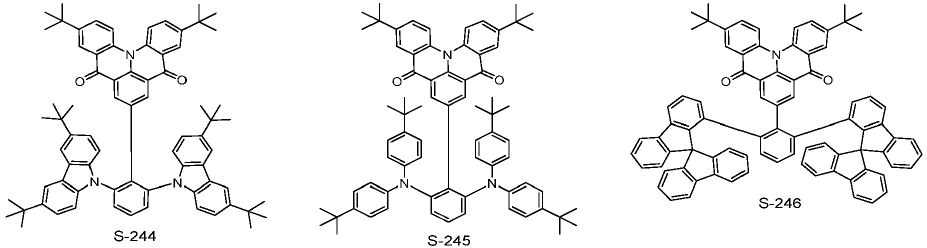

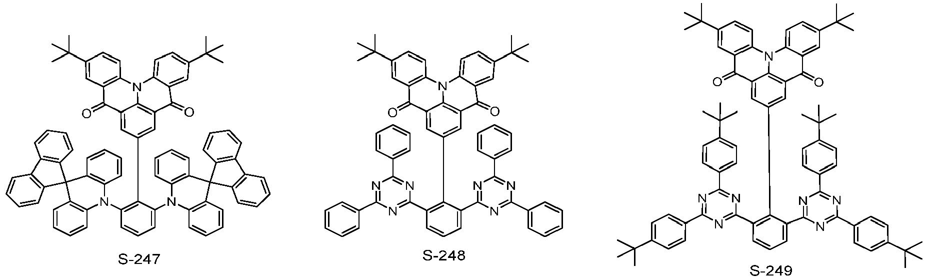

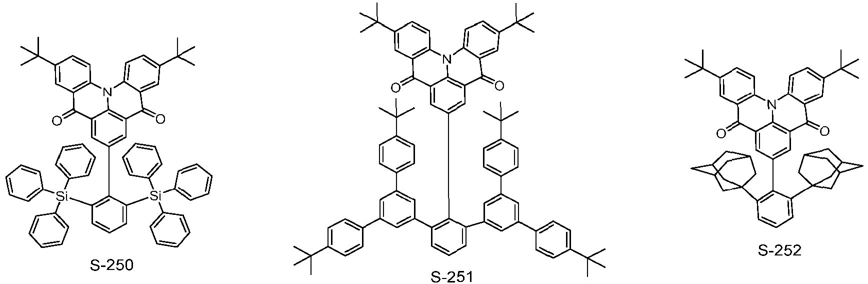

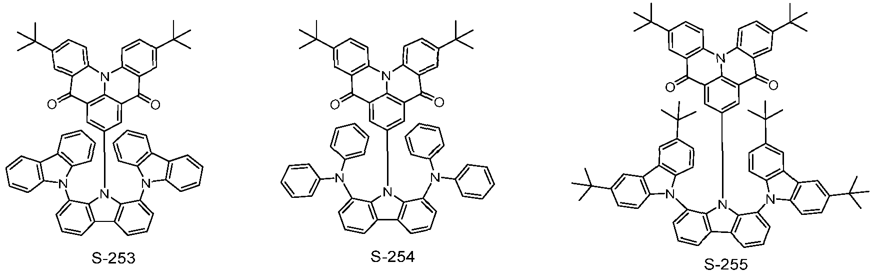

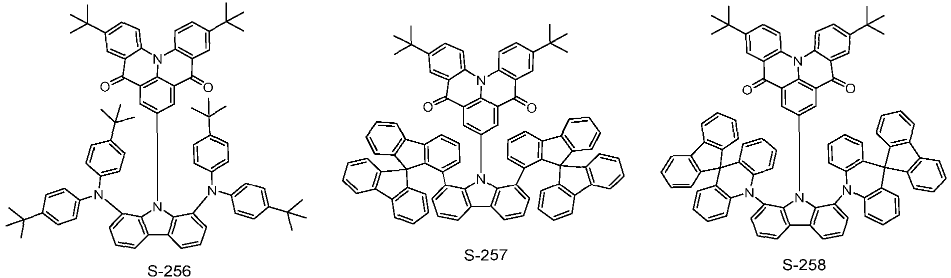

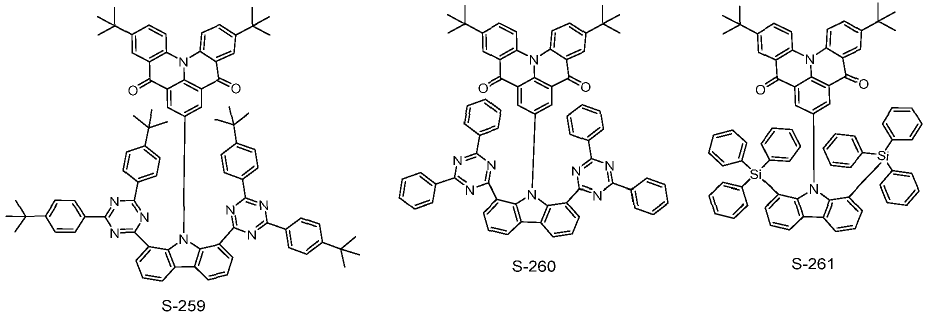

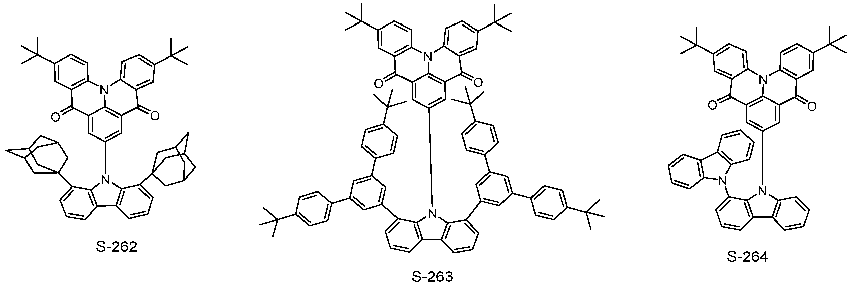

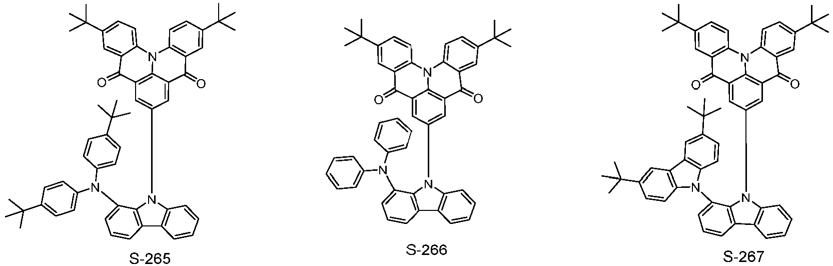

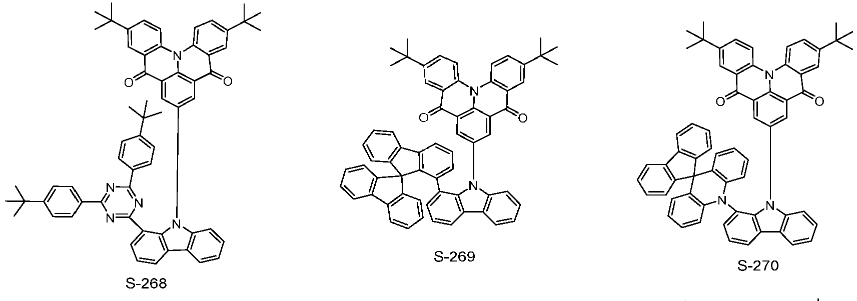

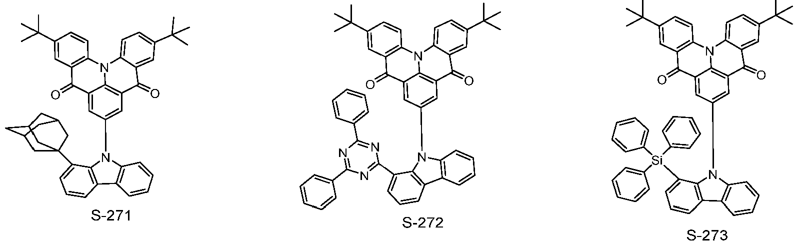

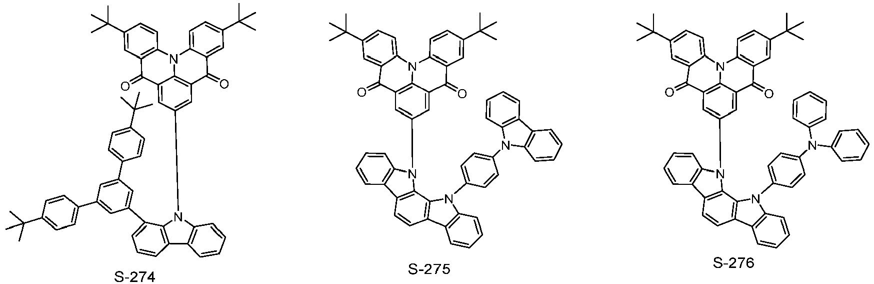

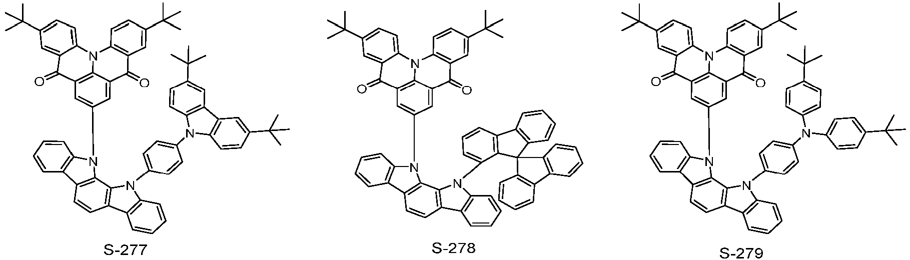

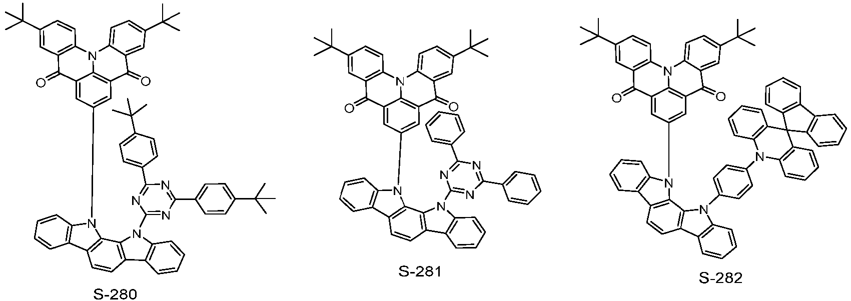

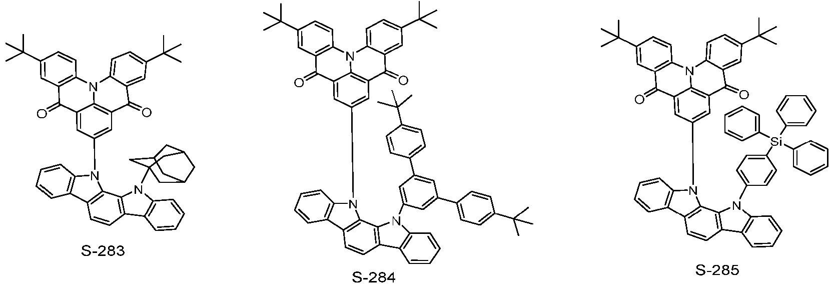

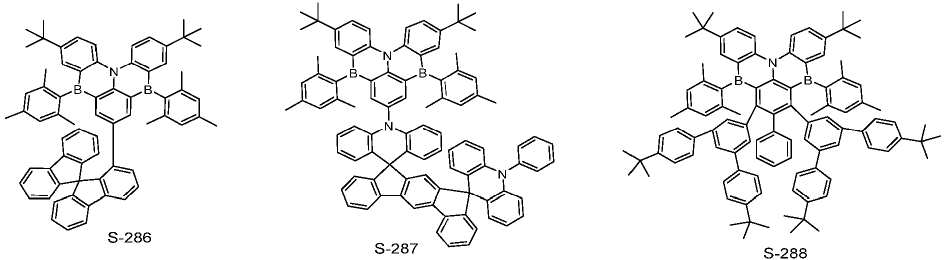

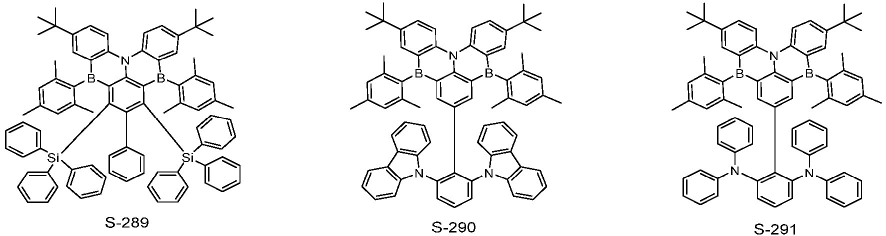

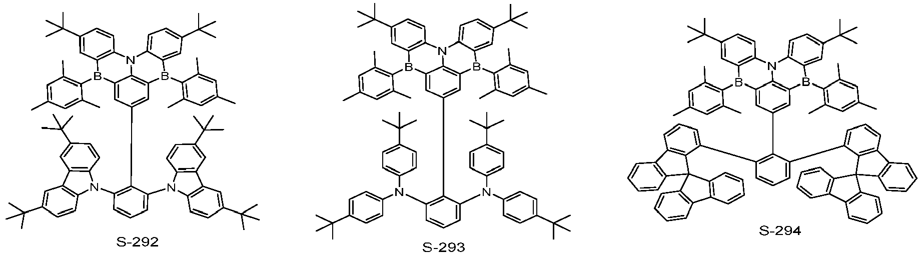

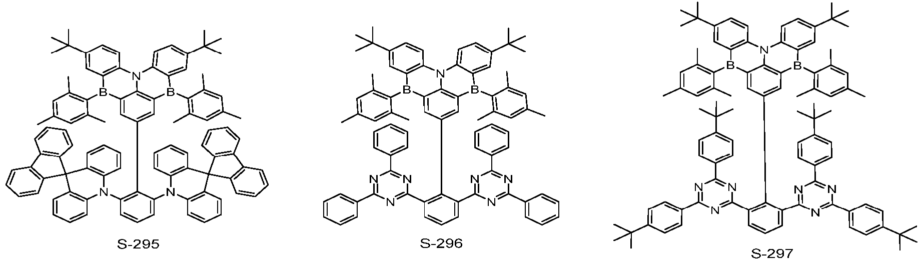

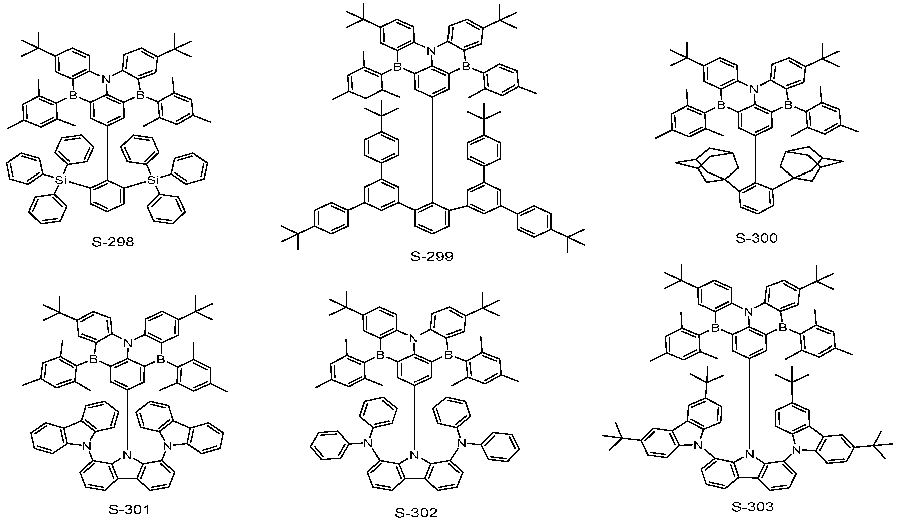

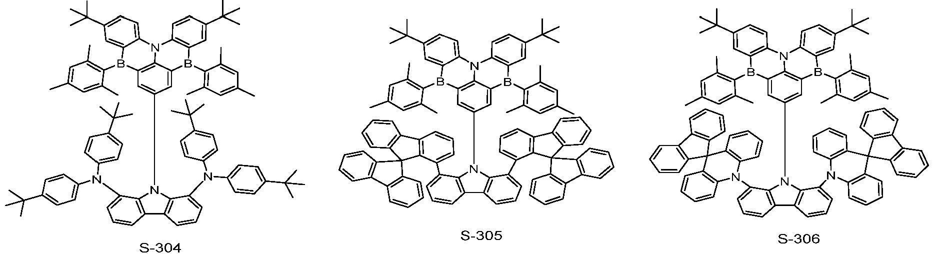

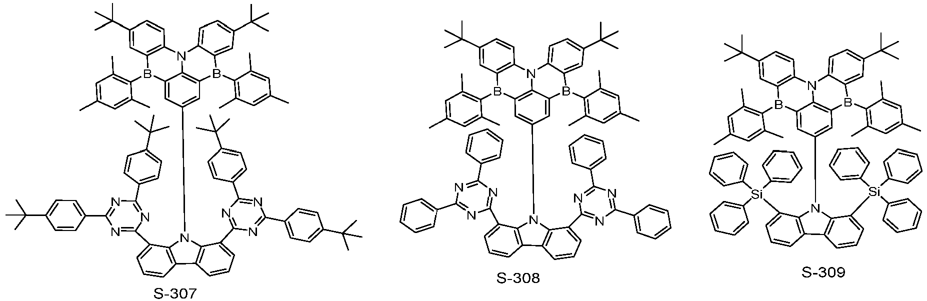

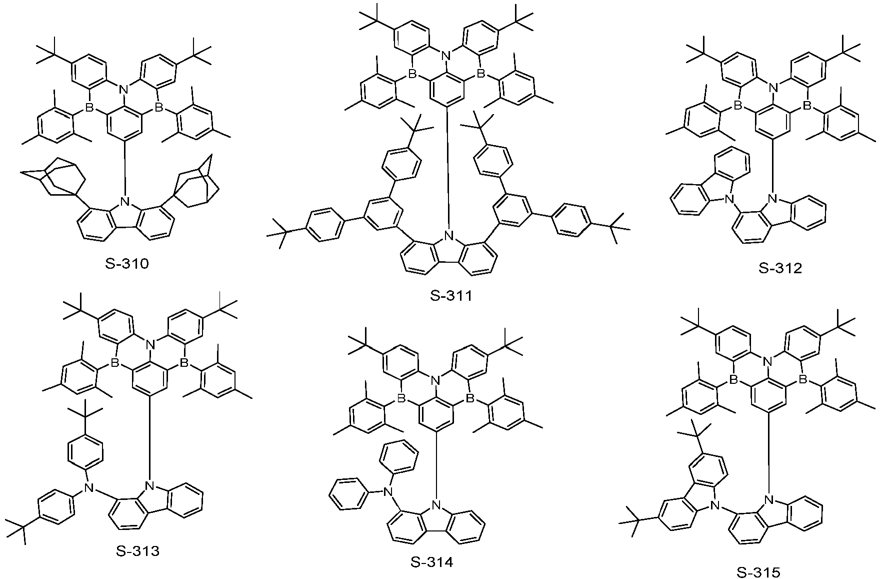

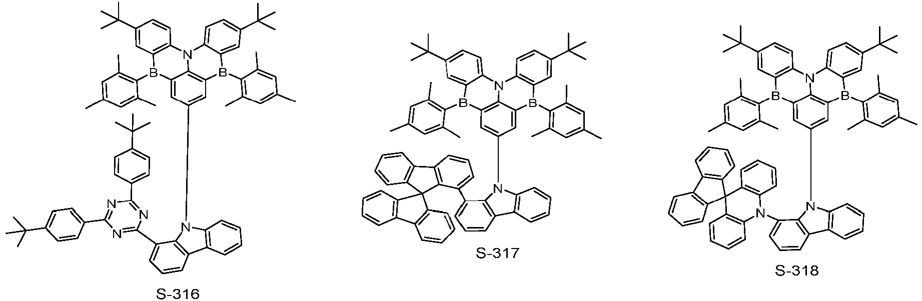

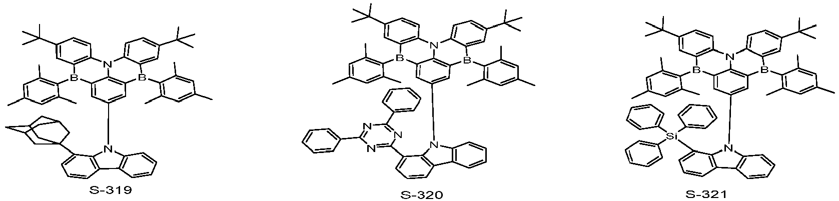

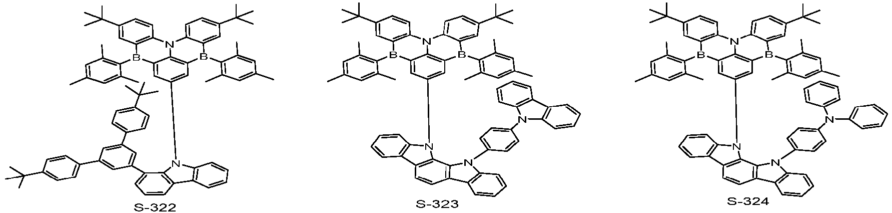

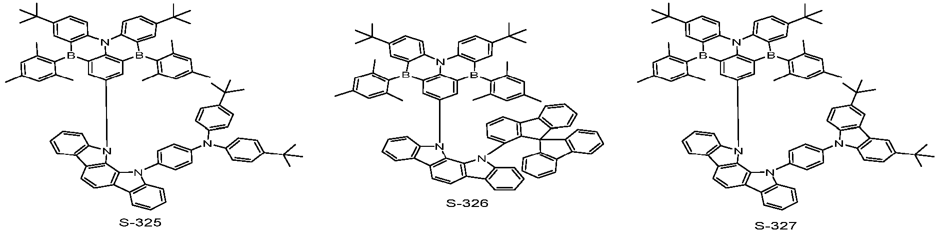

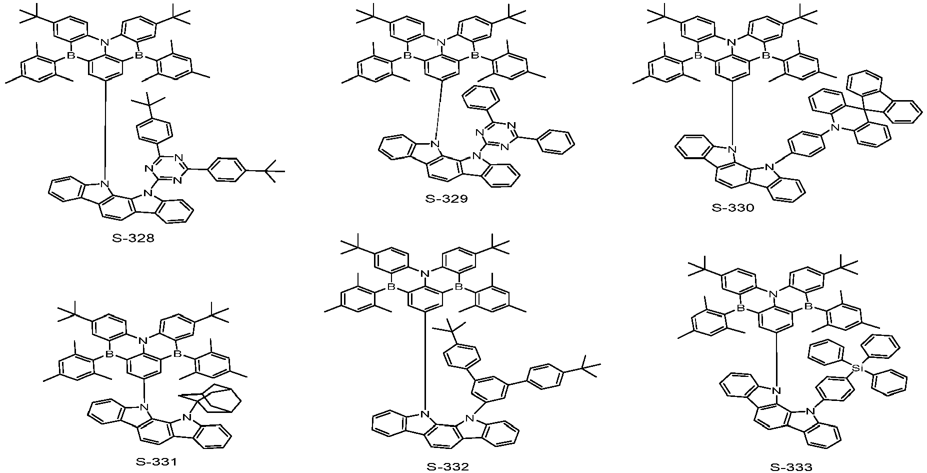

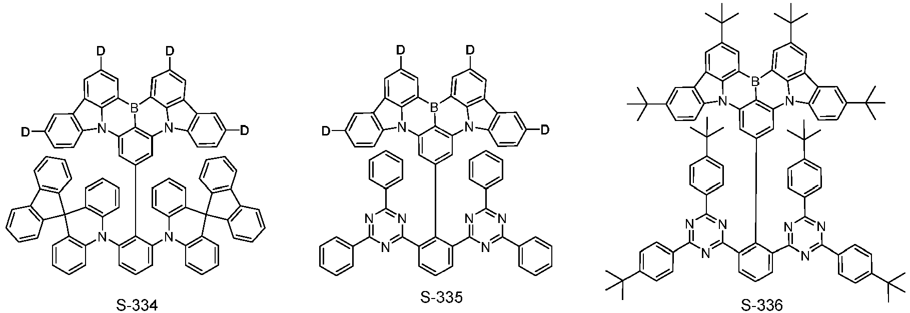

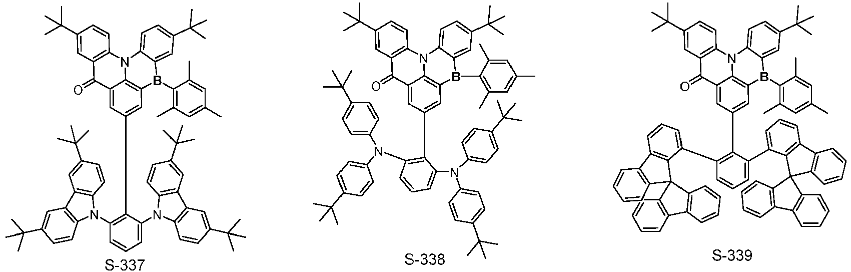

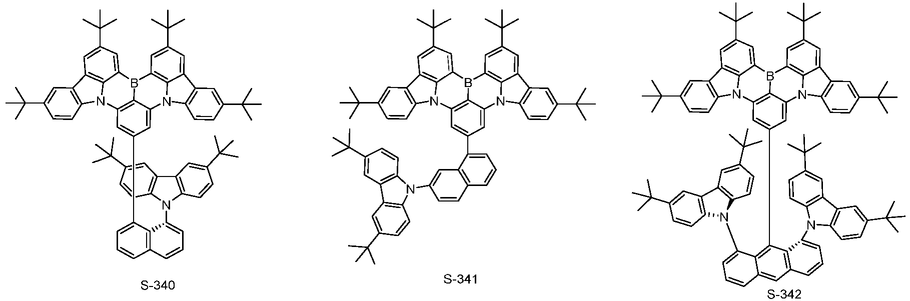

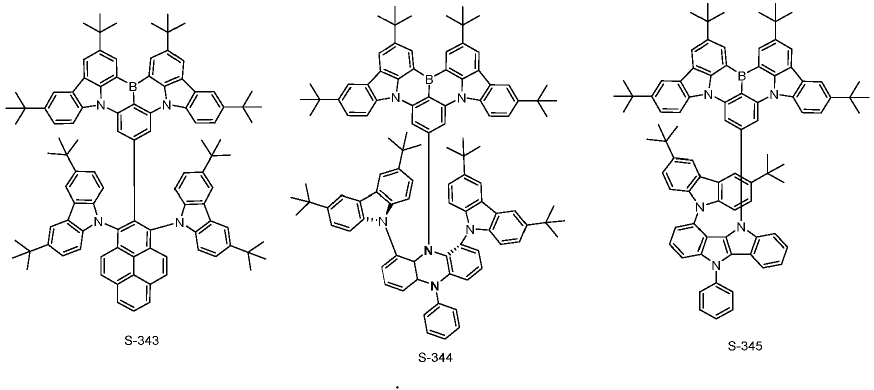

In the present application, there is no special limitation to the TTA host material in the light-emitting layer. Preferably, when the TTA host material is selected from at least one compound represented by BFH-1 to BFH-25, the performance of the organic electroluminescent device is improved more significantly. The compounds BFH-1-BFH-17 are not according to the invention and are present for illustration purposes only.

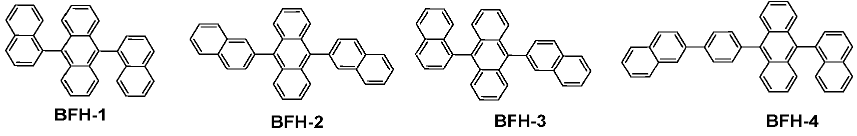

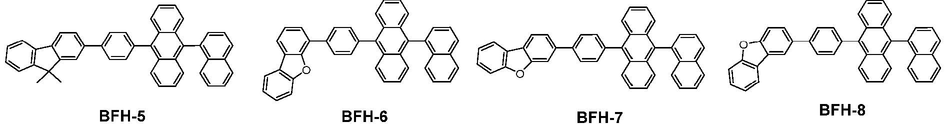

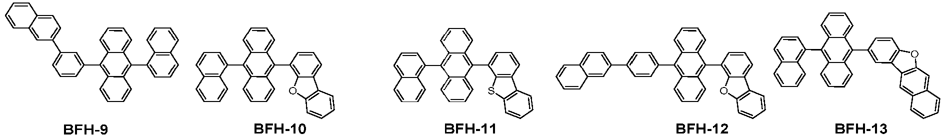

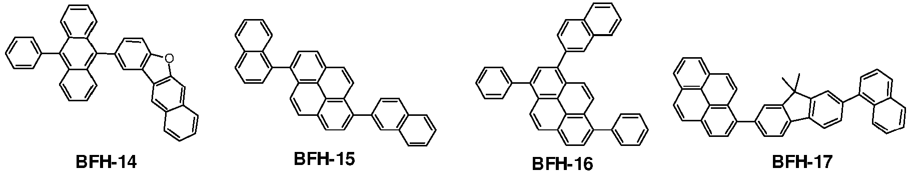

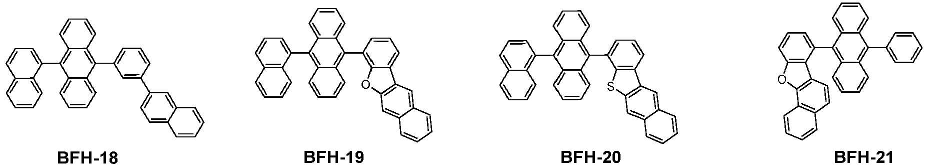

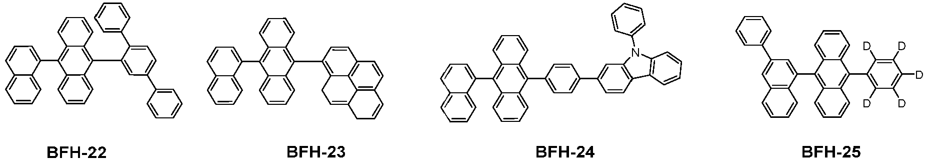

In a specific implementation of the present application, a mass proportion of the fluorescent dye in the light-emitting layer is generally controlled to be 0.1% to 50%. Reasonable controlling the doping amount of the dye in the light-emitting layer is beneficial for further improving the luminescence efficiency of the device. Of course, different host materials and dyes in the light-emitting layer of the organic electroluminescent device in the present application will affect the performance of the device. Therefore, in general, for different host materials and dyes, when the mass proportion of dyes in the light-emitting layer is controlled to 0.5%-20%, it can be basically ensured that the device has excellent luminescence efficiency.

The organic electroluminescent device of the present application has no special limitation to the thickness of the light-emitting layer, which is consistent with the thickness of the light-emitting layer of the existing device in the art, for example, 10-60nm.

Besides the light-emitting layer, the organic electroluminescent device of the present application further includes an anode located on one side of the light-emitting layer and a cathode located on the other side of the light-emitting layer, that is, the light-emitting layer is disposed between the cathode and the anode. The anode and cathode may employ materials commonly used in the art. For example, transparent conductive materials such as indium tin oxide (ITO), indium zinc oxide (IZO), stannic oxide (SnO₂), zinc oxide (ZnO) and other oxide and any combination thereof are used as the material for anode; metals or alloys such as magnesium (Mg), silver (Ag), aluminum (Al), aluminum-lithium (Al-Li), calcium (Ca), magnesium-indium (Mg-In), magnesium-silver (Mg-Ag) and any combination thereof are used as the material for cathode. Specifically, the cathode or anode can be formed by sputtering or depositing on a substrate as a corresponding material, the substrate is a glass or a polymer material with excellent mechanical strength, thermal stability, waterproofing effect, and transparency. In addition, thin film transistors (TFT) may also be provided on the substrate that is used for a display apparatus.

Further, in addition to the cathode, light-emitting layer and anode, the organic electroluminescent device of the present application further includes other auxiliary functional area that is conducive to inject and recombine carriers. For example, a hole transmission area is disposed between the anode and the light-emitting layer, and an electron transmission area is disposed between the cathode and the light-emitting layer.

Specifically, the hole transmission area can be a hole transmission layer (HTL) having a single layer structure, including a single-layer hole transmission layer containing only one compound and a single-layer hole transmission layer containing multiple compounds. In a direction of the anode pointing towards the light-emitting layer, the hole transmission area may also be a multi-layer structure that sequentially includes at least two layers of the hole injection layer (HIL), the hole transmission layer (HTL), and the electron blocking layer (EBL).

The material in the hole transmission area (including HIL, HTL and EBL) is selected from, but not limited to, a phthalocyanine derivative such as CuPc, a conductive polymer or a polymer containing a conductive dopant, such as polyphenylenevinylene, polyaniline/ dodecylbenzene sulfonic acid (Pani/DBSA), poly (3,4-ethylenedioxythiophene)/poly (4-styrene sulfonate)(PEDOT/PSS), polyaniline/camphorsulfonic acid(Pani/CSA), polyaniline/poly (4-styrene sulfonate)(Pani/PSS), and aromatic amine derivative.

Where, if the material of the hole transmission auxiliary layer is an aromatic amine derivative, it is one or more of the compounds represented by HT-1 to HT-34.

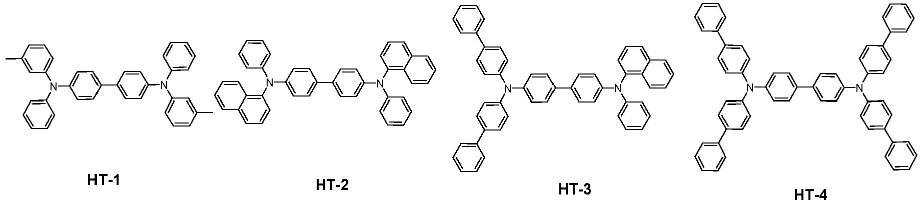

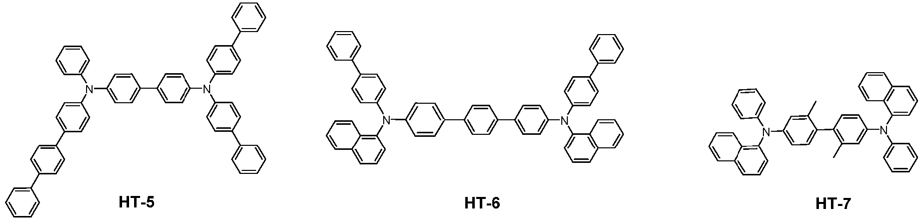

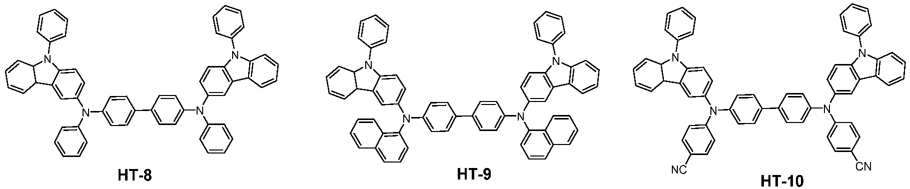

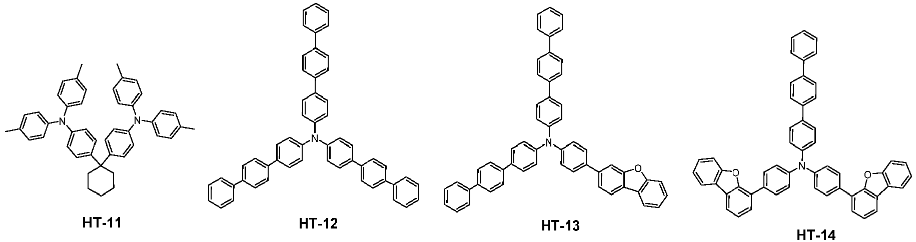

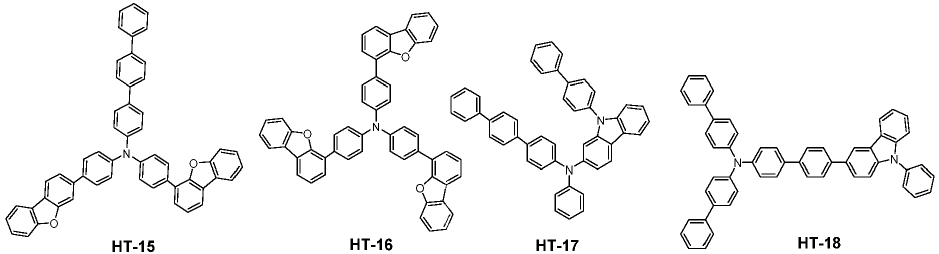

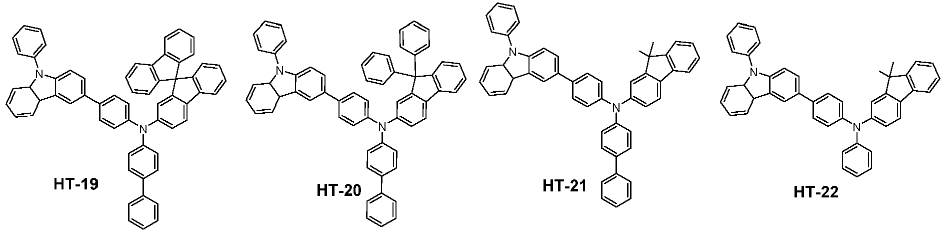

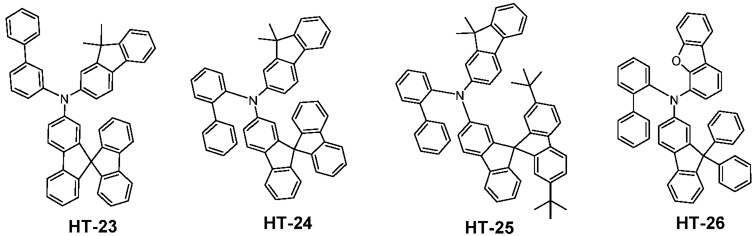

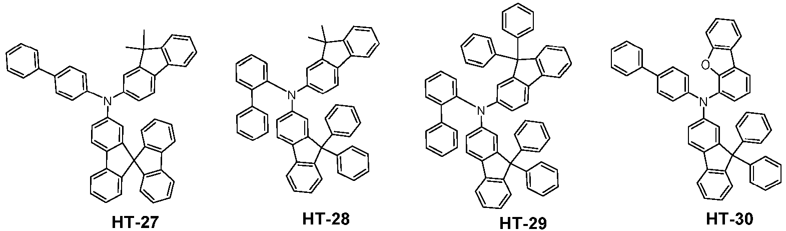

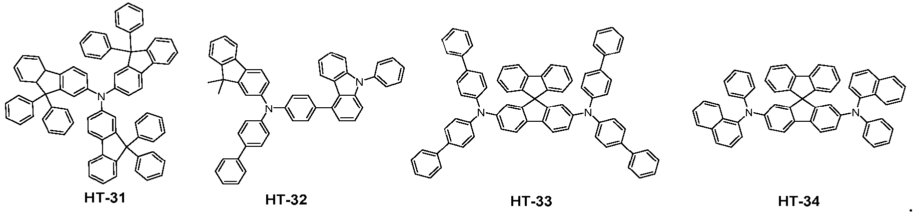

The hole injection layer is located between the anode and the hole transmission layer. The hole injection layer is a single compound material or a combination of multiple compounds. For example, the hole injection layer may employ one or more compounds of HT-1 to HT-34, or one or more compounds of HI1 to HI3; or one or more compounds of HT-1 to HT-34 that are doped with one or more of the following compounds of HI1 to HI3.

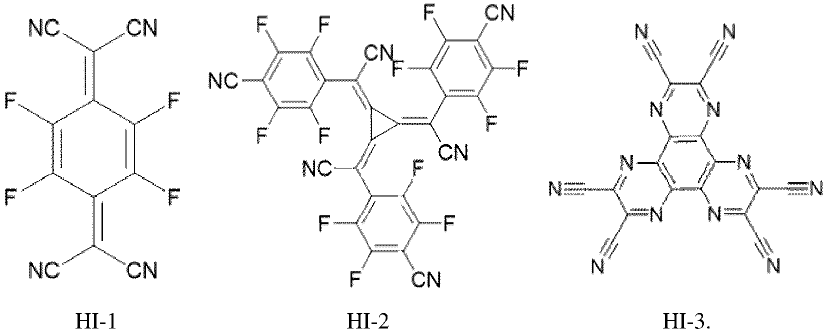

The electron transmission area is an electron transmission layer (ETL) with a single-layer structure, including a single-layer electron transmission layer containing only one compound and a single-layer electron transmission layer containing multiple compounds. In a direction of the cathode pointing towards the light-emitting layer, the electron transmission area may also be a multi-layer structure including at least two of the electron injection layer (EIL), the electron transmission layer (ETL), and the hole blocking layer (HBL).

The material of the electronic transmission layer is selected from, but not limited to, one or more from the following ET-1 to ET-73.

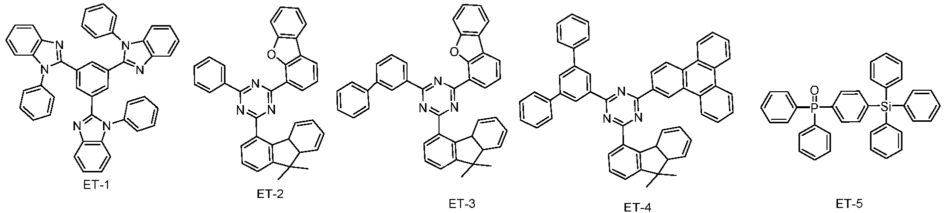

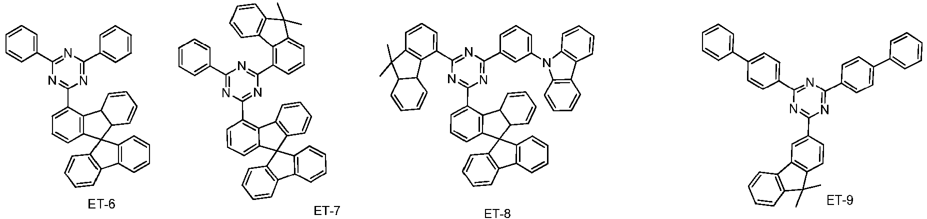

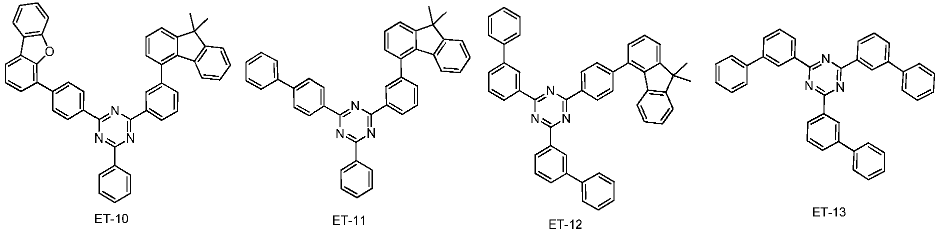

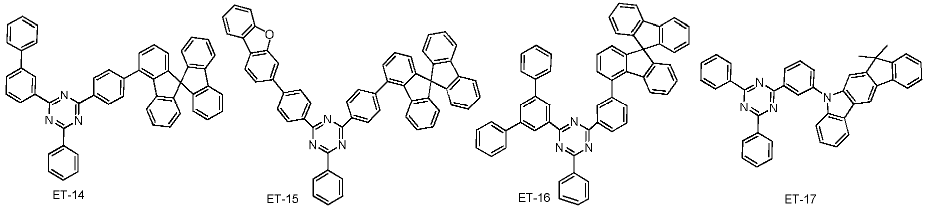

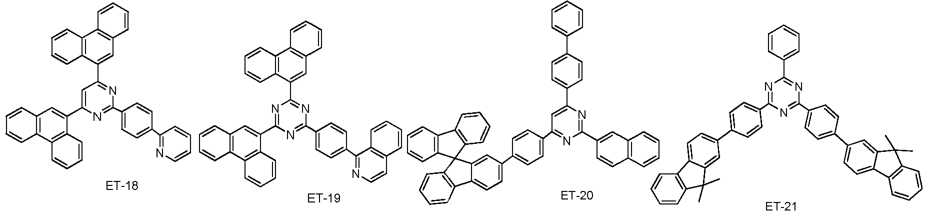

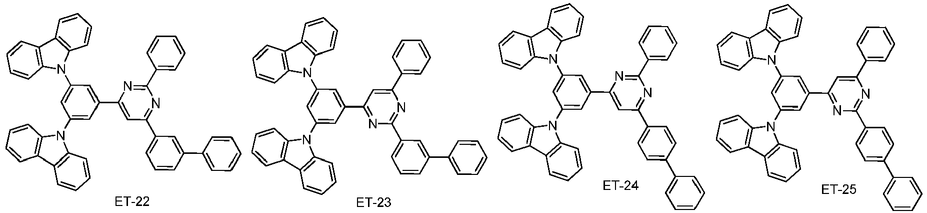

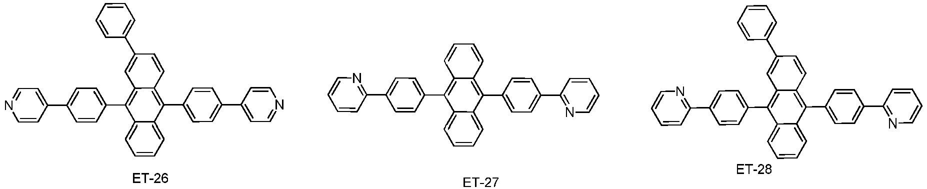

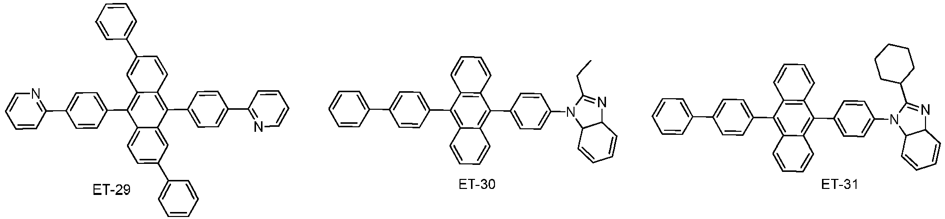

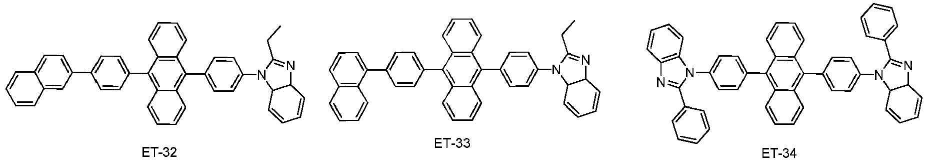

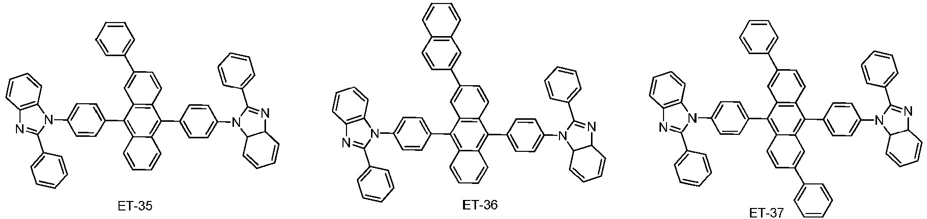

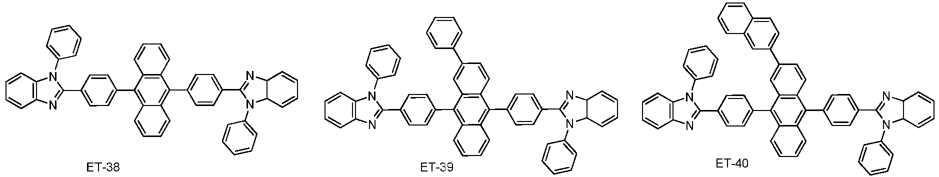

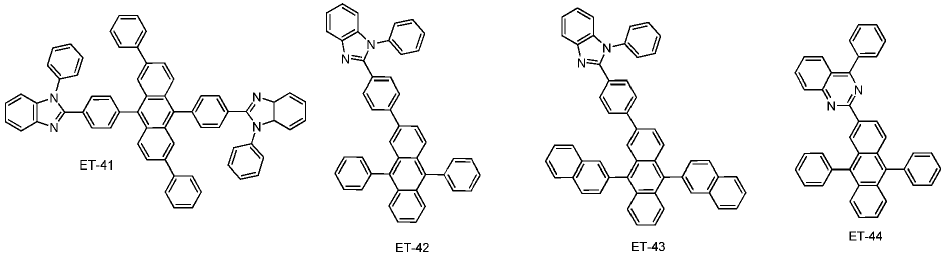

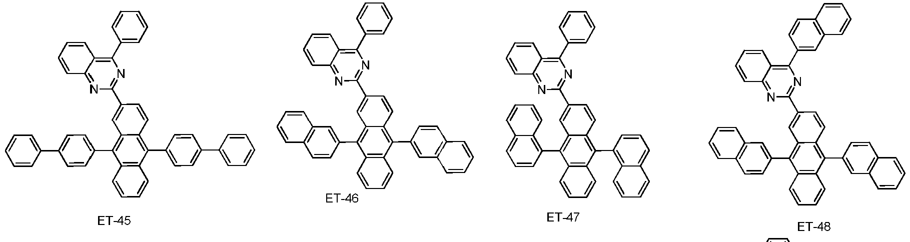

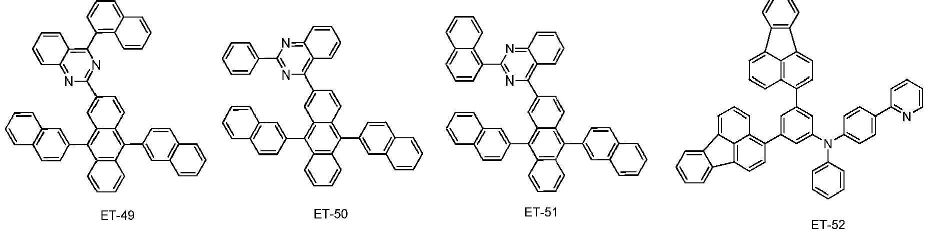

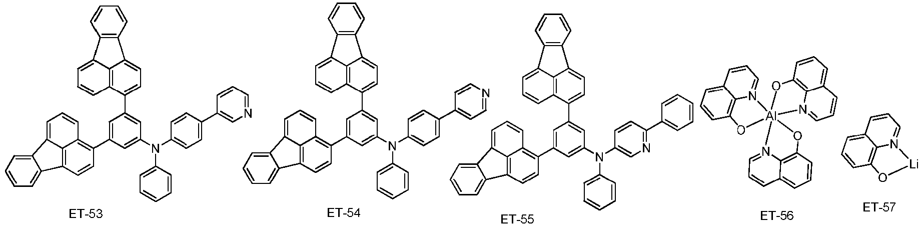

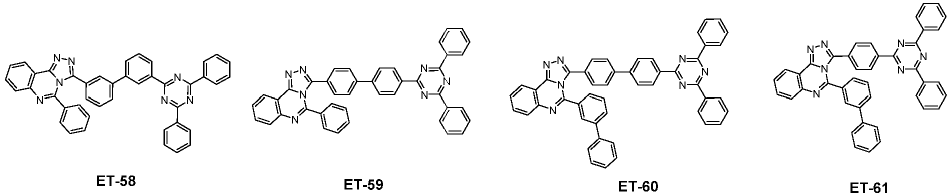

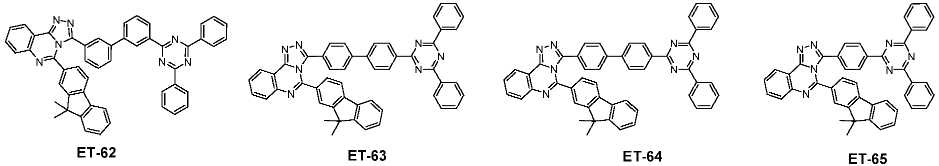

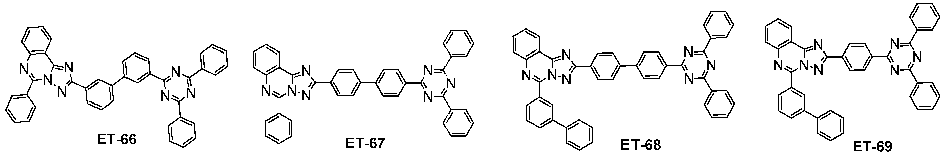

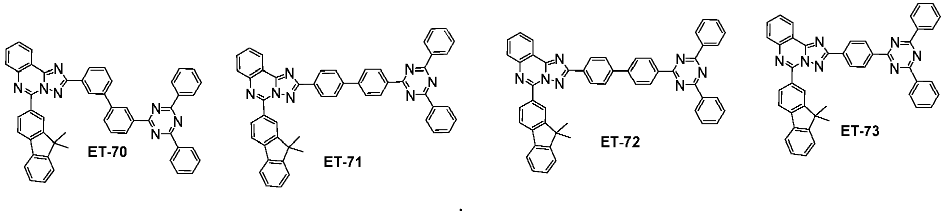

The hole barrier layer (HBL) is located between the electron transmission layer and the light-emitting layer. The hole blocking layer can use, but is not limited to, one or more compounds of the above ET-1 to ET-73.

The electron injection material in the electron injection layer includes any one or at least two from the following compounds: Liq, LiF, NaCl, CsF, Li₂O, Cs₂CO₃, BaO, Na, Li, Ca, Mg, Ag, and Yb.

A capping layer (CPL layer) is deposited via evaporation on the cathode of the device to improve the efficiency of the device and adjust the optical microcavity, etc.

The thickness of the above respective layers can be the conventional thickness of these layers in the art.

The present application further provides a preparation method of the organic electroluminescent device, including depositing an anode, a hole transmission area, a light-emitting layer, an electron transmission area, and a cathode on a substrate in sequence, and then sealing. In the preparation of the light-emitting layer, the evaporation speed of the host material and the evaporation speed of the fluorescent dye are adjusted by a method of multi-source co-evaporation to make the fluorescent dye reach a preset doping ratio, and the light-emitting layer is formed by a method of co-evaporation of the triplet-triplet annihilation material source and any one of the fluorescent dye sources mentioned above. The deposition methods of the anode, hole transmission area, electron transmission area, and cathode are the same as those existing methods in the art.

The organic electroluminescent device of the present application has the advantages of low driving voltage and high efficiency through the matching of specific materials of the light-emitting layer and the selection of special fluorescent dye.

A second aspect of the present application further provides a display apparatus, including the above organic electroluminescent device. The display apparatus may specifically include an OLED display and other display device, as well as any product or component with a displaying function such as televisions, digital cameras, mobile phones, tablets, etc. that includes the display apparatus. The display apparatus has the same advantages as the above organic electroluminescent device compared with the prior art, which will not be described here.

The following will take multiple synthesis Examples as examples to elaborate the preparation method of a specific compound of the fluorescent dye of the present application, but the preparation method of the dye of the present application is not limited to these synthesis Examples.

Various chemicals used in the synthesis process such as petroleum ether, tert-butylbenzene, ethyl acetate, sodium sulfate, toluene, dichloromethane, potassium carbonate, boron tribromide, N,N-diisopropylethylamine, reaction intermediate and other basic chemical raw materials are purchased from Shanghai Titan Technology Co., Ltd. (Titan) and Xilong Chemical Co., Ltd. The mass spectrometer used to determine the following compounds is ZAB-HS mass spectrometer (manufactured by UK Micromass company).

The synthetic method of the dye compound in the present application is briefly described below. Firstly, hydrogen and Cl atoms between/on X₁, X₂, X₃ and X₄ are ortho-metallized with N-butyl lithium or tert-butyl lithium. Then, after boron tribromide is added for lithium-boron metal exchange, the Bronsted base such as N, N-diisopropylethylamine is added, and the Tandem Bora-Friedel-Crafts Reaction is performed to obtain a target.

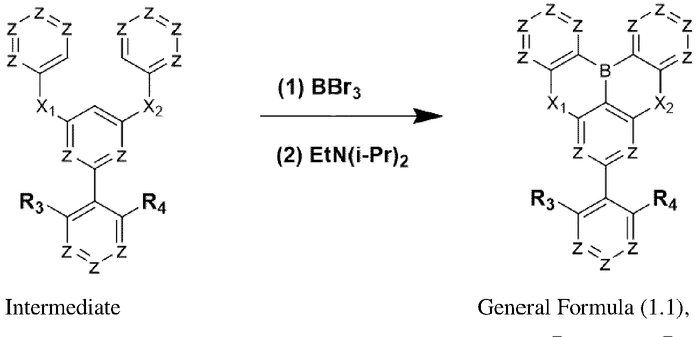

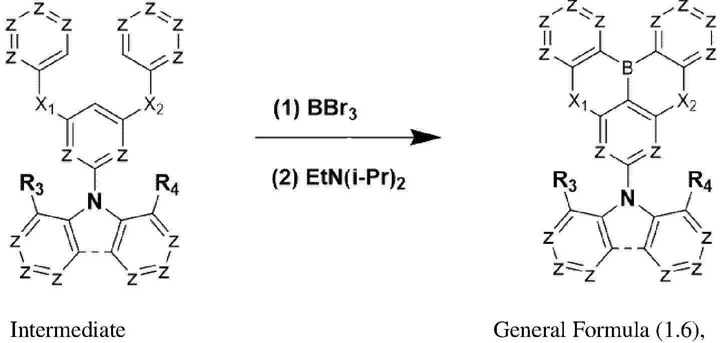

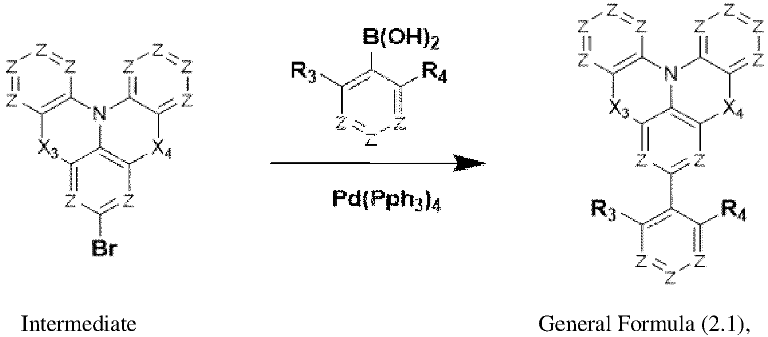

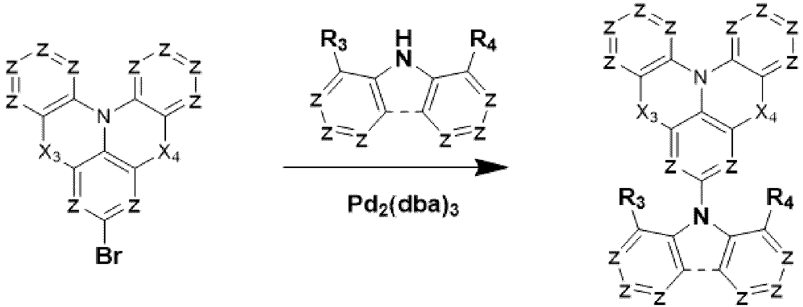

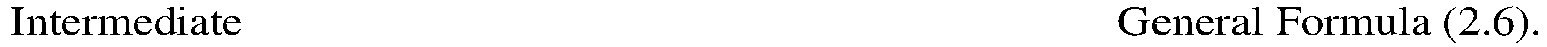

More specifically, a synthesis method for representative and specific fluorescent dye compounds in the present application is provided below. The following Synthesis Examples 1-4 are not according to the invention and are present for illustration purposes only.

### Synthesis Example 1: Synthesis of Compound S-7

1. Synthesis of compound S-7-2: in a three-necked bottle, under nitrogen protection, 0.01mol of S-7-1, 0.025mol of 3,6-di-tert-butylcarbazole and 150ml of toluene were added and mixed under stirring, and then 5×10⁻⁵ mol Pd₂(dba)₃ and 0.03mol sodium tert-butoxide were added for reflux reaction for 12 hours, followed by sampling and dropping on a plate, when if there was no bromine residue, the reaction was complete; the obtained reaction product was naturally cooled to room temperature and filtered, the filtrate was spin-evaporated to no fraction, and then purified through a neutral silica gel column (developing agent: dichloromethane and petroleum ether) to give a target compound S-7-2 (9.22g; yield: 73%; HPLC analysis purity: 99.56%) as a white powder. 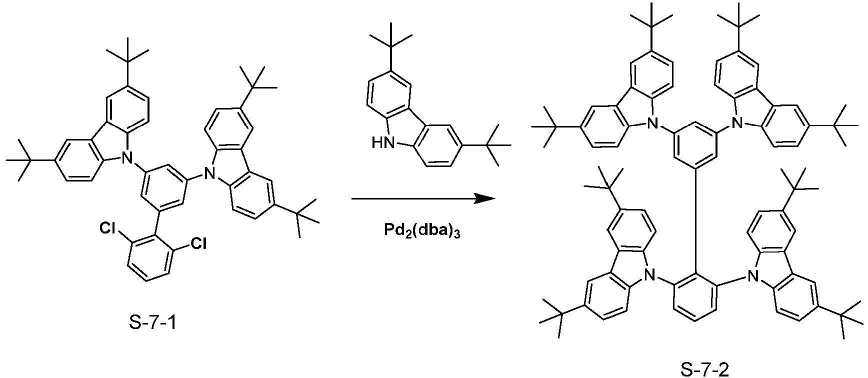
2. Synthesis of compound S-7: under nitrogen atmosphere, 0.03mol of BBr₃ was added into a solution of 0.01mol of S-7-2 in o-dichlorobenzene (100mL), and subjected to reaction at 190°C for 24 hours. The solvent after reaction was spin-dried under vacuum, followed by purification through a silica gel column (developing agent, ethyl acetate: petroleum ether=50:1), giving a target compound S-7 (0.64g; yield: 5%; HPLC analysis purity: 99.42%) as a green solid. MALDI-TOF-MS result: molecular-ion peak: 1271.55, element analysis result: theoretical value (%): C, 86.90; H, 7.85; B, 0.85; N, 4.41; experimental value (%): C, 86.80; H, 7.85; B. 0.85; N, 4.51.

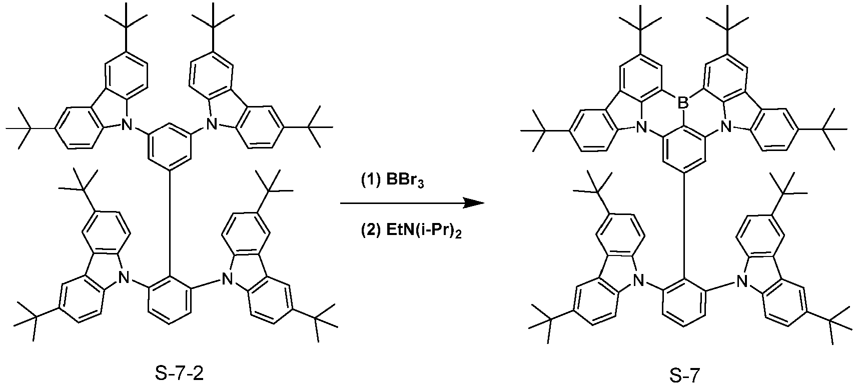

### Synthesis Example 2: Synthesis of Compound S-13

1. Synthesis of compound S-13-2: this Example was basically the same as the that of compound S-7-2, except that in this Example, S-7-1 was replaced by S-13-1 with an equal amount of substance. A target compound S-13-2 (10.38g; yield: 92%; HPLC analysis purity: 99.37%) as a white solid was obtained. 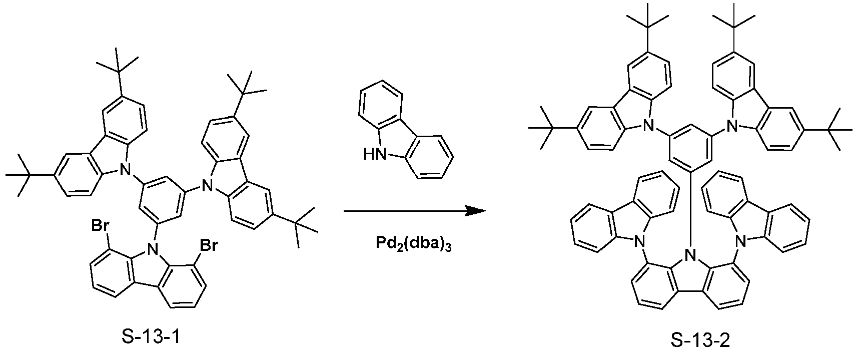
2. Synthesis of compound S-13: this Example was basically the same as that of compound S-7, except that in this Example, S-7-2 was replaced by S-13-2 with an equal amount of substance. A target compound S-13 (2.38g; yield: 21%; HPLC analysis purity: 99.33%) as a green solid was obtained. MALDI-TOF-MS result: molecular-ion peak: 1135.62, element analysis result: theoretical value (%): C, 86.68; H, 6.21; B, 0.95; N, 6.16; experimental value (%): C, 86.78; H, 6.31; B, 0.96; N, 6.15.

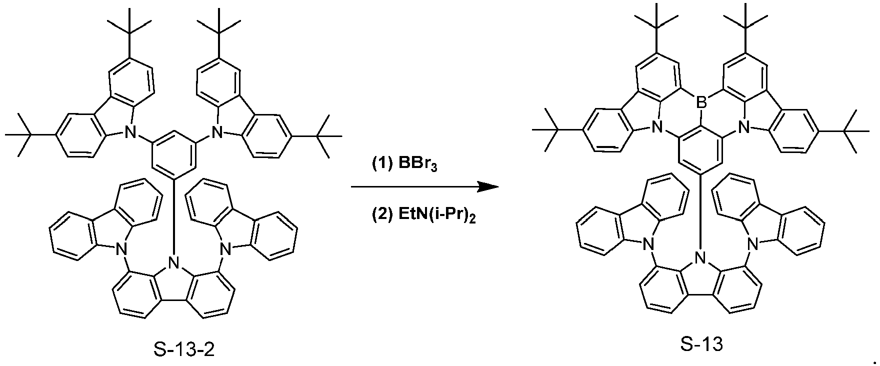

### Synthesis Example 3: Synthesis of Compound S-52

1. Synthesis of compound S-52-2: this Example was basically the same as that of compound S-7-2, except that S-7-1 was replaced by S-52-1 with an equivalent amount of substance. A target compound S-52-2 (10.89g; yield: 86%; HPLC analysis purity: 99.53%) as a white solid was obtained. 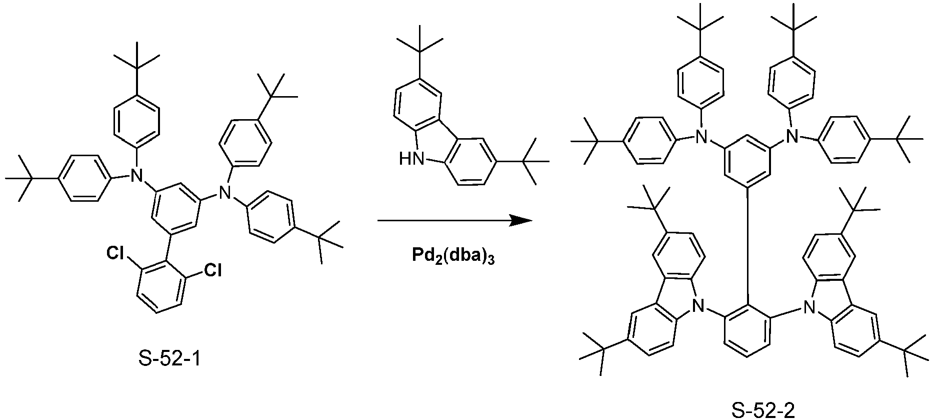
2. Synthesis of compound S-52: this Example was basically the same as the synthesis of compound S-7, except that in this example, S-7-2 was replaced by S-52-2 with an equivalent amount. A target compound S-52 (4.59g; yield: 36%; HPLC analysis purity: 99.23%) as a green solid was obtained. MALDI-TOF-MS result: molecular-ion peak: 1275.02, element analysis result: theoretical value (%): C, 86.62; H, 8.14; B, 0.85; N, 4.39; experimental value (%): C, 86.52; H, 8.24; B, 0.86; N, 4.38.

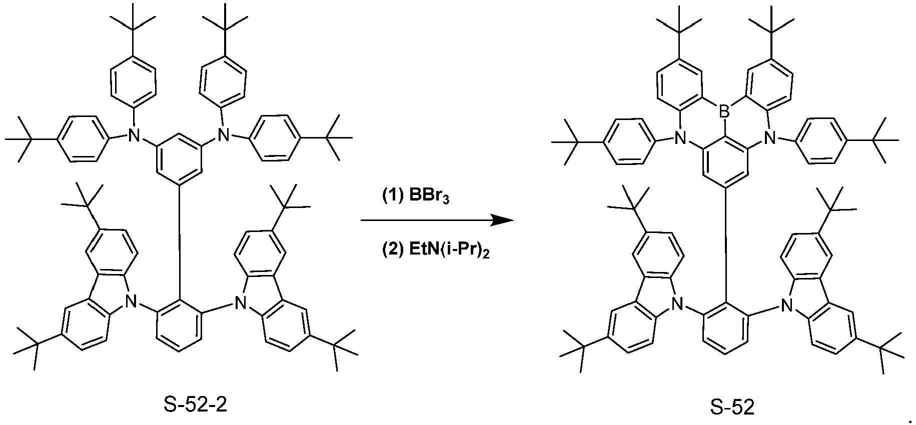

### Synthesis Example 4: Synthesis of Compound S-244

1. This Example was basically the same as the synthesis of compound S-7-2, except that in this Example, S-7-1 was replaced by S-244-1 with an equivalent amount of substance. A target compound S-244 (3.43g, yield: 33%, purity: 99.39% by HPLC analysis) was a green solid. MALDI-TOF-MS result: molecular-ion peak: 1039.62, element analysis result: theoretical value (%): C, 85.43; H, 7.46; N, 4.04; O, 3.08; experimental value (%): C, 85.53; H, 7.36; N, 4.06; O, 3.06.

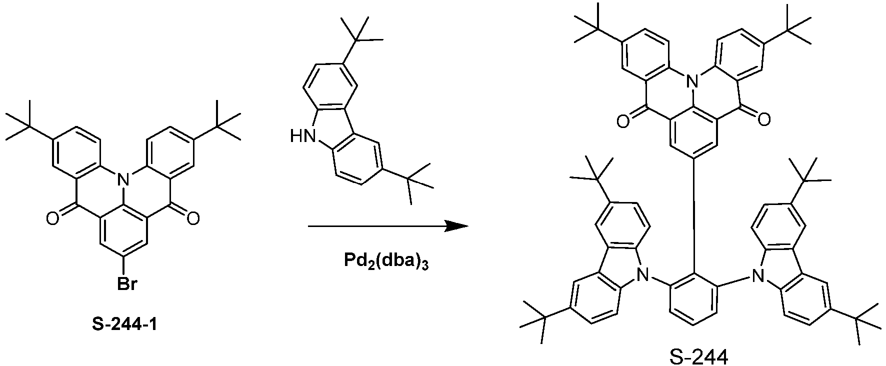

In addition, other obtained fluorescent dyes in the present application were also characterized through mass spectrometry (MALDI-TOF-MS molecular ion peak), as shown in Table 1 below.

**Table 1**

| | Mass spectrum | |
|---|---|---|
| | Theoretical value | Experimental value |
| S-24 | 970.51 | 970.75 |
| S-49 | 1236.63 | 1236.71 |
| S-50 | 1050.57 | 1050.69 |
| S-58 | 1236.63 | 1236.77 |
| S-69 | 1325.66 | 1325.71 |
| S-96 | 1267.77 | 1267.89 |
| S-108 | 1267.77 | 1267.81 |
| S-146 | 919.46 | 919.56 |
| S-252 | 753.45 | 753.51 |

The organic electroluminescent device of the present application is further introduced below through specific embodiments. The following Examples 1-19 and 22-25 are not according to the invention and are present for illustration purposes only.

### Examples 1-29

Examples 1-29 respectively provided organic electroluminescent devices, the structure of the device successively included an anode, a hole injection layer (HIL), a hole transmission layer (HTL), an electron blocking layer (EBL), a light-emitting layer (EML), a hole blocking layer (HBL), an electron transmission layer (ETL), an electron injection layer (EIL), a cathode, and a capping layer (CPL). Taking Example 1 as an example, the specific preparation method was as following:
(1) A glass plate coated with a conductive layer of ITO/Ag/ITO was ultrasonically treated in a commercial cleaning agent, rinsed in deionized water, ultrasonically degreased in a mixed solvent of acetone and ethanol, baked in a clean environment until the water was completely removed, cleaned with ultraviolet light and ozone, and then subjected to a surface bombardment with a low energy cation beam;
(2) The above glass plate with the anode was placed in a vacuum chamber and vacuumized to less than 1×10⁻⁵ Pa, HT-24 and HI-2 as the hole injection layer were deposited on the anode layer film via co-evaporation, where the ratio of HI-2 was 3%, an evaporation speed of HT-24 was 0.1 nm/s, and an evaporation film had a thickness of 10 nm;
(3) The hole transmission layer HT-24 was deposited on the hole injection layer via vacuum evaporation, with an evaporation speed of 0.1nm/s and a total thickness of evaporation film of 110nm;
(4) The electron blocking layer EB-1 was deposited on the hole transmission layer via vacuum evaporation, with an evaporation speed of 0.1nm/s and a total thickness of evaporation film of 5nm;
(5) The light-emitting layer was deposited on the electron blocking layer via vacuum co-evaporation, the light-emitting layer included the host material BFH-4 and fluorescent dye S-7, a multi-source co-evaporation method was used, with a doping ratio of the dye being 2% for evaporation, an evaporation speed of the host being 0.1nm/s, and an evaporation film thickness being 20nm;
(6) The hole blocking layer HB-1 was deposited on the light-emitting layer via vacuum evaporation, with an evaporation speed of 0.1nn/s and a total thickness of evaporation film of 5nm;
(7) ET-57 and ET-69 (with a mass ratio of 1:1) as the electron transmission layer were deposited on the hole blocking layer via vacuum evaporation, the evaporation speeds of ET-57 and ET-69 were both 0.1nm/s, and a total thickness of evaporation film was 30nm;
(8) Yb with a thickness of 1nm was deposited via vacuum evaporation on the electron transmission layer as the electron injection layer;
(9) An magnesium-silver (Mg-Ag) alloy layer with a thickness of 15nm was deposited via evaporation on the electron injection layer as the cathode of the device, with a mass ratio of Mg: Ag=1:9;
(10) C-1 layer with a thickness of 65nm was deposited via evaporation on the cathode as the capping layer of the device.

Specifically, the device is a top-emitting structure that includes an anode, a hole injection layer, a hole transmission layer, an electron blocking layer, a light-emitting layer, a hole blocking layer, an electron transmission layer, an electron injection layer, a cathode, and a capping layer from bottom to top.

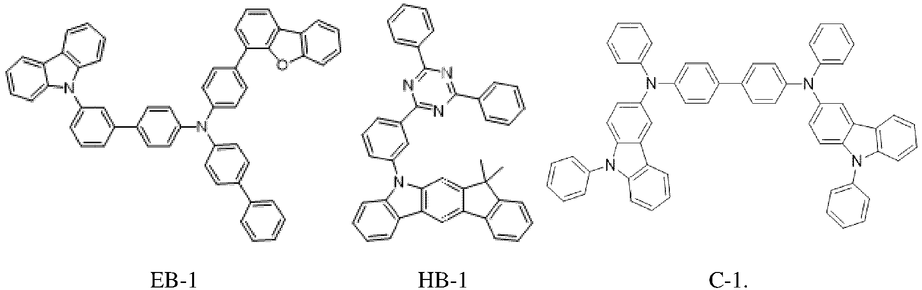

In the organic electroluminescent devices provided by Examples 2-29, the specific preparation methods are similar to that of Example 1, except for the specific selection of the host material and fluorescent dye, and the mass proportion of fluorescent dye in the light-emitting layer. The relevant characterizations of fluorescent dye in some devices in the Examples are shown in Table 2 below.

### Comparative Examples 1-8

Comparative Examples of 1-8 provide organic electroluminescent devices. The device structures are consistent with those of Examples 1-29, and the parameters of the corresponding functional layers are basically consistent with those of Examples 1-29. The difference is only that the host material and dye of the light-emitting layer are inconsistent with the materials or the doping concentration used in the Examples.

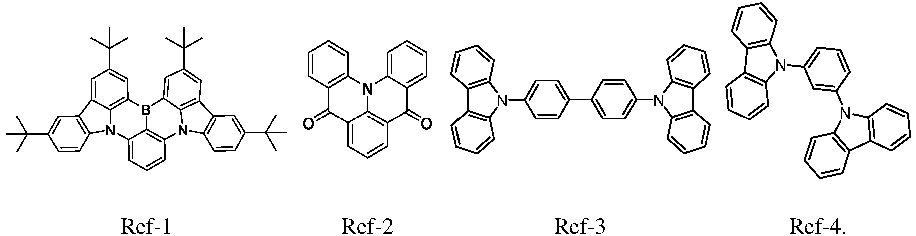

The specific composition of organic electroluminescent devices for Examples 1-29 and Comparative Examples 1-8 is shown in Table 2.

The following tests were conducted on the devices of Examples and Comparative Examples, and the test results are shown in Table 2.

Under the same brightness, Keithley K 2400 digital source meter and PR 655 spectral scanning luminance meter are used to measure the driving voltage and BI value of the organic electroluminescent devices prepared in Examples 1-29 and Comparative Examples 1-8. Specifically, the voltage is increased at a speed of 0.1V per second, and when the brightness of the organic electroluminescent device reaches 1000cd/m², the measured voltage is the driving voltage and the current density is measured at this time, and the ratio of brightness to current density is the current efficiency; BI value of the device at 1000cd/m² is derived from the current efficiency at 1000cd/m² by dividing the CIEy value of the spectrum of the device at this time.

**Table 2**

| | Host material | Dyes and mass proportion | Voltage (V) under 1000cd/m² | BI value(CE/CIEy) under1000cd/m² |
|---|---|---|---|---|
| Example 1 | BFH-4 | S-7, 2% | 3.98V | 176 |
| Example 2 | BFH-4 | S-7, 10% | 3.99V | 174 |
| Example 3 | BFH-4 | S-7, 15% | 4.02V | 170 |
| Example 4 | BFH-4 | S-7, 20% | 4.02V | 169 |
| Example 5 | BFH-4 | S-7, 30% | 4.05V | 166 |
| Example 6 | BFH-14 | S-7, 2% | 3.51V | 177 |
| Example 7 | BFH-14 | S-7, 15% | 3.58V | 171 |
| Example 8 | BFH-14 | S-7, 20% | 3.59V | 170 |
| Example 9 | BFH-14 | S-7, 30% | 3.77 V | 167 |
| Example 10 | BFH-4 | S-13, 2% | 3.97V | 178 |
| Example 11 | BFH-4 | S-13, 15% | 4.04V | 173 |
| Example 12 | BFH-4 | S-24, 2% | 4.03V | 169 |
| Example 13 | BFH-4 | S-24, 15% | 4.07V | 165 |
| Example 14 | BFH-4 | S-50, 2% | 3.87V | 180 |
| Example 15 | BFH-4 | S-50, 15% | 3.90V | 175 |
| Example 16 | BFH-4 | S-52, 2% | 3.88V | 183 |
| Example 17 | BFH-4 | S-52, 15% | 3.92V | 180 |
| Example 18 | BFH-4 | S-69, 2% | 3.95V | 181 |
| Example 19 | BFH-4 | S-69, 15% | 3.98V | 179 |
| Example 20 | BFH-4 | S-146, 2% | 4.05V | 166 |
| Example 21 | BFH-4 | S-146, 15% | 4.09V | 162 |
| Example 22 | BFH-4 | S-244, 2% | 4.06V | 171 |
| Example 23 | BFH-4 | S-244, 15% | 4.11V | 164 |
| Example 24 | BFH-4 | S-252, 2% | 4.11V | 170 |
| Example 25 | BFH-4 | S-252, 15% | 4.13V | 163 |
| Example 26 | BFH-21 | S-49, 2% | 3.61V | 172 |
| Example 27 | BFH-21 | S-58, 2% | 3.58V | 176 |
| Example 28 | BFH-18 | S-96, 2% | 3.99V | 173 |
| Example 29 | BFH-18 | S-108, 2% | 3.96V | 178 |
| Comparative Example 1 | BFH-4 | Ref-1, 2% | 4.26V | 152 |
| Comparative Example 2 | BFH-4 | Ref-1, 15% | 4.39V | 121 |
| Comparative Example 3 | BFH-4 | Ref-2, 2% | 4.33V | 135 |
| Comparative Example 4 | BFH-4 | Ref-2, 15% | 4.44V | 87 |
| Comparative Example 5 | Ref-3 | S-7, 2% | 4.62V | 103 |
| Comparative Example 6 | Ref-3 | S-7, 15% | 4.67V | 92 |
| Comparative Example 7 | Ref-4 | S-7, 2% | 4.55V | 95 |
| Comparative Example 8 | Ref-4 | S-7, 15% | 4.59V | 83 |

According to Table 2, it can be seen that:
1. Compared with the Comparative Examples, organic electroluminescent devices in Examples 1-29 of the present application can effectively reduce the driving voltage and improve the luminescence efficiency by introducing carbon ring groups or heterocyclic groups represented by A and coated with a group with large steric effect in the molecular structure;
2. Compared with Comparative Examples 1-4, organic electroluminescent devices using the fluorescent dye represented by Formula (1-1), (1-2), (1-3), (1-4), (1-5), (1-6), (2-1), (2-2), or (2-3) in Examples 1-29 of the present application have less dependence on the mass proportion of the fluorescent dye; with the change of the mass proportion of the fluorescent dye, the fluctuation of the driving voltage and the luminescence efficiency of the device is not significant; further, from Examples 1-5 and 6-9, it can be seen that the performance of the device is more excellent when the mass proportion of the fluorescent dye is 0.5-20%;
3. From Examples 26-27 and 28-29, it can be seen that when Z₉ and Z₁₀ of the fluorescent dye represented by Formula (1-1), (1-2), (1-3), (1-4), (1-5), (1-6), (2-1), (2-2), or (2-3) are each CH, it is more beneficial to reduce the driving voltage of the device and improve the luminescence efficiency thereof;
4. For Comparative Examples 1-4, when molecules represented by Ref-1 and Ref-2 are used as the dyes, the driving voltage and luminescence efficiency of the device are not as good as those in the Examples; when the doping concentration of dye is changed by changing Ref1 and Ref2 at the same time, the luminescence efficiency and driving voltage of the device have significant changes; for Comparative Examples 5-8, other types of non-triplet-triplet quenching host (ref-3 and ref-4 molecules) are used, the driving voltage of the device is significantly increased compared with Examples, and the luminescence efficiency of the device is reduced. Therefore, the present application can achieve better device performance by matching a class of triplet-triplet annihilation hosts, which can meet the requirements of current panel manufacturers for high-performance materials, and has good application prospects.

Finally, it should be noted that the above embodiments are only used to illustrate the technical solution of the present application and not to limit it; although the present application has been described in detail with reference to the aforementioned embodiments, ordinary technical personnel in the art should understand that they can still make modification on the technical solutions recorded in the aforementioned embodiments, or make an equivalent substitution some or all of the technical features; and these modification or substitute do not make the essence of the corresponding technical solutions deviate from the scope of the technical solutions of the various embodiments of the present application.

## Claims

1. An organic electroluminescent device comprising a light-emitting layer, the light-emitting layer comprises a triplet-triplet annihilation type host and a fluorescent dye, **characterized in that** the fluorescent dye has a structure represented by the following Formula (1-2), (1-3), (1-6) or (2-3): 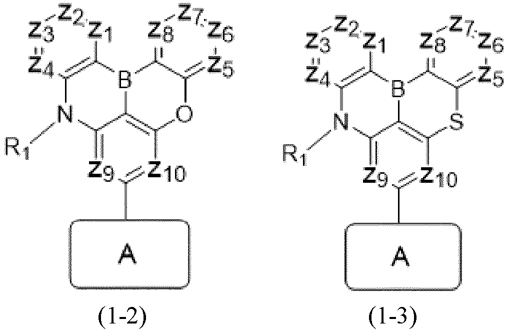 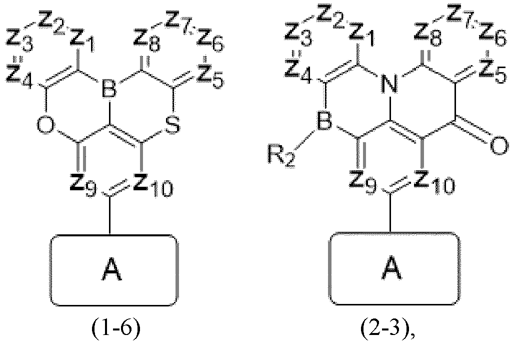
in Formula (1-2), (1-3), (1-6) or (2-3), A represents one of a substituted or unsubstituted C₆-C₆₀ carbocyclic group, and a substituted or unsubstituted C₃-C₆₀ heterocyclyl; a substituted group in A is one or a combination of at least two selected from deuterium, tritium, cyano, halogen, C₁-C₁₀ alkyl, C₃-C₁₀ cycloalkyl, silyl, C₆-C₃₀ arylamino, C₆-C₃₀ aryl, and C₂-C₃₀ heteroaryl, and the substituted group is independently connected to a connected aromatic ring or heteroaromatic ring to form a ring, or the substituted group is independently connected to a connected aromatic ring or heteroaromatic ring to not form a ring;
in Formula (1-2), (1-3), (1-6) or (2-3), Z₁-Z₁₀ are each independently represented as N or CR, with R being the same or different each time, two adjacent R can bond to each other to form a ring;
R₁ represents one of a substituted or unsubstituted C₁-C₁₀ alkyl, a substituted or unsubstituted C₃-C₁₀ cycloalkyl, a substituted or unsubstituted C₆-C₃₀ aryl, and a substituted or unsubstituted C₂-C₃₀ heteroaryl;
R₂ represents one of a substituted or unsubstituted C₆-C₃₀ aryl, and a substituted or unsubstituted C₂-C₃₀ heteroaryl;
R represents one of hydrogen, deuterium, tritium, cyano, halogen, a substituted or unsubstituted C₁-C₁₀ alkyl, a substituted or unsubstituted C₃-C₁₀ cycloalkyl, a substituted or unsubstituted C₁-C₁₀ alkoxy, a substituted or unsubstituted C₆-C₃₀ aryloxy, a substituted or unsubstituted C₆-C₃₀ arylamino, a substituted or unsubstituted C₆-C₃₀ aryl, and a substituted or unsubstituted C₂-C₃₀ heteroaryl;
a substituted group in the each substituted R₁, R₂, and R is one or a combination of at least two selected from deuterium, tritium, cyano, halogen, C₁-C₁₀ alkyl, C₃-C₁₀ cycloalkyl, silyl, C₆-C₃₀ arylamino, C₆-C₃₀ aryl, and C₂-C₃₀ heteroaryl, and the substituted group is independently connected to the connected aromatic ring or heteroaromatic ring to form a ring, or the substituted group is independently connected to the connected aromatic ring or heteroaromatic ring to not form a ring;
wherein the triplet-triplet annihilation type host material is selected from at least one of compounds represented by BFH-18 to BFH-25:

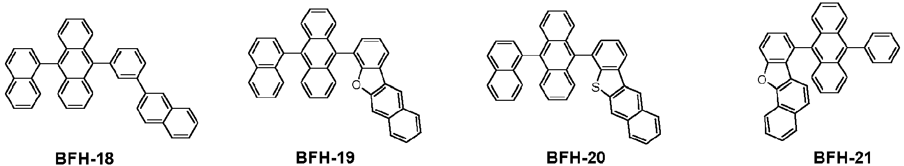

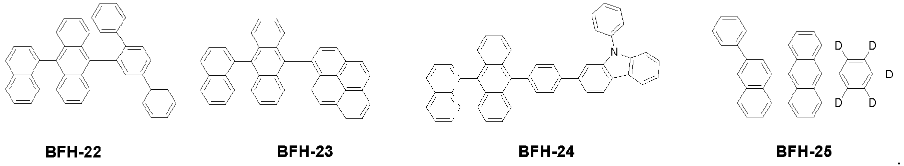

2. The organic electroluminescent device according to claim 1, wherein A represents a substituted group represented by any one of the following (3-1), (3-2), (3-3), (3-4), (3-5), (3-6), (3-7), (3-8) or (3-9): 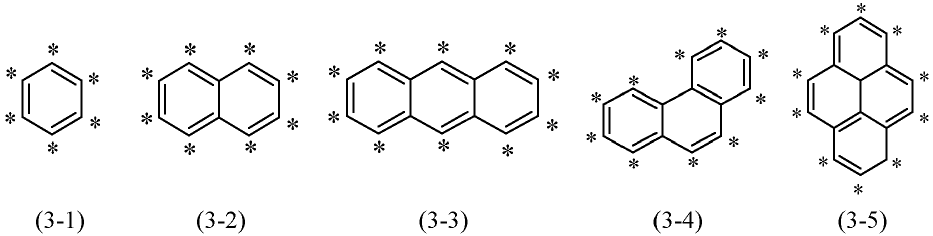 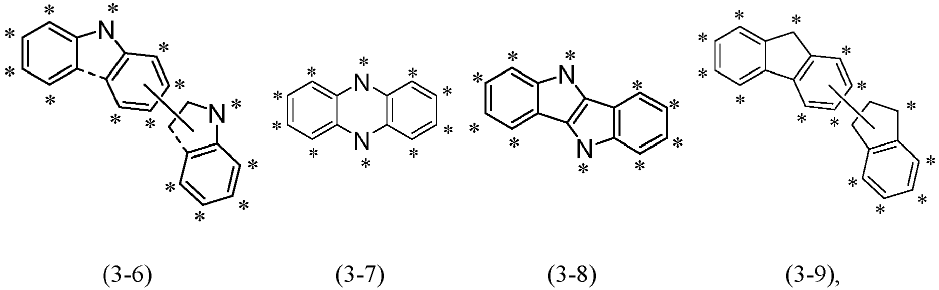
an asterisk in the Formulas denotes a connectable site, this connection denotes connection to a parent nucleus and/or connection with a substituted group; an expression of a ring structure streaked by "-" denotes any position on the ring structure where the connectable site can form a bond; a dashed line in the Formulas represents being connected or being unconnected;
wherein a substituted group in A is one or a combination of at least two selecting from deuterium, tritium, cyano, halogen, C₁-C₁₀ alkyl, C₃-C₁₀ cycloalkyl, C₆-C₃₀ arylamino, C₆-C₃₀ aryl, and C₂-C₃₀ heteroaryl, and the substituted group is independently connected to a connected aromatic ring or heteroaromatic ring to form a ring, or the substituted group is independently connected to a connected aromatic ring or heteroaromatic ring to not form a ring.

3. The organic electroluminescent device according to claim 1 or claim 2, wherein A is represented by any one of the following structural formulas: 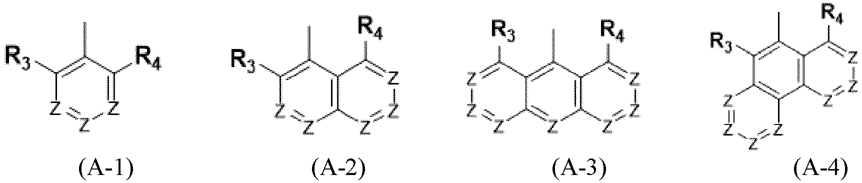 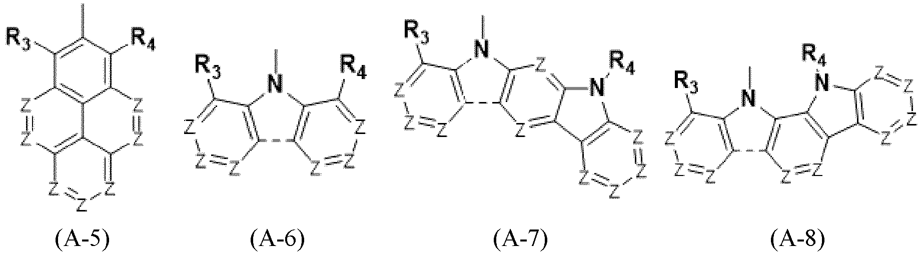 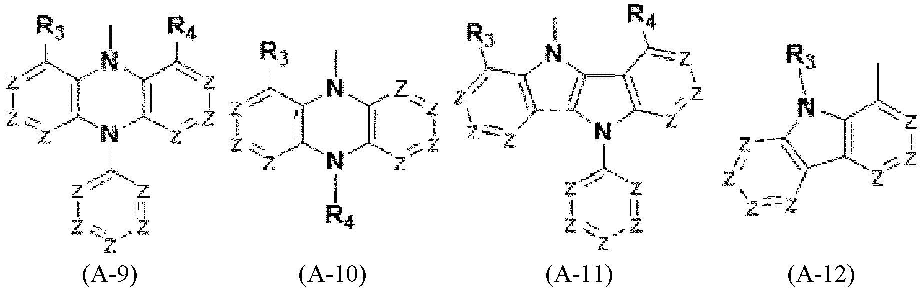
wherein, R₃ and R₄ are each independently represented as any one of hydrogen, deuterium, tritium, a substituted or unsubstituted C₁-C₃₀ alkyl, a substituted or unsubstituted C₃-C₃₀ cycloalkyl, a silyl, a substituted or unsubstituted C₁-C₃₀ alkoxy, a substituted or unsubstituted C₆-C₆₀ aryloxy, a substituted or unsubstituted C₆-C₆₀ arylamino, a substituted or unsubstituted C₆-C₆₀ aryl, and a substituted or unsubstituted C₂-C₆₀ heteroaryl;
Z is independently represented as N or CR₅, with R₅ being the same or different each time; R₅ is represented as one of hydrogen, deuterium, tritium, cyano, halogen, a substituted or unsubstituted C₁-C₁₀ alkyl, a substituted or unsubstituted C₃-C₁₀ cycloalkyl, a substituted or unsubstituted C₁-C₁₀ alkoxy, a substituted or unsubstituted C₆-C₃₀ aryloxy, a substituted or unsubstituted C₆-C₃₀ arylamino, a substituted or unsubstituted C₆-C₃₀ aryl, or a substituted or unsubstituted C₂-C₃₀ heteroaryl;
a substituted group in the each substituted R₃, R₄, and R₅ is one or a combination of at least two selected from deuterium, tritium, cyano, halogen, C₁-C₁₀ alkyl, C₃-C₁₀ cycloalkyl, silyl, C₆-C₃₀ arylamino, C₆-C₃₀ aryl, and C₂-C₃₀ heteroaryl, which is independently connected to the connected aromatic ring or heteroaromatic ring to form a ring, or is independently connected to the connected aromatic ring or heteroaromatic ring to not form a ring.

4. The organic electroluminescent device according to claim 3, wherein the R₃ and R₄ are each independently represented as any one of a substituted or unsubstituted C₁-C₃₀ alkyl, a substituted or unsubstituted C₃-C₃₀ cycloalkyl, a substituted or unsubstituted C₆-C₆₀ aryl, and a substituted or unsubstituted C₂-C₆₀ heteroaryl.

5. The organic electroluminescent device according to claim 4, wherein at least one of the R₃ and R₄ is selected from one of the following groups with large steric effect: terphenyl, trimericphenyl, tetraphenyl, fluorenyl, spirodifluorenyl, dihydrophenanthryl, dihydropyrenyl, tetrahydropyrenyl, cis or trans indenofluorenyl, trimericindenyl, isotrimericindenyl, spiro-trimericindenyl, spiro-isotrimericindenyl, furyl, benzofuryl, isobenzofuryl, dibenzofuryl, benzothienyl, isobenzothienyl, dibenzothienyl, isoindolyl, carbazolyl, indenocarbazolyl, isoquinolyl, acridinyl, phenanthridinyl, benzo-5,6-quinolyl, benzo-6,7-quinolyl, benzo-7,8-quinolyl, benzimidazolyl, naphthoimidazolyl, phenanthroimidazolyl, pyridino imidazolyl, pyrazino imidazolyl, quinoxalino imidazolyl, oxazolyl, benzo-oxazolyl, naphtho-oxazolyl, anthro-oxazolyl, phenanthro-oxazolyl, 1,2-thiazolyl, 1,3-thiazolyl, benzothiazolyl, pyridazinyl, benzopyridazinyl, 1,5-diaza anthryl, 2,7-diaza pyrenyl, 2,3-diaza pyrenyl, 1,6-diaza pyrenyl, 1,8-diaza pyrenyl, 4,5-diaza pyrenyl, 4,5,9,10-tetraaza perylenyl, phenazinyl, phenothiazinyl, azacarbazolyl, benzocarbolinyl, phenanthrolinyl, 1,2,3-triazolyl, 1,2,4-triazolyl, benzotriazolyl, 1,2,3-oxadiazolyl, 1,2,4-oxadiazolyl, 1,2,5-oxadiazolyl, 1,2,3-thiadiazolyl, 1,2,4-thiadiazolyl, 1,2,5-thiadiazolyl, 1,3,4-thiadiazolyl, 1,3,5-triazinyl, 1,2,4-triazinyl, 1,2,3-triazinyl, tetrazolyl, 1,2,4,5-tetrazinyl, 1,2,3,4-tetrazinyl, 1,2,3,5-tetrazinyl, purinyl, pteridyl, indozinyl, benzothiadiazolyl, 9,9-dimethylacridinyl, diphenylamindo, adamantyl, fluorophenyl, methylphenyl, trimethylphenyl, cyanophenyl, silyl; or at least one of R₃ or R₄ is a combination formed by at least two groups selected from the aforementioned groups with large steric effect.

6. The organic electroluminescent device according to any one of claims 1-5, wherein the R denotes one of hydrogen, deuterium, tritium, fluorine atom, cyano, methyl, deuterated methyl, tritiated methyl, ethyl, deuterated ethyl, tritiated ethyl, isopropyl, deuterated isopropyl, tritiated isopropyl, tert-butyl, deuterated tert-buty, tritiated tert-butyl, deuterated cyclopentyl, tritiated cyclopentyl, cyclohexyl, cyclopentyl, adamantyl, phenyl, deuterated phenyl, tritiated phenyl, biphenyl, deuterated biphenyl, tritiated biphenyl, deuterated terphenyl, tritiated terphenyl, terphenyl, naphthyl, anthryl, phenanthryl, pyridyl, quinolyl, furyl, thienyl, dibenzofuryl, dibenzothienyl, carbazolyl, N-phenylcarbazolyl, 9,9-dimethylfluorenyl, 9,9-diphenylfluorenyl, spirofluorenyl, methyl-substituted phenyl, ethyl-substituted phenyl, isopropyl-substituted phenyl, tert-butyl-substituted phenyl, methyl-substituted biphenyl, ethyl-substituted biphenyl, isopropyl-substituted biphenyl, tert-butyl-substituted biphenyl, deuterated methyl-substituted phenyl, deuterated ethyl-substituted phenyl, deuterated isopropyl-substituted phenyl, deuterated tert-butyl-substituted phenyl, deuterated methyl-substituted biphenyl, deuterated ethyl-substituted biphenyl, deuterated isopropyl-substituted biphenyl, deuterated tert-butyl-substituted diphenyl, tritiated methyl-substituted phenyl, tritiated ethyl-substituted phenyl, tritiated isopropyl-substituted phenyl, tritiated tert-butyl-substituted phenyl, tritiated methyl-substituted diphenyl, tritiated ethyl-substituted diphenyl, tritiated isopropyl-substituted diphenyl, tritiated tert-butyl-substituted diphenyl, diphenylamido, di-biphenylamido, and triphenylamino;
wherein R₁ represents one of methyl, deuterated methyl, tritiated methyl, ethyl, deuterated ethyl, tritiated ethyl, isopropyl, deuterated isopropyl, tritiated isopropyl, tert-butyl, deuterated tert-butyl, tritiated tert-butyl, deuterated cyclopentyl, tritiated cyclopentyl, cyclopentyl, adamantyl, phenyl, deuterated phenyl, tritiated phenyl, biphenyl, deuterated biphenyl, tritiated biphenyl, deuterated terphenyl, tritiated terphenyl, terphenyl, naphthyl, anthryl, phenanthryl, pyridyl, quinolyl, furyl, thienyl, dibenzofuryl, dibenzothienyl, carbazolyl, N-phenylcarbazolyl, 9,9-dimethylfluorenyl, 9,9-diphenylfluorenyl, spirofluorenyl, methyl-substituted phenyl, ethyl-substituted phenyl, isopropyl-substituted phenyl, tert-butyl-substituted phenyl, methyl-substituted diphenyl, ethyl-substituted diphenyl, isopropyl-substituted diphenyl, tert-butyl-substituted diphenyl, deuterated methyl-substituted phenyl, deuterated ethyl-substituted phenyl, deuterated isopropyl-substituted phenyl, deuterated tert-butyl-substituted phenyl, deuterated methyl-substituted diphenyl, deuterated ethyl-substituted diphenyl, deuterated isopropyl-substituted diphenyl, deuterated tert-butyl-substituted diphenyl, tritiated methyl-substituted phenyl, tritiated ethyl-substituted phenyl, tritiated isopropyl-substituted phenyl, tritiated tert-butyl-substituted phenyl, tritiated methyl-substituted diphenyl, tritiated ethyl-substituted diphenyl, tritiated isopropyl-substituted diphenyl, and tritiated tert-butyl-substituted diphenyl;
wherein the R₂ represents one of phenyl, deuterated phenyl, tritiated phenyl, biphenyl, deuterated biphenyl, tritiated biphenyl, deuterated terphenyl, tritiated terphenyl, terphenyl, naphthyl, anthryl, phenanthryl, pyridyl, quinolyl, dibenzofuryl, dibenzothienyl, N-phenylcarbazolyl, methyl-substituted phenyl, amidine, ethyl-substituted phenyl, isopropyl-substituted phenyl, tert-butyl-substituted phenyl, methyl-substituted biphenyl, ethyl-substituted biphenyl, isopropyl-substituted biphenyl, tert-butyl-substituted biphenyl, deuterated methyl-substituted phenyl, deuterated ethyl-substituted phenyl, deuterated isopropyl-substituted phenyl, deuterated tert-butyl-substituted phenyl, deuterated methyl-substituted biphenyl, deuterated ethyl-substituted biphenyl, deuterated isopropyl-substituted biphenyl, deuterated tert-butyl-substituted biphenyl, tritiated methyl-substituted phenyl, tritiated ethyl-substituted phenyl, tritiated isopropyl-substituted phenyl, tritiated tert-butyl-substituted phenyl, tritiated methyl-substituted diphenyl, tritiated ethyl-substituted diphenyl, tritiated isopropyl-substituted diphenyl, and tritiated tert-butyl-substituted diphenyl.

7. The organic electroluminescent device according to claim 6, wherein in Formula (1-2), (1-3), (1-6) or (2-3), the Z₉ and Z₁₀ are each CH.

8. The organic electroluminescent device according to claim 6, wherein in Formula (1-2), (1-3), (1-6) or (2-3), the Z₂ and Z₇ are each CC(CH₃)₃, and Z₁, Z₃-Z₆, and Z₈-Z₁₀ are each CH.

9. The organic electroluminescent device according to claim 1, wherein the fluorescent dye is selected from a compound represented by the following specific structural formulas:

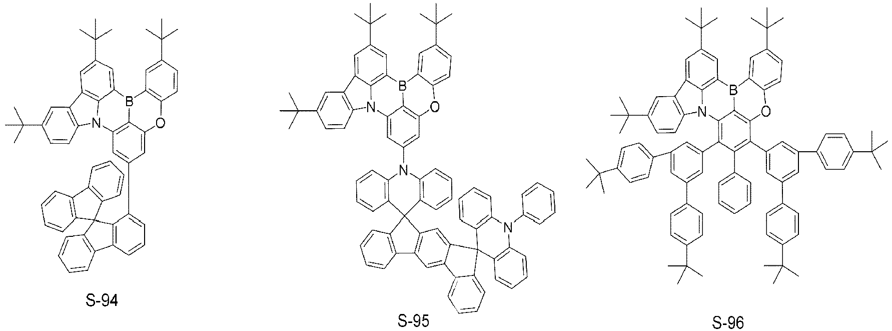

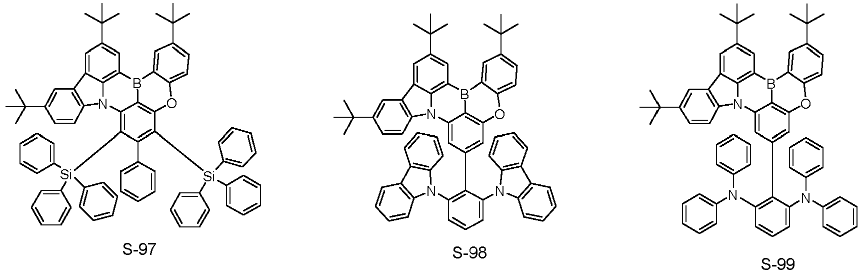

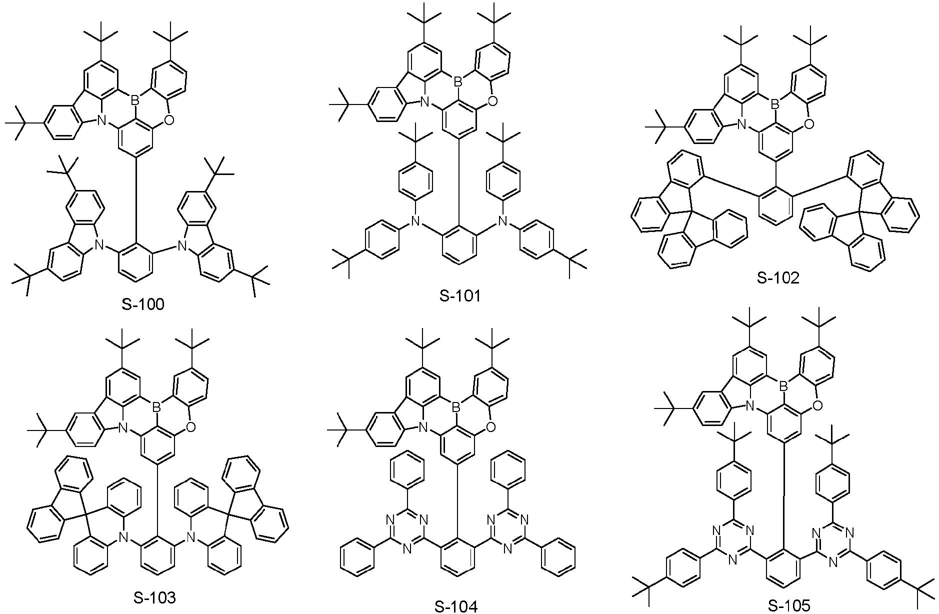

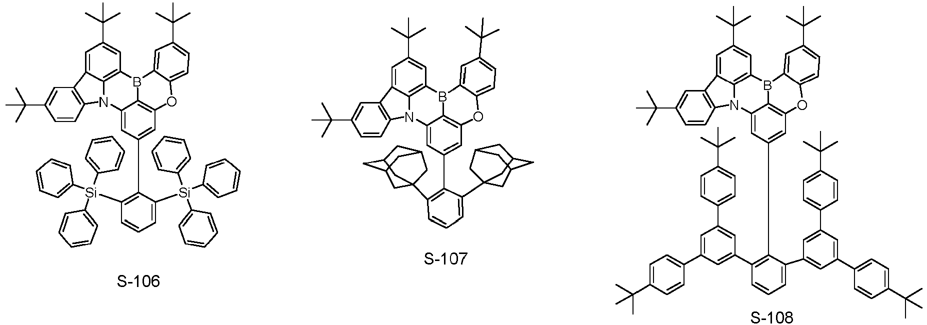

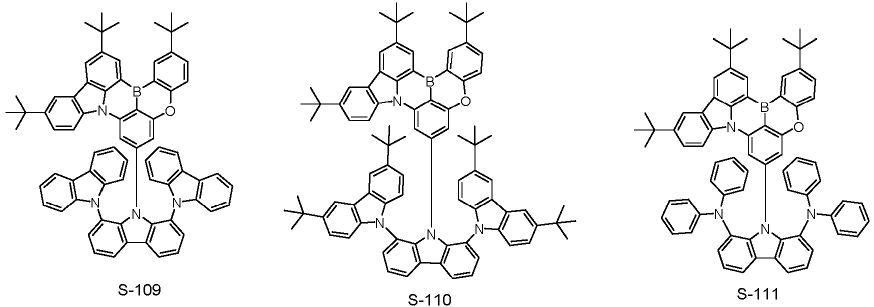

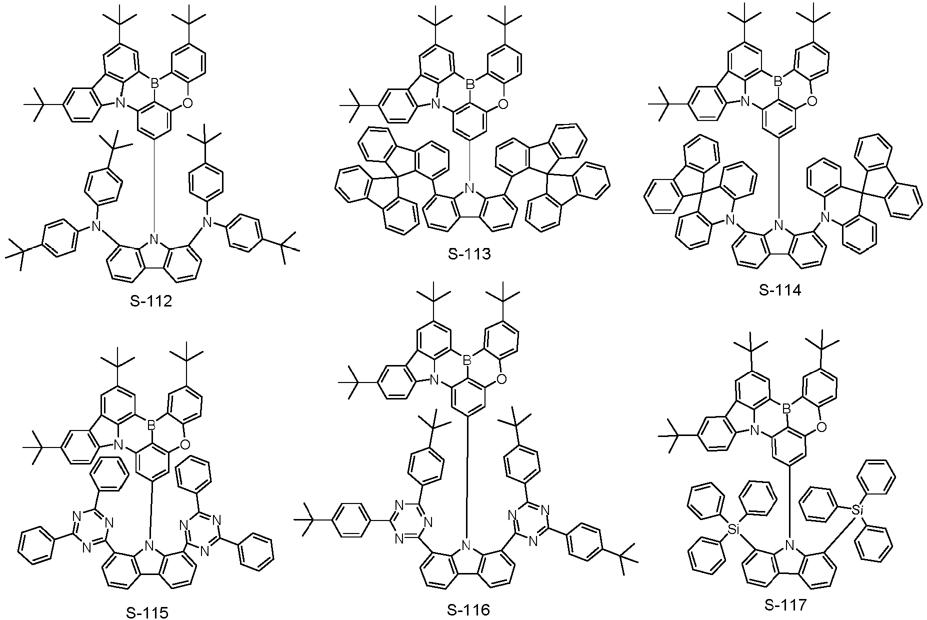

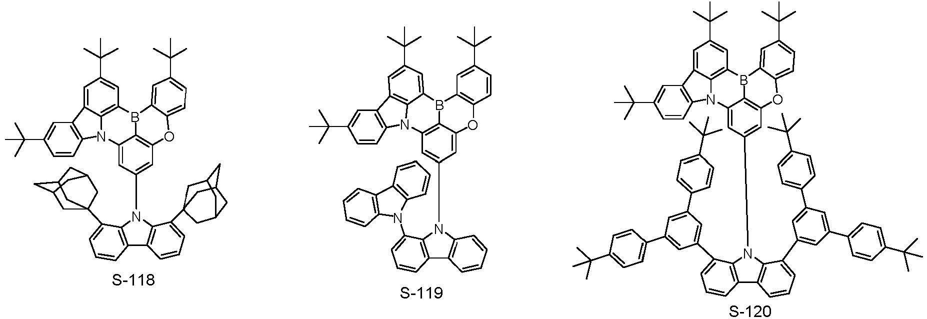

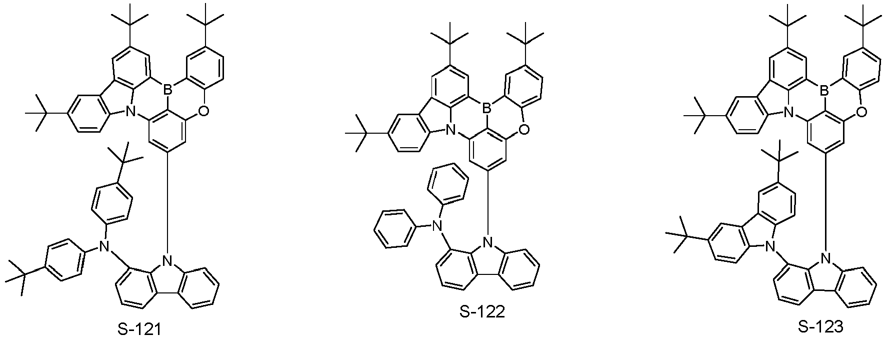

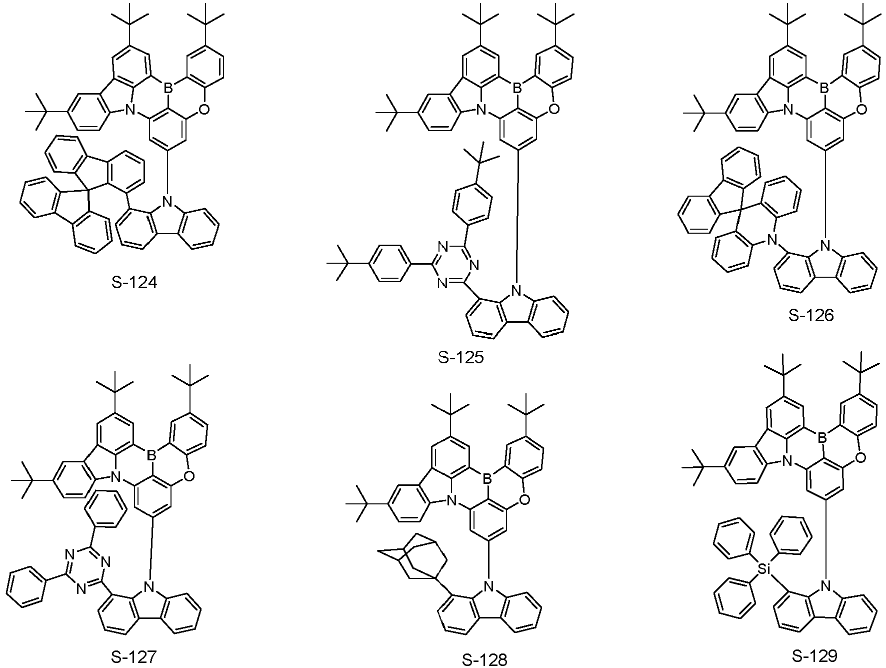

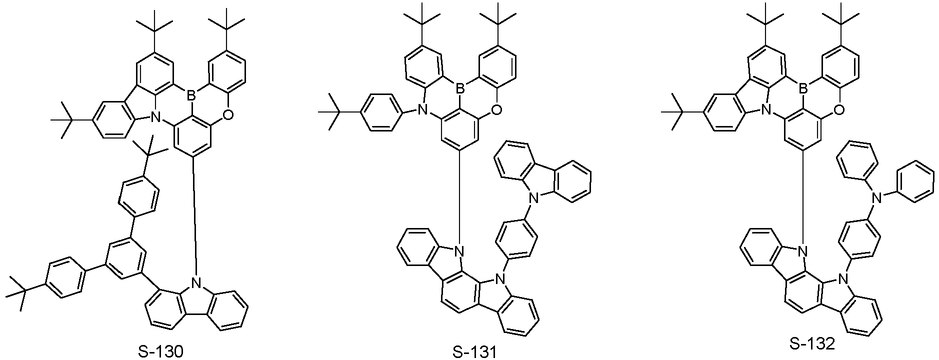

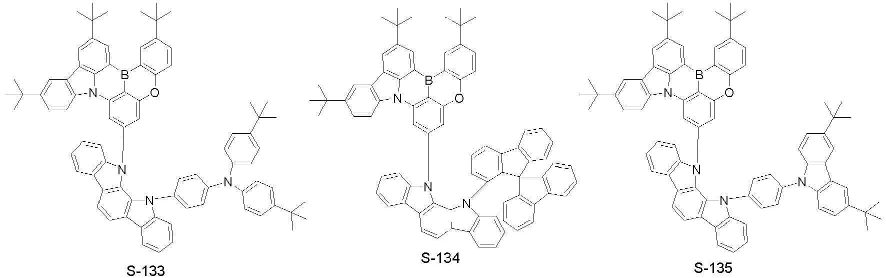

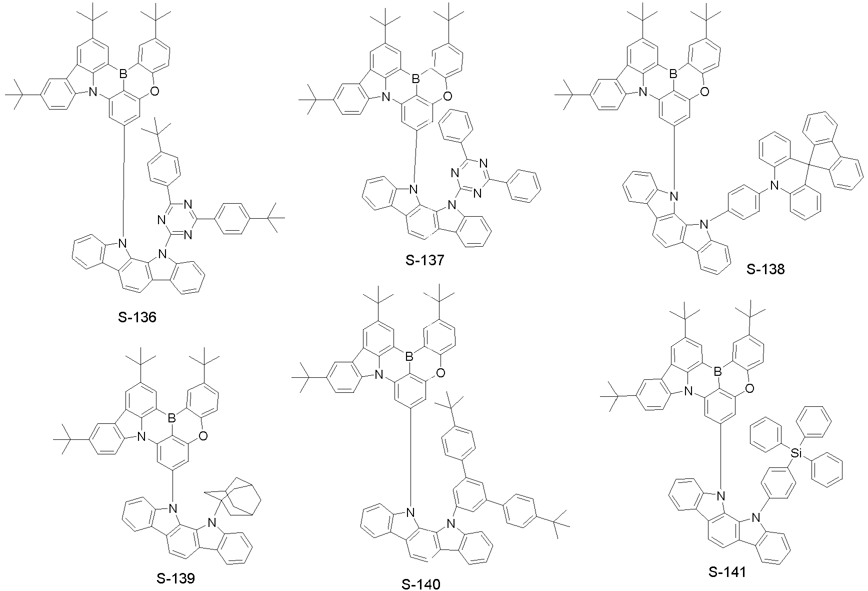

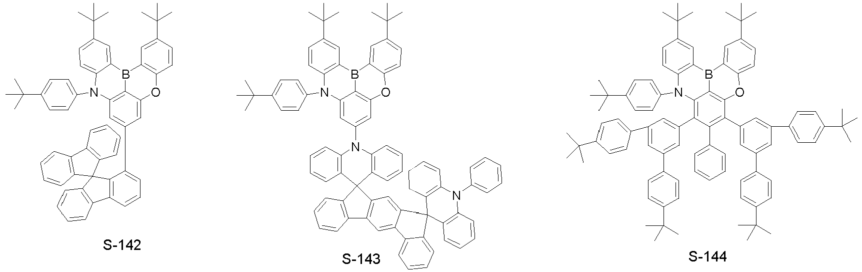

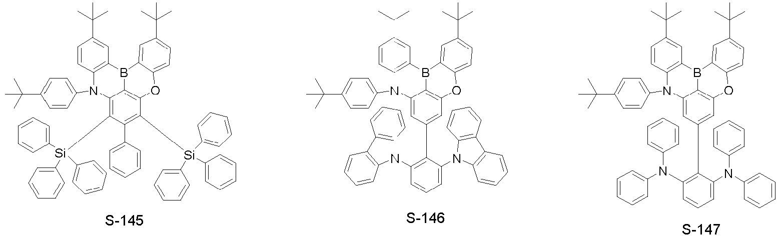

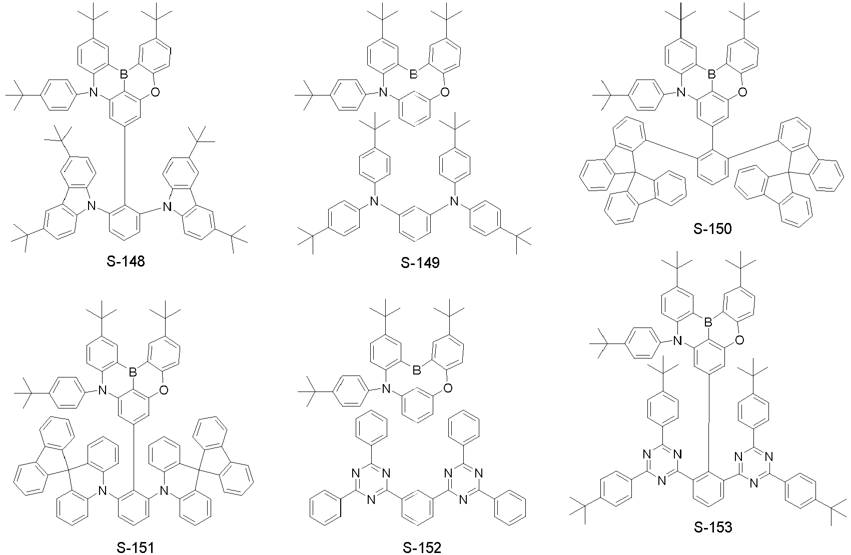

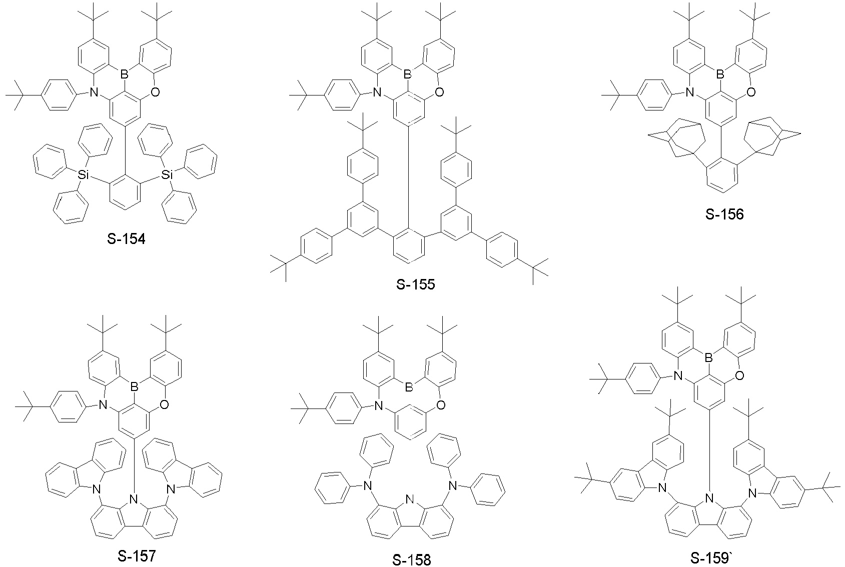

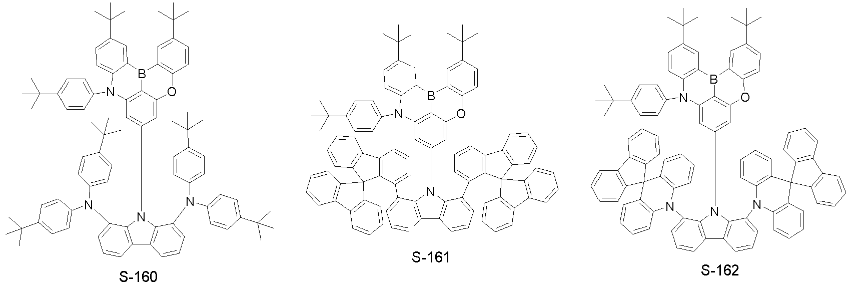

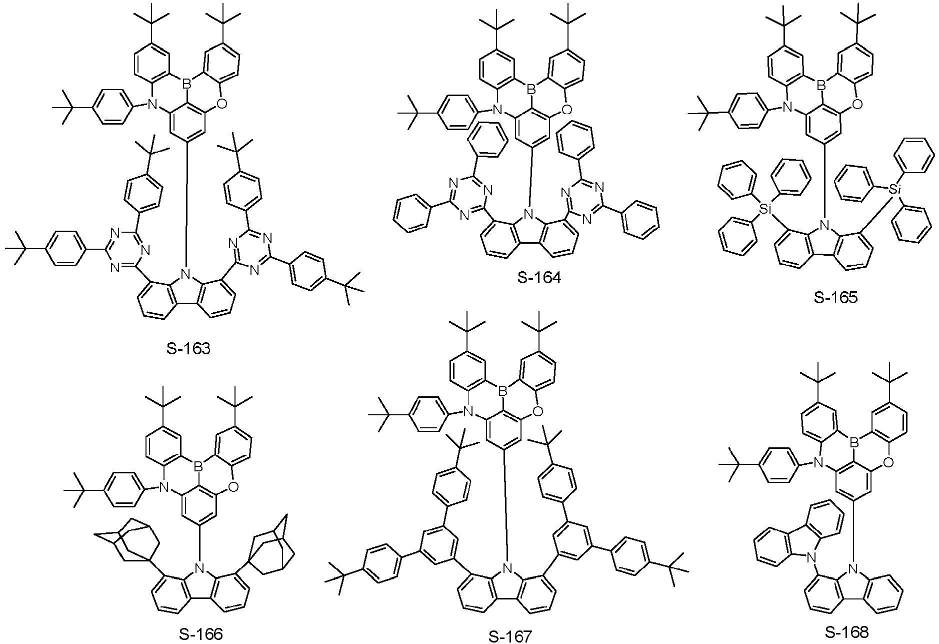

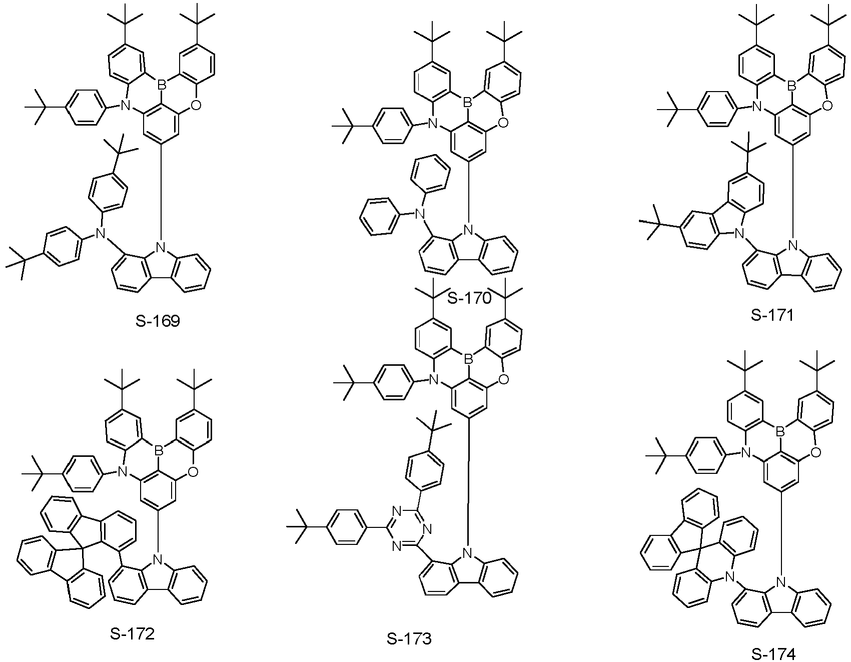

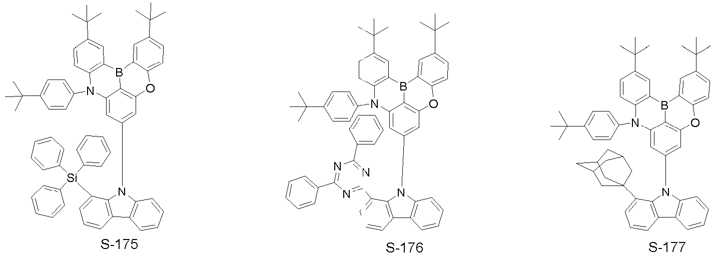

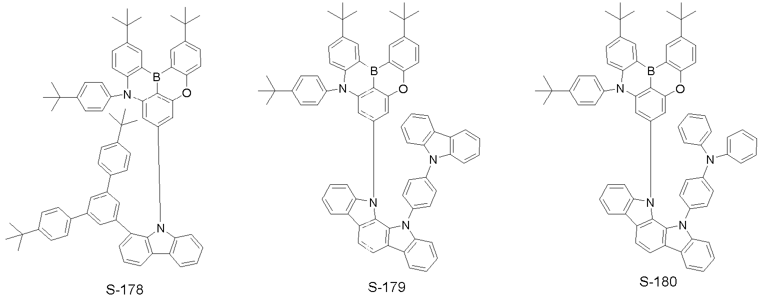

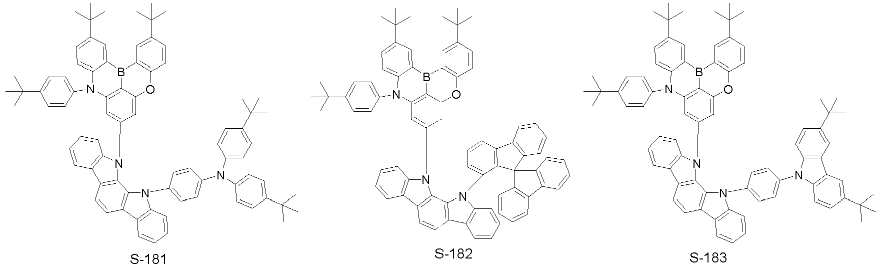

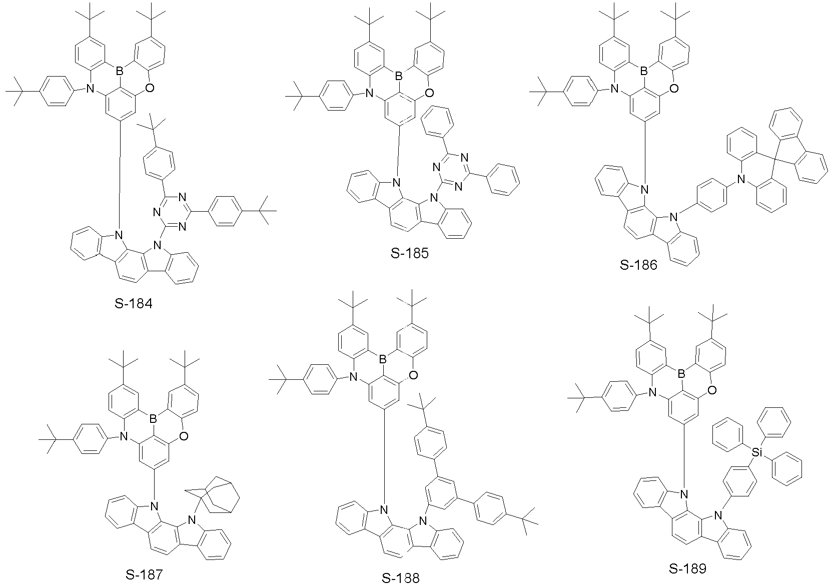

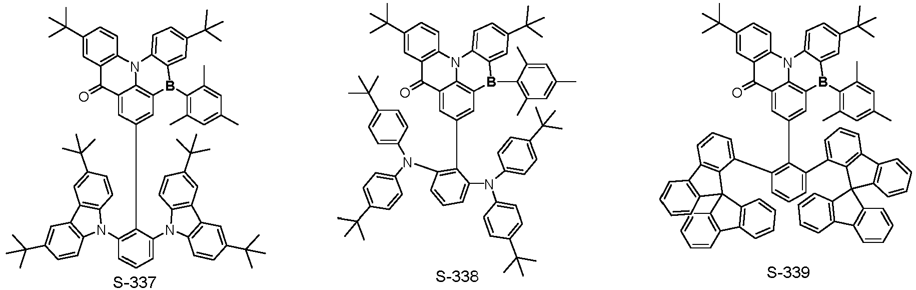

10. The organic electroluminescent device according to any one of claims 1-9, wherein a mass ratio of the fluorescent dye to the light-emitting layer is 0.1% -50%.

11. The organic electroluminescent device according to claim 10, wherein the mass ratio of the fluorescent dye to the light-emitting layer is 0.5% -20%.

12. A display apparatus, comprising the organic electroluminescent device according to any one of claims 1-11.

## Patentansprüche

1. Organische Elektrolumineszenzvorrichtung, umfassend eine lichtemittierende Schicht, wobei die lichtemittierende Schicht einen Triplett-Triplett-Wirt des Annihilationstyps und einen Fluoreszenzfarbstoff umfasst, **dadurch gekennzeichnet, dass** der Fluoreszenzfarbstoff eine Struktur aufweist, die durch die folgende Formel (1-2), (1-3), (1-6) oder (2-3) dargestellt wird:, 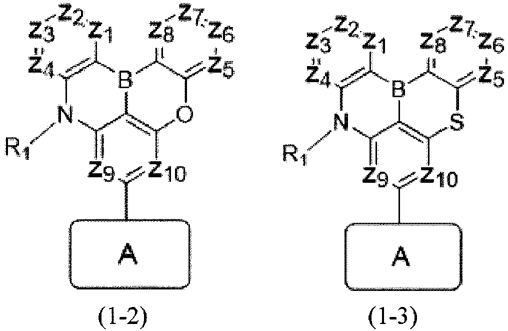 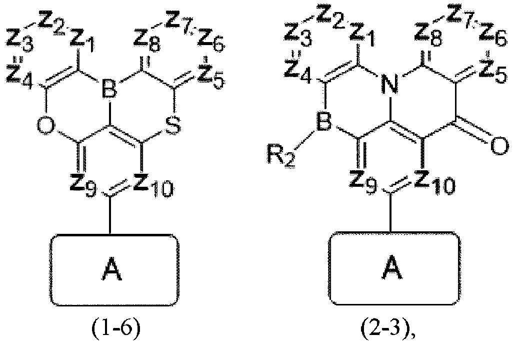
wobei in Formel (1-2), (1-3), (1-6) oder (2-3) A eines von einer substituierten oder unsubstituierten carbocyclischen C₆-C₆₀-Gruppe oder einer substituierten oder unsubstituierten C₃-C₆₀-Heterocyclylgruppe darstellt; eine substituierte Gruppe in A eine oder eine Kombination von mindestens zwei ist, ausgewählt aus Deuterium, Tritium, Cyano, Halogen, C₁-C₁₀-Alkyl, C₃-C₁₀-Cycloalkyl, Silyl, C₆-C₃₀-Arylamino, C₆-C₃₀-Aryl und C₂-C₃₀-Heteroaryl, und die substituierte Gruppe unabhängig mit einem verbundenen aromatischen Ring oder heteroaromatischen Ring verbunden ist, um einen Ring auszubilden, oder die substituierte Gruppe unabhängig mit einem verbundenen aromatischen Ring oder heteroaromatischen Ring verbunden ist, um keinen Ring auszubilden;
in Formel (1-2), (1-3), (1-6) oder (2-3) Z₁-Z₁₀ jeweils unabhängig als N oder CR dargestellt sind, wobei R jedes Mal gleich oder unterschiedlich ist, zwei angrenzende R miteinander binden können, um einen Ring auszubilden;
R₁ eines von einem substituierten oder unsubstituierten C₁-C₁₀-Alkyl, einem substituierten oder unsubstituierten C₃-C₁₀-Cycloalkyl, einem substituierten oder unsubstituierten C₆-C₃₀-Aryl und einem substituierten oder unsubstituierten C₂-C₃₀-Heteroaryl darstellt;
R₂ eines von einem substituierten oder unsubstituierten C₆-C₃₀-Aryl und einem substituierten oder unsubstituierten C₂-C₃₀-Heteroaryl darstellt;
R eines von Wasserstoff, Deuterium, Tritium, Cyano, Halogen, einem substituierten oder unsubstituierten C₁-C₁₀-Alkyl, einem substituierten oder unsubstituierten C₃-C₁₀-Cycloalkyl, einem substituierten oder unsubstituierten C₁-C₁₀-Alkoxy, einem substituierten oder unsubstituierten C₆-C₃₀-Aryloxy, einem substituierten oder unsubstituierten C₆-C₃₀-Arylamino, einem substituierten oder unsubstituierten C₆-C₃₀-Aryl und einem substituierten oder unsubstituierten C₂-C₃₀-Heteroaryl darstellt;
eine substituierte Gruppe in jedem substituierten R₁, R₂ und R eine oder eine Kombination von mindestens zwei ist, ausgewählt aus Deuterium, Tritium, Cyano, Halogen, C₁-C₁₀-Alkyl, C₃-C₁₀-Cycloalkyl, Silyl, C₆-C₃₀-Arylamino, C₆-C₃₀-Aryl und C₂-C₃₀-Heteroaryl, und die substituierte Gruppe unabhängig mit dem verbundenen aromatischen Ring oder heteroaromatischen Ring verbunden ist, um einen Ring auszubilden, oder die substituierte Gruppe unabhängig mit dem verbundenen aromatischen Ring oder heteroaromatischen Ring verbunden ist, um keinen Ring auszubilden;
wobei das Triplett-Triplett-Wirtsmaterial des Annihilationstyps aus mindestens einer der Verbindungen ausgewählt ist, dargestellt durch BFH-18 bis BFH-25:

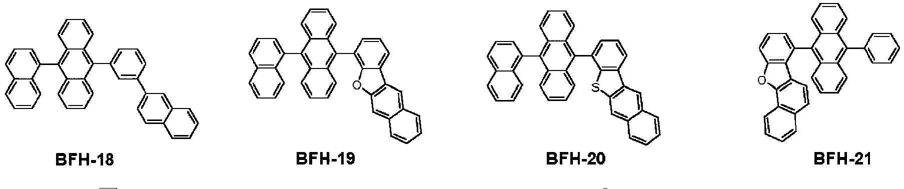

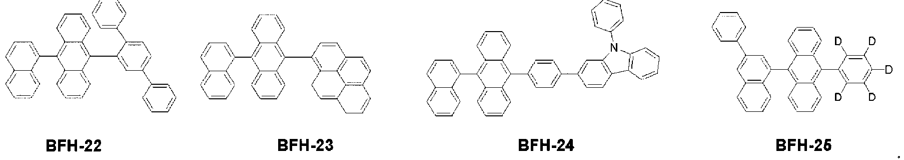

2. Organische Elektrolumineszenzvorrichtung nach Anspruch 1, wobei A eine substituierte Gruppe darstellt, die durch eine beliebige der folgenden (3-1), (3-2), (3-3), (3-4), (3-5), (3-6), (3-7), (3-8) oder (3-9) dargestellt wird: 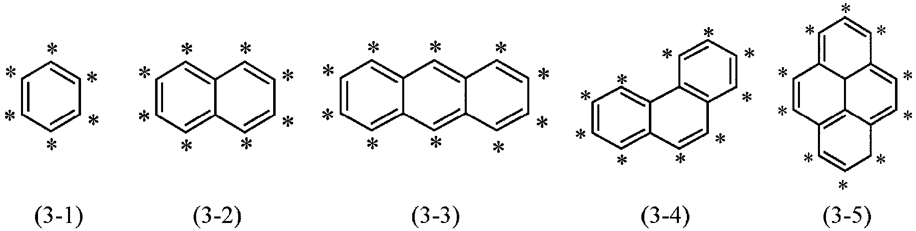 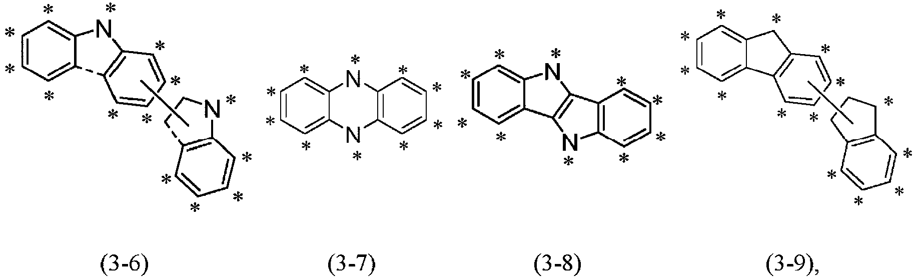
wobei ein Sternchen in den Formeln eine verbindbare Stelle bezeichnet, diese Verbindung eine Verbindung zu einem Stammkern und/oder eine Verbindung mit einer substituierten Gruppe bezeichnet; ein Ausdruck einer Ringstruktur, die durch "-" durchgezogen ist, eine beliebige Position auf der Ringstruktur bezeichnet, an der die verbindbare Stelle eine Bindung ausbilden kann; eine gestrichelte Linie in den Formeln eine Verbindung oder keine Verbindung darstellt;
wobei eine substituierte Gruppe in A eine oder eine Kombination von mindestens zwei ist, ausgewählt aus Deuterium, Tritium, Cyano, Halogen, C₁-C₁₀-Alkyl, C₃-C₁₀-Cycloalkyl, C₆-C₃₀-Arylamino, C₆-C₃₀-Aryl und C₂-C₃₀-Heteroaryl, und die substituierte Gruppe unabhängig mit einem verbundenen aromatischen Ring oder heteroaromatischen Ring verbunden ist, um einen Ring auszubilden, oder die substituierte Gruppe unabhängig mit einem verbundenen aromatischen Ring oder heteroaromatischen Ring verbunden ist, um keinen Ring auszubilden.

3. Organische Elektrolumineszenzvorrichtung nach Anspruch 1 oder 2, wobei A durch eine beliebige der folgenden Strukturformeln dargestellt wird: 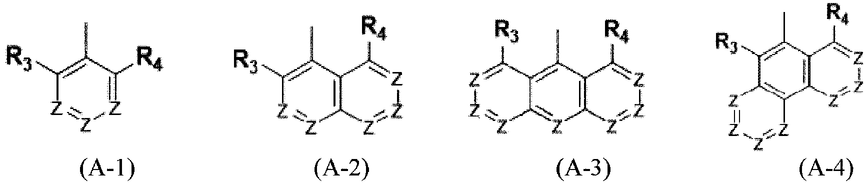 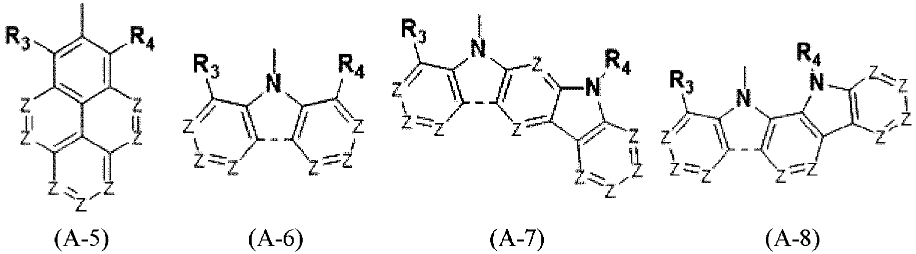 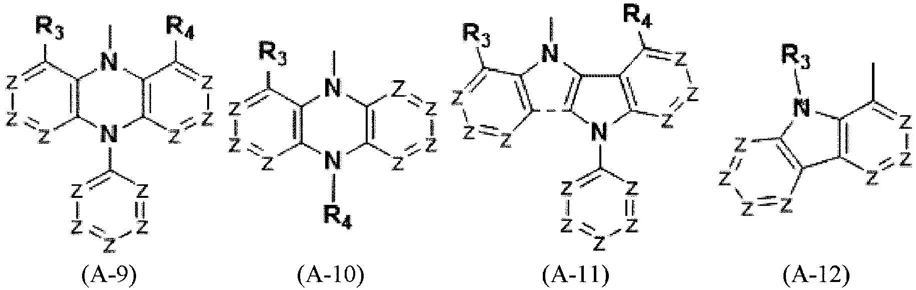
wobei R₃ und R₄ jeweils unabhängig dargestellt sind als eines von Wasserstoff, Deuterium, Tritium, einem substituierten oder unsubstituierten C₁-C₃₀-Alkyl, einem substituierten oder unsubstituierten C₃-C₃₀-Cycloalkyl, einem Silyl, einem substituierten oder unsubstituierten C₁-C₃₀-Alkoxy, einem substituierten oder unsubstituierten C₆-C₆₀-Aryloxy, einem substituierten oder unsubstituierten C₆-C₆₀-Arylamino, einem substituierten oder unsubstituierten C₆-C₆₀-Aryl und einem substituierten oder unsubstituierten C₂-C₆₀-Heteroaryl;
Z unabhängig als N oder CR₅ dargestellt ist, wobei R₅ jedes Mal gleich oder unterschiedlich ist; R₅ dargestellt ist als eines von Wasserstoff, Deuterium, Tritium, Cyano, Halogen, einem substituierten oder unsubstituierten C₁-C₁₀-Alkyl, einem substituierten oder unsubstituierten C₃-C₁₀-Cycloalkyl, einem substituierten oder unsubstituierten C₁-C₁₀-Alkoxy, einem substituierten oder unsubstituierten C₆-C₃₀-Aryloxy, einem substituierten oder unsubstituierten C₆-C₃₀-Arylamino, einem substituierten oder unsubstituierten C₆-C₃₀-Aryl oder einem substituierten oder unsubstituierten C₂-C₃₀-Heteroaryl;
eine substituierte Gruppe in jedem substituierten R₃, R₄ und R₅ eines oder eine Kombination von mindestens zwei ist, ausgewählt aus Deuterium, Tritium, Cyano, Halogen, C₁-C₁₀-Alkyl, C₃-C₁₀-Cycloalkyl, Silyl, C₆-C₃₀-Arylamino, C₆-C₃₀-Aryl und C₂-C₃₀-Heteroaryl, das unabhängig mit dem verbundenen aromatischen Ring oder heteroaromatischen Ring verbunden ist, um einen Ring auszubilden, oder unabhängig mit dem verbundenen aromatischen Ring oder heteroaromatischen Ring verbunden ist, um keinen Ring auszubilden.

4. Organische Elektrolumineszenzvorrichtung nach Anspruch 3, wobei die R₃ und R₄ jeweils unabhängig als ein beliebiges von einem substituierten oder unsubstituierten C₁-C₃₀-Alkyl, einem substituierten oder unsubstituierten C₃-C₃₀Cycloalkyl, einem substituierten oder unsubstituierten C₆-C₆₀-Aryl und einem substituierten oder unsubstituierten C₂-C₆₀-Heteroaryl dargestellt sind.

5. Organische Elektrolumineszenzvorrichtung nach Anspruch 4, wobei mindestens eines der R₃ und R₄ ausgewählt ist aus einer der folgenden Gruppen mit großer sterischer Wirkung: Terphenyl, Trimericphenyl, Tetraphenyl, Fluorenyl, Spirodifluorenyl, Dihydrophenanthryl, Dihydropyrenyl, Tetrahydropyrenyl, cis- oder trans-Indenofluorenyl, Trimericindenyl, Isotrimericindenyl, Spiro-trimericindenyl, Spiro-isotrimericindenyl, Furyl, Benzofuryl, Isobenzofuryl, Dibenzofuryl, Benzothienyl, Isobenzothienyl, Dibenzothienyl, Isoindolyl, Carbazolyl, Indenocarbazolyl, Isoquinolyl, Acridinyl, Phenanthridinyl, Benzo-5,6-quinolyl, Benzo-6,7-quinolyl, Benzo-7,8-quinolyl, Benzimidazolyl, Naphthoimidazolyl, Phenanthroimidazolyl, Pyridinoimidazolyl, Pyrazinoimidazolyl, Quinoxalinoimidazolyl, Oxazolyl, Benzo-oxazolyl, Naphtho-oxazolyl, Anthro-oxazolyl, Phenanthro-oxazolyl, 1,2-Thiazolyl, 1,3-Thiazolyl, Benzothiazolyl, Pyridazinyl, Benzopyridazinyl, 1,5-Diazaanthryl, 2,7-Diazapyrenyl, 2,3-Diazapyrenyl, 1,6-Diazapyrenyl, 1,8-Diazapyrenyl, 4,5-Diazapyrenyl, 4,5,9,10-Tetraazaperylenyl, Phenazinyl, Phenothiazinyl, Azacarbazolyl, Benzocarbolinyl, Phenanthrolinyl, 1,2,3-Triazolyl, 1,2,4-Triazolyl, Benzotriazolyl, 1,2,3-Oxadiazolyl, 1,2,4-Oxadiazolyl, 1,2,5-Oxadiazolyl, 1,2,3-Thiadiazolyl, 1,2,4-Thiadiazolyl, 1,2,5-Thiadiazolyl, 1,3,4-Thiadiazolyl, 1,3,5-Triazinyl, 1,2,4-Triazinyl, 1,2,3-Triazinyl, Tetrazolyl, 1,2,4,5-Tetrazinyl, 1,2,3,4-Tetrazinyl, 1,2,3,5-Tetrazinyl, Purinyl, Pteridyl, Indozinyl, Benzothiadiazolyl, 9,9-Dimethylacridinyl, Diphenylamindo, Adamantyl, Fluorphenyl, Methylphenyl, Trimethylphenyl, Cyanophenyl, Silyl; oder mindestens einer von R₃ oder R₄ eine Kombination ist, die durch mindestens zwei Gruppen ausgebildet wird, die aus den vorstehend erwähnten Gruppen mit großer sterischer Wirkung ausgewählt sind.

6. Organische Elektrolumineszenzvorrichtung nach einem der Ansprüche 1 bis 5, wobei das R eines bezeichnet von Wasserstoff, Deuterium, Tritium, Fluoratom, Cyano, Methyl, deuteriertem Methyl, tritiiertem Methyl, Ethyl, deuteriertem Ethyl, tritiiertem Ethyl, Isopropyl, deuteriertem Isopropyl, tritiiertem Isopropyl, tert-Butyl, deuteriertem tert-Butyl, tritiiertem tert-Butyl, deuteriertem Cyclopentyl, tritiiertem Cyclopentyl, Cyclohexyl, Cyclopentyl, Adamantyl, Phenyl, deuteriertem Phenyl, tritiiertem Phenyl, Biphenyl, deuteriertem Biphenyl, tritiiertem Biphenyl, deuteriertem Terphenyl, tritiiertem Terphenyl, Terphenyl, Naphthyl, Anthryl, Phenanthryl, Pyridyl, Chinolyl, Furyl, Thienyl, Dibenzofuryl, Dibenzothienyl, Carbazolyl, N-Phenylcarbazolyl, 9,9-Dimethylfluorenyl, 9,9-Diphenylfluorenyl, Spirofluorenyl, methylsubstituiertem Phenyl, ethylsubstituiertem Phenyl, isopropylsubstituiertem Phenyl, tert-butylsubstituiertem Phenyl, methylsubstituiertem Biphenyl, ethylsubstituiertem Biphenyl, isopropylsubstituiertem Biphenyl, tert-butylsubstituiertem Biphenyl, deuteriertem methylsubstituiertem Phenyl, deuteriertem ethylsubstituiertem Phenyl, deuteriertem isopropylsubstituiertem Phenyl, deuteriertem tert-butylsubstituiertem Phenyl, deuteriertem methylsubstituiertem Biphenyl, deuteriertem ethylsubstituiertem Biphenyl, deuteriertem isopropylsubstituiertem Biphenyl, deuteriertem tert-butylsubstituiertem Diphenyl, tritiiertem methylsubstituiertem Phenyl, tritiiertem ethylsubstituiertem Phenyl, tritiiertem isopropylsubstituiertem Phenyl, tritiiertem tert-butylsubstituiertem Phenyl, tritiiertem methylsubstituiertem Diphenyl, tritiiertem ethylsubstituiertem Diphenyl, tritiiertem isopropylsubstituiertem Diphenyl, tritiiertem tert-butylsubstituiertem Diphenyl, Diphenylamido, Di-biphenylamido und Triphenylamino;
wobei R₁ eines darstellt von Methyl, deuteriertem Methyl, tritiiertem Methyl, Ethyl, deuteriertem Ethyl, tritiiertem Ethyl, Isopropyl, deuteriertem Isopropyl, tritiiertem Isopropyl, tert-Butyl, deuteriertem tert-Butyl, tritiiertem tert-Butyl, deuteriertem Cyclopentyl, tritiiertem Cyclopentyl, Cyclopentyl, Adamantyl, Phenyl, deuteriertem Phenyl, tritiiertem Phenyl, Biphenyl, deuteriertem Biphenyl, tritiiertem Biphenyl, deuteriertem Terphenyl, tritiiertem Terphenyl, Terphenyl, Naphthyl, Anthryl, Phenanthryl, Pyridyl, Chinolyl, Furyl, Thienyl, Dibenzofuryl, Dibenzothienyl, Carbazolyl, N-Phenylcarbazolyl, 9,9-Dimethylfluorenyl, 9,9-Diphenylfluorenyl, Spirofluorenyl, methylsubstituiertem Phenyl, ethylsubstituiertem Phenyl, isopropylsubstituiertem Phenyl, tert-butylsubstituiertem Phenyl, methylsubstituiertem Diphenyl, ethylsubstituiertem Diphenyl, isopropylsubstituiertem Diphenyl, tert-butylsubstituiertem Diphenyl, deuteriertem methylsubstituiertem Phenyl, deuteriertem ethylsubstituiertem Phenyl, deuteriertem isopropylsubstituiertem Phenyl, deuteriertem tert-butylsubstituiertem Phenyl, deuteriertem methylsubstituiertem Diphenyl, deuteriertem ethylsubstituiertem Diphenyl, deuteriertem isopropylsubstituiertem Diphenyl, deuteriertem tert-butylsubstituiertem Diphenyl, tritiiertem methylsubstituiertem Phenyl, tritiiertem ethylsubstituiertem Phenyl, tritiiertem isopropylsubstituiertem Phenyl, tritiiertem tert-butylsubstituiertem Phenyl, tritiiertem methylsubstituiertem Diphenyl, tritiiertem ethylsubstituiertem Diphenyl, tritiiertem isopropylsubstituiertem Diphenyl und tritiiertem tert-butylsubstituiertem Diphenyl;
wobei das R₂ eines darstellt von Phenyl, deuteriertem Phenyl, tritiiertem Phenyl, Biphenyl, deuteriertem Biphenyl, tritiiertem Biphenyl, deuteriertem Terphenyl, tritiiertem Terphenyl, Terphenyl, Naphthyl, Anthryl, Phenanthryl, Pyridyl, Chinolyl, Dibenzofuryl, Dibenzothienyl, N-Phenylcarbazolyl, methylsubstituiertem Phenyl, Amidin, ethylsubstituiertem Phenyl, isopropylsubstituiertem Phenyl, tert-butylsubstituiertem Phenyl, methylsubstituiertem Biphenyl, ethylsubstituiertem Biphenyl, isopropylsubstituiertem Biphenyl, tert-butylsubstituiertem Biphenyl, deuteriertem methylsubstituiertem Phenyl, deuteriertem ethylsubstituiertem Phenyl, deuteriertem isopropylsubstituiertem Phenyl, deuteriertem tert-butylsubstituiertem Phenyl, deuteriertem methylsubstituiertem Biphenyl, deuteriertem ethylsubstituiertem Biphenyl, deuteriertem isopropylsubstituiertem Biphenyl, deuteriertem tert-butylsubstituiertem Biphenyl, tritiiertem methylsubstituiertem Phenyl, tritiiertem ethylsubstituiertem Phenyl, tritiiertem isopropylsubstituiertem Phenyl, tritiiertem tert-butylsubstituiertem Phenyl, tritiiertem methylsubstituiertem Diphenyl, tritiiertem ethylsubstituiertem Diphenyl, tritiiertem isopropylsubstituiertem Diphenyl und tritiiertem tert-butylsubstituiertem Diphenyl.

7. Organische Elektrolumineszenzvorrichtung nach Anspruch 6, wobei in Formel (1-2), (1-3), (1-6) oder (2-3) die Z₉ und Z₁₀ jeweils CH sind.

8. Organische Elektrolumineszenzvorrichtung nach Anspruch 6, wobei in Formel (1-2), (1-3), (1-6) oder (2-3) die Z₂ und Z₇ jeweils CC(CH₃)₃ sind und Z1, Z₃-Z₆ und Z₈-Z₁₀ jeweils CH sind.

9. Organische Elektrolumineszenzvorrichtung nach Anspruch 1, wobei der Fluoreszenzfarbstoff ausgewählt ist aus einer Verbindung, die durch die folgenden spezifischen Strukturformeln dargestellt ist:

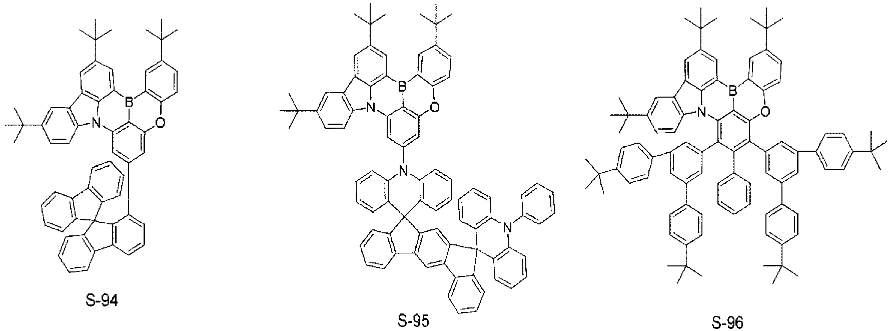

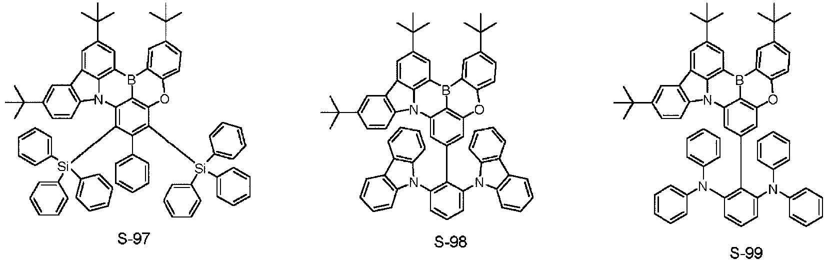

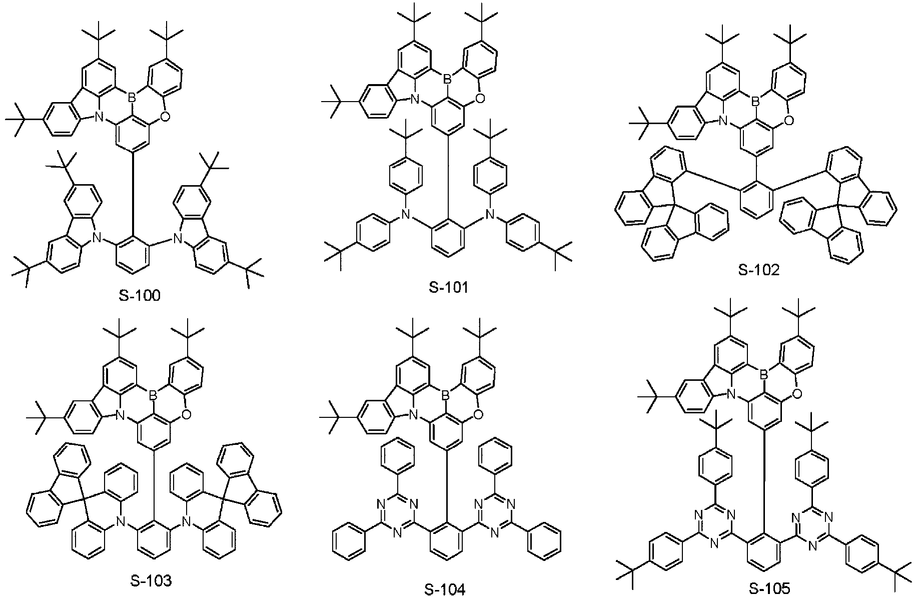

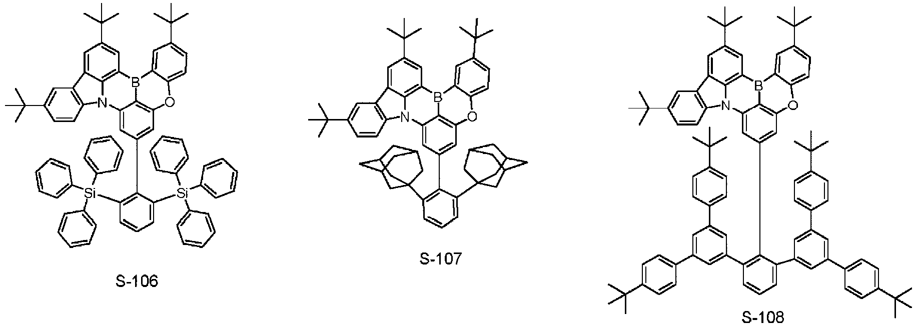

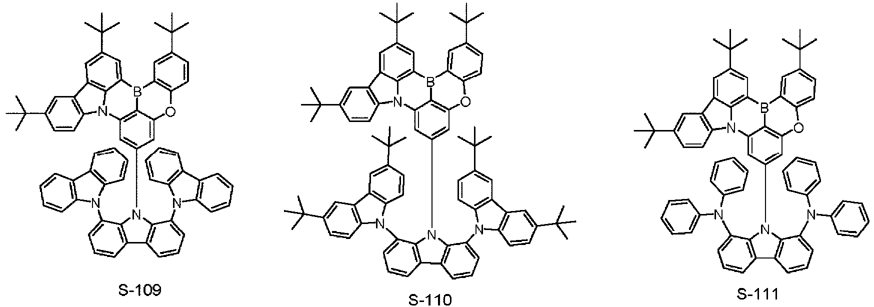

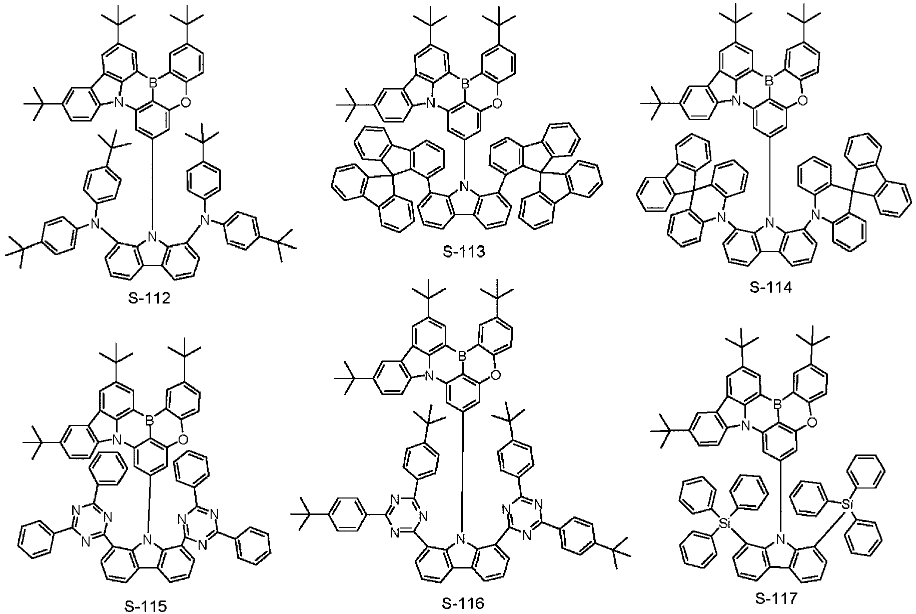

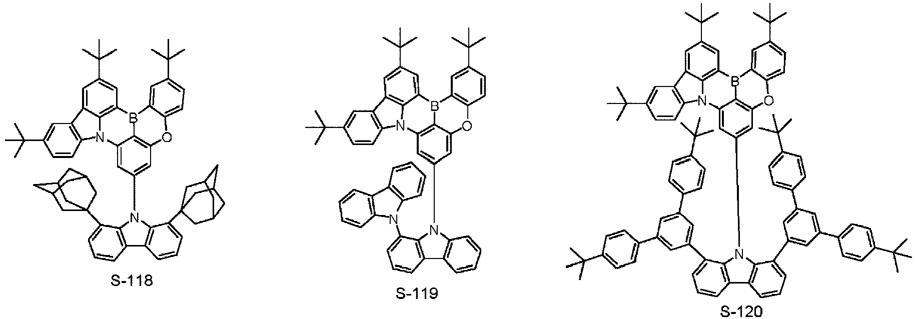

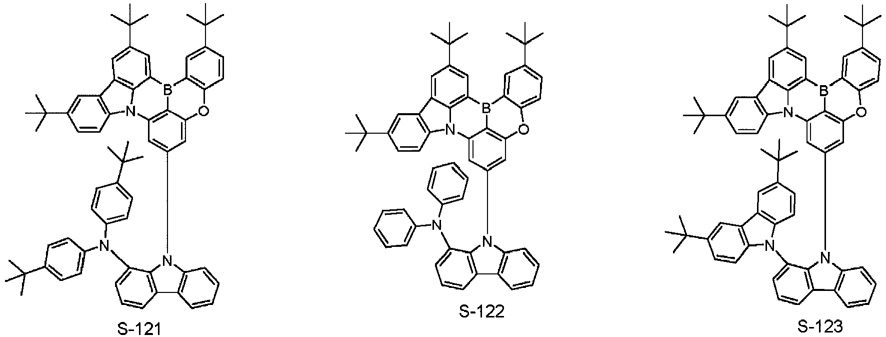

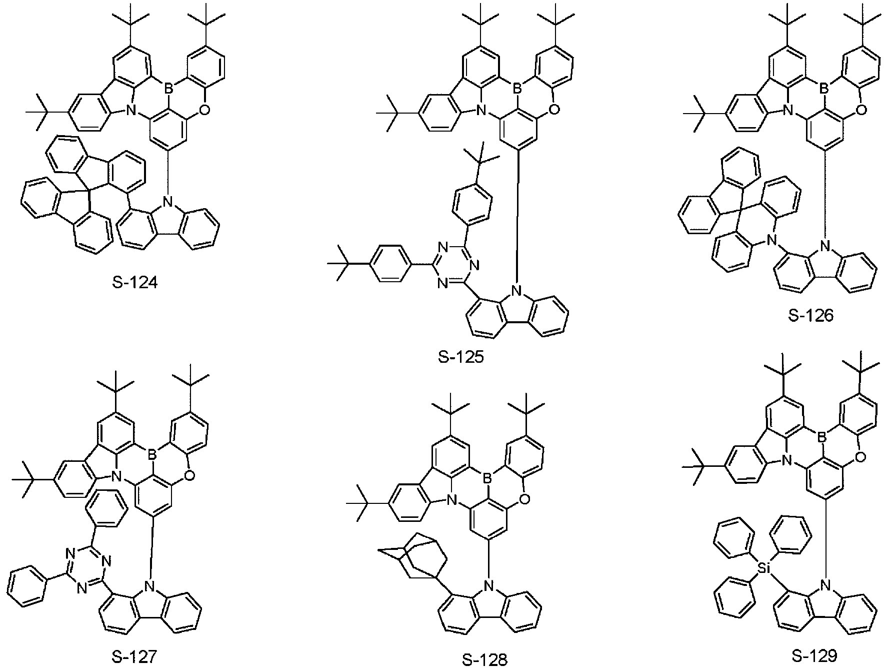

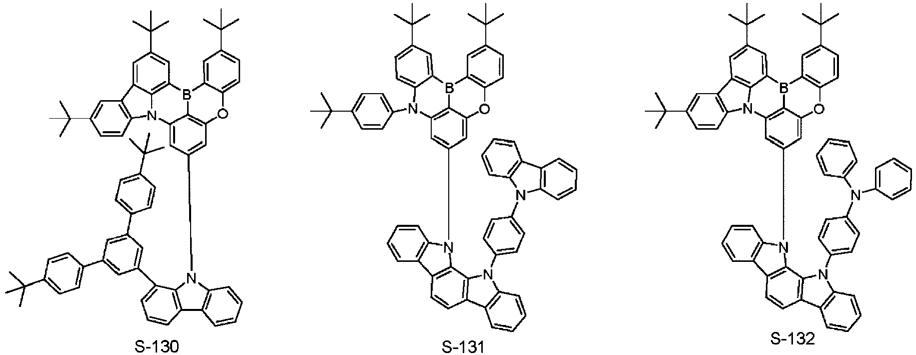

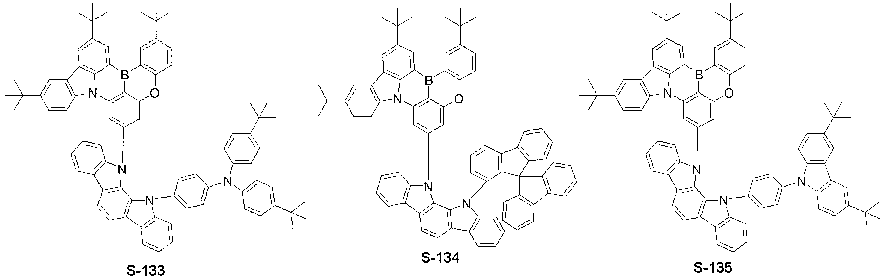

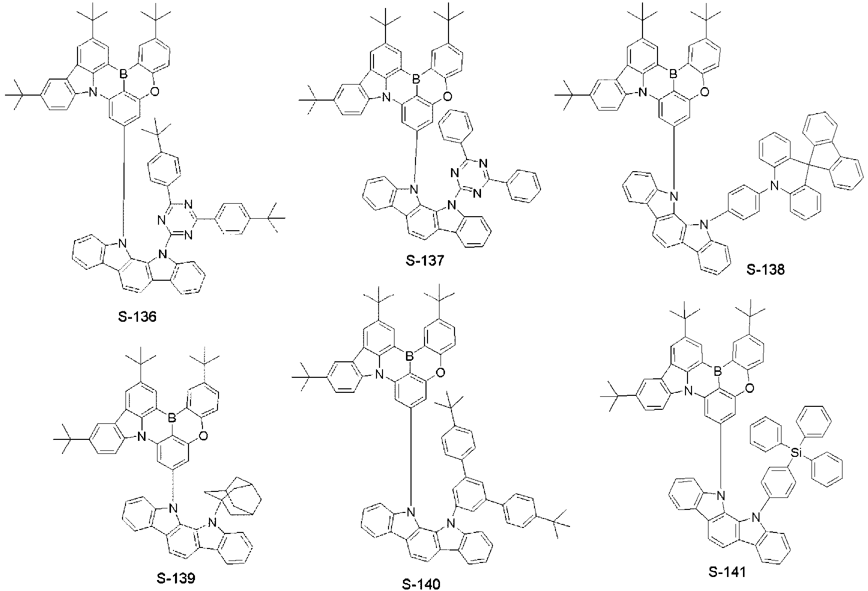

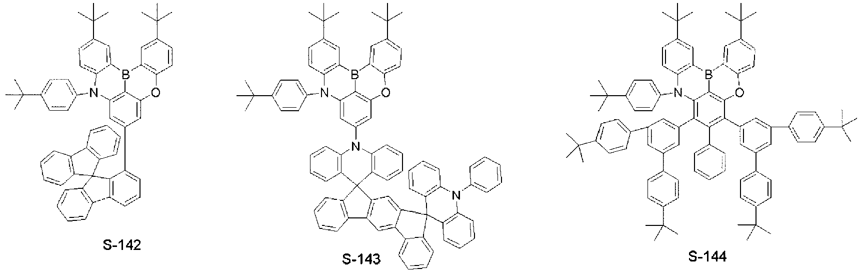

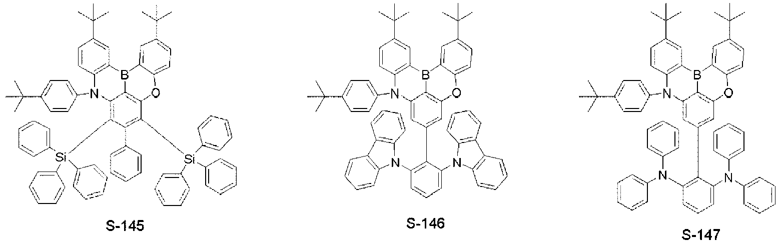

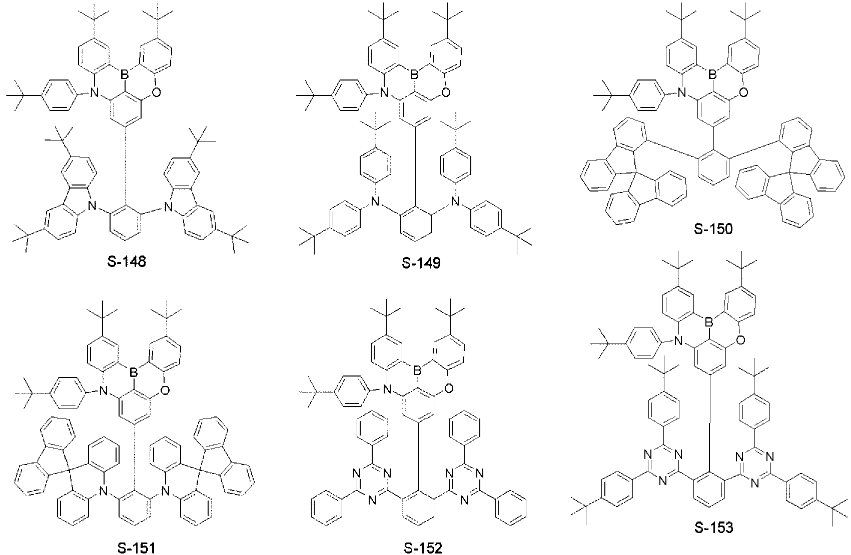

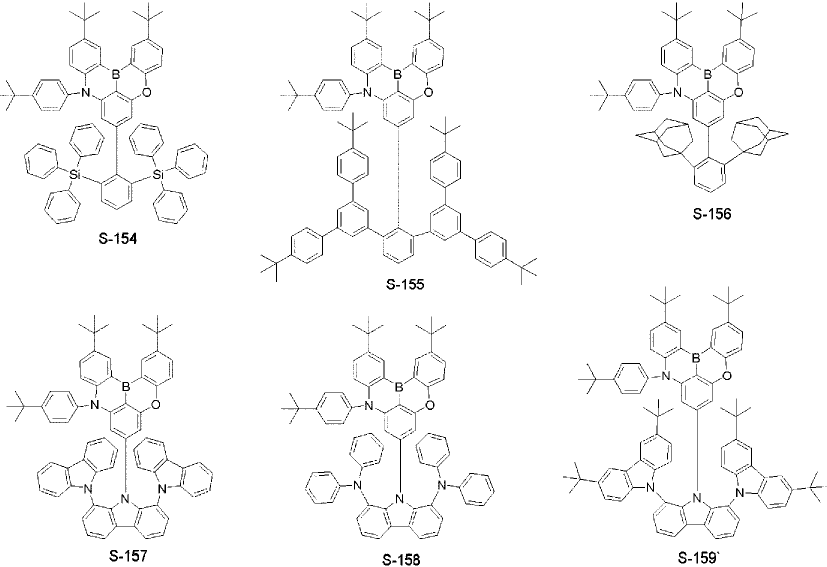

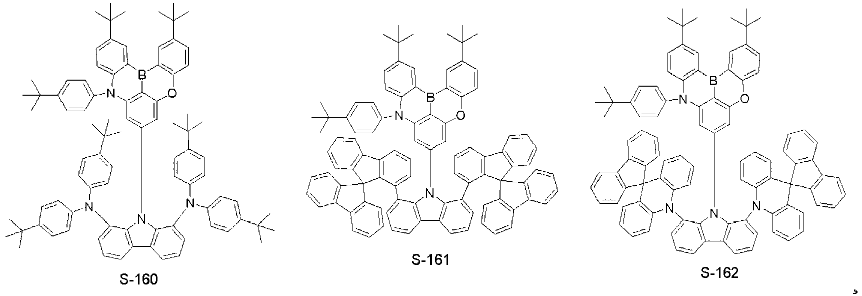

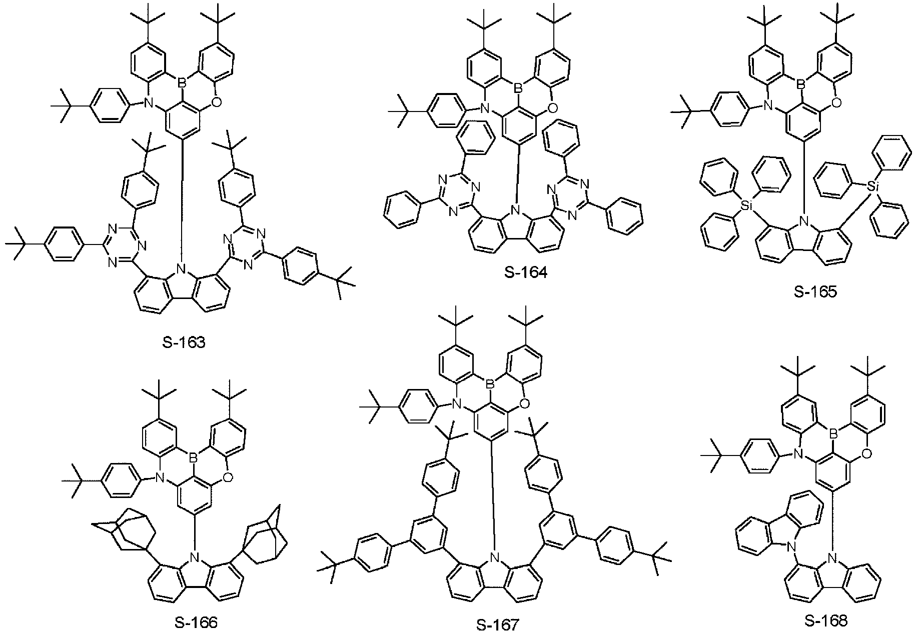

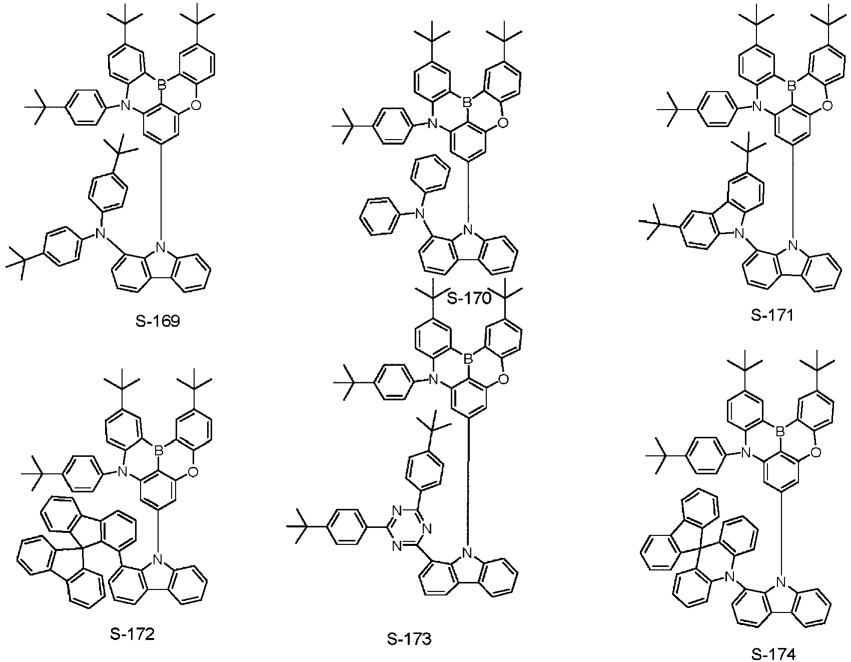

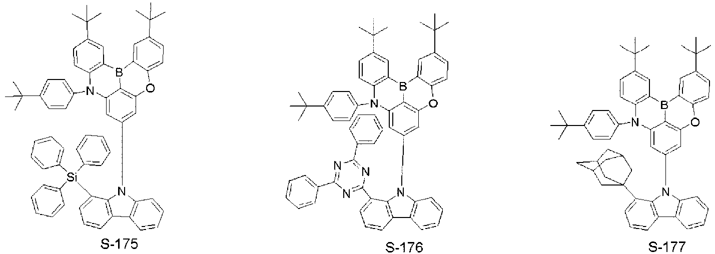

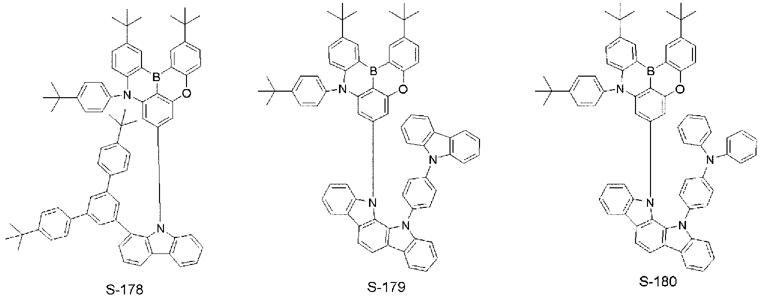

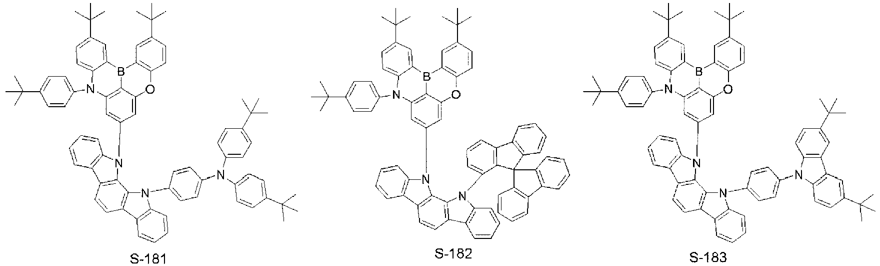

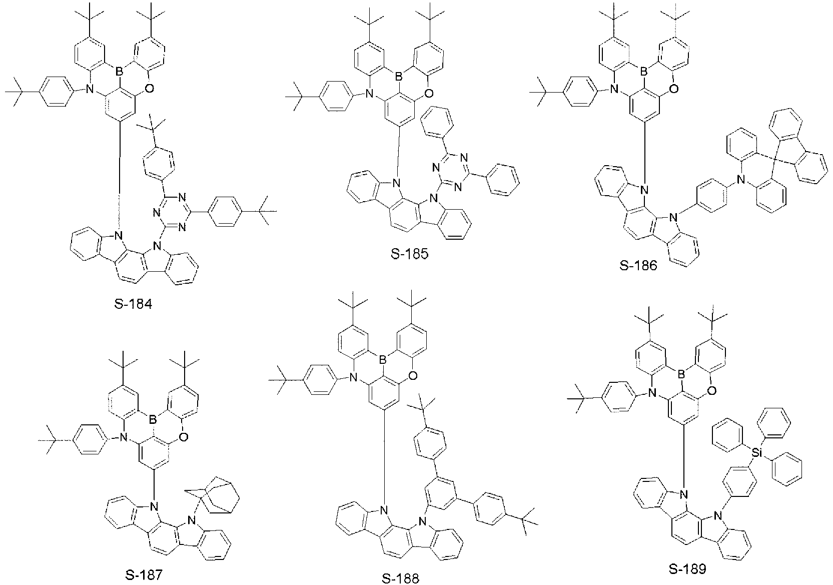

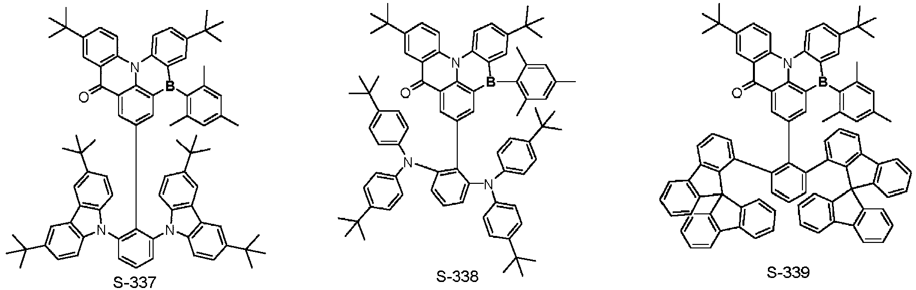

10. Organische Elektrolumineszenzvorrichtung nach einem der Ansprüche 1 bis 9, wobei ein Massenverhältnis des Fluoreszenzfarbstoffs zu der lichtemittierenden Schicht 0,1 %-50 % beträgt.

11. Organische Elektrolumineszenzvorrichtung nach Anspruch 10, wobei das Massenverhältnis des Fluoreszenzfarbstoffs zu der lichtemittierenden Schicht 0,5 %-20 % beträgt.

12. Anzeigeeinrichtung, umfassend die organische Elektrolumineszenzvorrichtung nach einem der Ansprüche 1 bis 11.

## Revendications

1. Dispositif électroluminescent organique comprenant une couche luminescente, la couche luminescente comprend un hôte de type annihilation triplet-triplet et un colorant fluorescent, **caractérisé en ce que** le colorant fluorescent a une structure représentée par la Formule (1-2), (1-3), (1-6) ou (2-3) suivante : 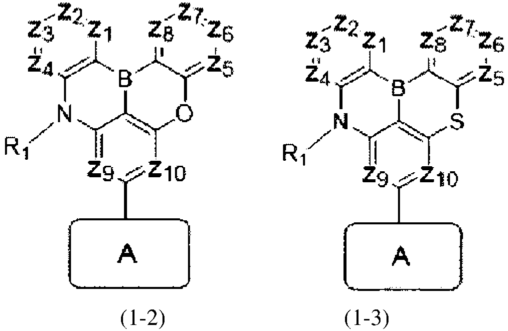 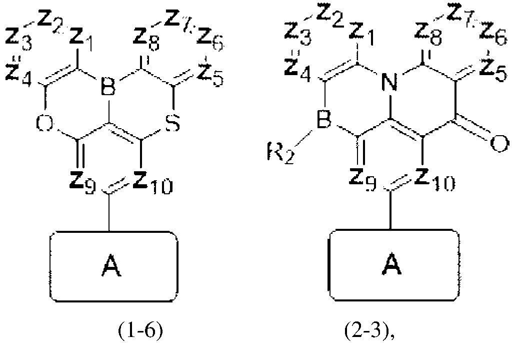
dans la Formule (1-2), (1-3), (1-6) ou (2-3), A représente l'un parmi un groupe carbocyclique en C₆ à C₆₀ substitué ou non substitué, et un hétérocyclyle en C₃ à C₆₀ substitué ou non substitué ; un groupe substitué en A est l'un ou une combinaison d'au moins deux choisis parmi deutérium, tritium, cyano, halogène, alkyle en C₁ à C₁₀, cycloalkyle en C₃ à C₁₀, silyle, arylamino en C₆ à C₃₀, aryle en C₆ à C₃₀, et hétéroaryle en C₂ à C₃₀, et le groupe substitué est indépendamment relié à un cycle aromatique ou cycle hétéroaromatique relié pour former un cycle, ou le groupe substitué est indépendamment relié à un cycle aromatique ou cycle hétéroaromatique relié pour ne pas former de cycle ;
dans la Formule (1-2), (1-3), (1-6) ou (2-3), Z₁ à Z₁₀ sont indépendamment représentés chacun en tant que N ou CR, avec R étant identique ou différent chaque fois, deux R adjacents peuvent se lier l'un à l'autre pour former un cycle ;
R₁ représente l'un parmi un alkyle en C₁ à C₁₀ substitué ou non substitué, un cycloalkyle en C₃ à C₁₀ substitué ou non substitué, un aryle en C₆ à C₃₀ substitué ou non substitué, et un hétéroaryle en C₂ à C₃₀ substitué ou non substitué ;
R₂ représente l'un parmi un aryle en C₆ à C₃₀ substitué ou non substitué, et un hétéroaryle en C₂ à C₃₀ substitué ou non substitué ;
R représente l'un parmi hydrogène, deutérium, tritium, cyano, halogène, un alkyle en C₁ à C₁₀ substitué ou non substitué, un cycloalkyle en C₃ à C₁₀ substitué ou non substitué, un alcoxy en C₁ à C₁₀ substitué ou non substitué, un aryloxy en C₆ à C₃₀ substitué ou non substitué, un arylamino en C₆ à C₃₀ substitué ou non substitué, un aryle en C₆ à C₃₀ substitué ou non substitué, et un hétéroaryle en C₂ à C₃₀ substitué ou non substitué ;
un groupe substitué dans chacun des R₁, R₂ et R substitués est l'un ou une combinaison d'au moins deux choisis parmi deutérium, tritium, cyano, halogène, alkyle en C₁ à C₁₀, cycloalkyle en C₃ à C₁₀, silyle, arylamino en C₆ à C₃₀, aryle en C₆ à C₃₀, et hétéroaryle en C₂ à C₃₀, et le groupe substitué est indépendamment relié au cycle aromatique ou cycle hétéroaromatique relié pour former un cycle, ou le groupe substitué est indépendamment relié au cycle aromatique ou cycle hétéroaromatique relié pour ne pas former de cycle ;
dans lequel le matériau hôte de type annihilation triplet-triplet est choisi parmi au moins l'un des composés représentés par BFH-18 à BFH-25 :

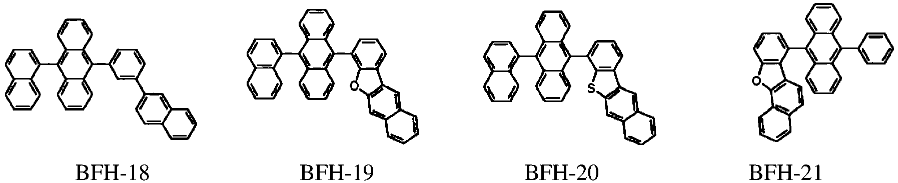

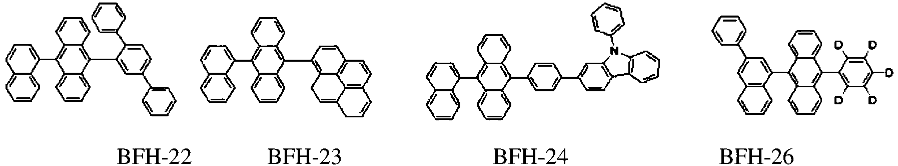

2. Dispositif électroluminescent organique selon la revendication 1, dans lequel A représente un groupe substitué représenté par l'un quelconque parmi les (3-1), (3-2), (3-3), (3-4), (3-5), (3-6), (3-7), (3-8) ou (3-9) suivants : 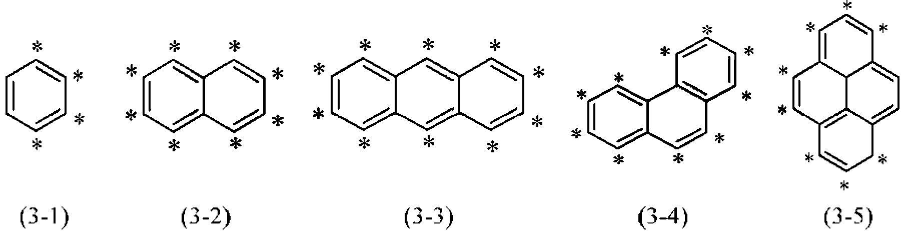 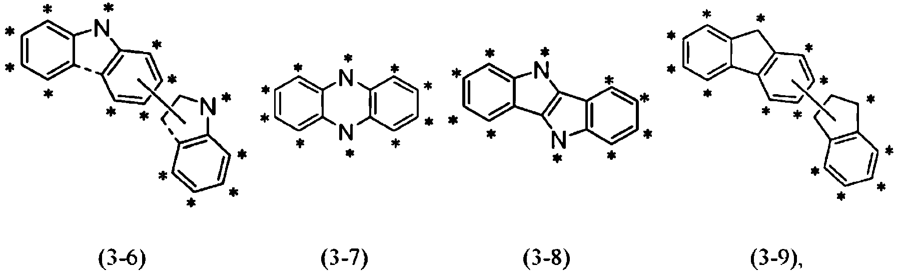
un astérisque dans les Formules désigne un site pouvant être relié, ce lien désigne un lien avec un noyau parent et/ou un lien avec un groupe substitué ; une expression d'une structure cyclique barrée par « - » désigne l'une quelconque position sur la structure cyclique où le site pouvant être relié peut former une liaison ; une ligne discontinue dans les Formules représente le fait d'être relié ou d'être non relié ;
dans lequel un groupe substitué en A est l'un ou une combinaison d'au moins deux choisis parmi deutérium, tritium, cyano, halogène, alkyle en C₁ à C₁₀, cycloalkyle en C₃ à C₁₀, silyle, arylamino en C₆ à C₃₀, aryle en C₆ à C₃₀, et hétéroaryle en C₂ à C₃₀, et le groupe substitué est indépendamment relié à un cycle aromatique ou cycle hétéroaromatique relié pour former un cycle, ou le groupe substitué est indépendamment relié à un cycle aromatique ou cycle hétéroaromatique relié pour ne pas former de cycle.

3. Dispositif électroluminescent organique selon la revendication 1 ou la revendication 2, dans lequel A est représenté par l'une quelconque des formules développées suivantes : 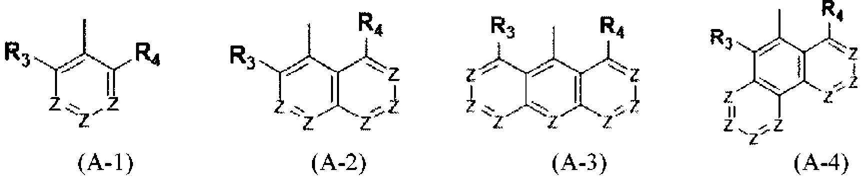 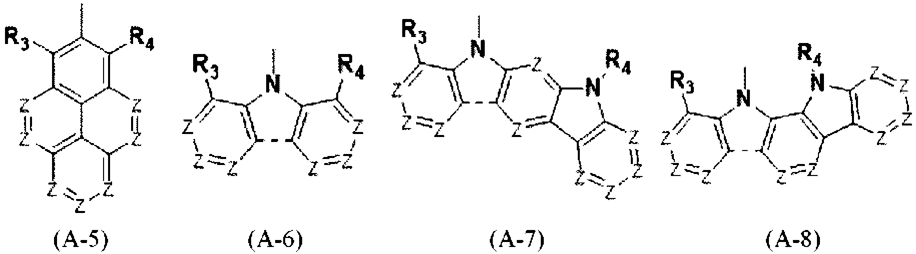 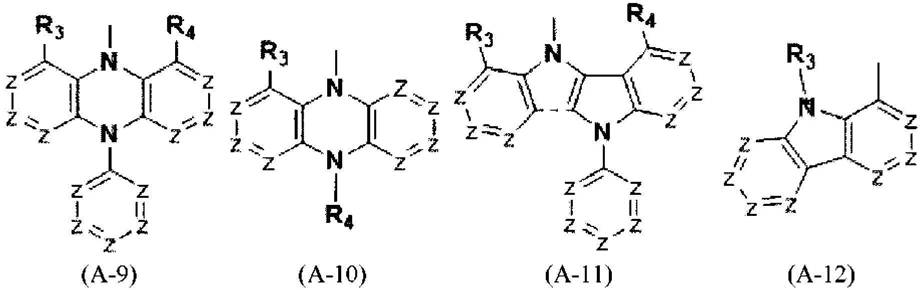
dans lequel, R₃ et R₄ sont indépendamment représentés chacun en tant que l'un quelconque parmi hydrogène, deutérium, tritium, un alkyle en C₁ à C₃₀ substitué ou non substitué, un cycloalkyle en C₃ à C₃₀ substitué ou non substitué, un silyle, un alcoxy en C₁ à C₃₀ substitué ou non substitué, un aryloxy en C₆ à C₆₀ substitué ou non substitué, un arylamino en C₆ à C₆₀ substitué ou non substitué, un aryle en C₆ à C₆₀ substitué ou non substitué, et un hétéroaryle en C₂ à C₆₀ substitué ou non substitué ;
Z est indépendamment représenté en tant que N ou CR₅, avec R₅ étant identique ou différent chaque fois ; R₅ est représenté en tant que l'un parmi hydrogène, deutérium, tritium, cyano, halogène, un alkyle en C₁ à C₁₀ substitué ou non substitué, un cycloalkyle en C₃ à C₁₀ substitué ou non substitué, un alcoxy en C₁ à C₁₀ substitué ou non substitué, un aryloxy en C₆ à C₃₀ substitué ou non substitué, un arylamino en C₆ à C₃₀ substitué ou non substitué, un aryle en C₆ à C₃₀ substitué ou non substitué, ou un hétéroaryle en C₂ à C₃₀ substitué ou non substitué ;
un groupe substitué dans chacun des R₃, R₄ et R₅ substitué est l'un ou une combinaison d'au moins deux choisis parmi deutérium, tritium, cyano, halogène, alkyle en C₁ à C₁₀, cycloalkyle en C₃ à C₁₀, silyle, arylamino en C₆ à C₃₀, aryle en C₆ à C₃₀, et hétéroaryle en C₂ à C₃₀, qui est indépendamment relié au cycle aromatique ou cycle hétéroaromatique relié pour former un cycle, ou est indépendamment relié au cycle aromatique ou cycle hétéroaromatique relié pour ne pas former de cycle.

4. Dispositif électroluminescent organique selon la revendication 3, dans lequel les R₃ et R₄ sont indépendamment représentés chacun en tant que l'un quelconque parmi un alkyle en C₁ à C₃₀ substitué ou non substitué, un cycloalkyle en C₃ à C₃₀ substitué ou non substitué, un aryle en C₆ à C₆₀ substitué ou non substitué, et un hétéroaryle en C₂ à C₆₀ substitué ou non substitué.

5. Dispositif électroluminescent organique selon la revendication 4, dans lequel au moins l'un parmi R₃ et R₄ est choisi parmi l'un des groupes suivants avec un effet stérique important : terphényle, trimèrephényle, tétraphényle, fluorényle, spirodifluorényle, dihydrophénanthryle, dihydropyrényle, tétrahydropyrényle, cis ou trans indénofluorényle, trimèreindényle, isotrimère-indényle, spiro-trimère-indényle, spiro-isotrimère-indényle, furyle, benzofuryle, isobenzofuryle, dibenzofuryle, benzothiényle, isobenzothiényle, dibenzothiényle, isoindolyle, carbazolyle, indénocarbazolyle, isoquinolyle, acridinyle, phénanthridinyle, benzo-5,6-quinolyle, benzo-6,7-quinolyle, benzo-7,8-quinolyle, benzimidazolyle, naphto-imidazolyle, phénanthro-imidazolyle, pyridino-imidazolyle, pyrazino-imidazolyle, quinoxalino-imidazolyle, oxazolyle, benzo-oxazolyle, naphto-oxazolyle, anthro-oxazolyle, phénanthro-oxazolyle, 1,2-thiazolyle, 1,3-thiazolyle, benzothiazolyle, pyridazinyle, benzopyridazinyle, 1,5-diaza-anthryle, 2,7-diaza-pyrényle, 2,3-diaza-pyrényle, 1,6-diaza-pyrényle, 1,8-diaza-pyrényle, 4,5-diaza-pyrényle, 4,5,9,10-tétraaza-pérylényle, phénazinyle, phénothiazinyle, azacarbazolyle, benzocarbolinyle, phénanthrolinyle, 1,2,3-triazolyle, 1,2,4-triazolyle, benzotriazolyle, 1,2,3-oxadiazolyle, 1,2,4-oxadiazolyle, 1,2,5-oxadiazolyle, 1,2,3-thiadiazolyle, 1,2,4-thiadiazolyle, 1,2,5-thiadiazolyle, 1,3,4-thiadiazolyle, 1,3,5-triazinyle, 1,2,4-triazinyle, 1,2,3-triazinyle, tétrazolyle, 1,2,4,5-tétrazinyle, 1,2,3,4-tétrazinyle, 1,2,3,5-tétrazinyle, purinyle, ptéridyle, indozinyle, benzothiadiazolyle, 9,9-diméthylacridinyle, diphénylamido, adamantyle, fluorophényle, méthylphényle, triméthylphényle, cyanophényle, silyle ; ou au moins l'un parmi R₃ ou R₄ est une combinaison formée par au moins deux groupes choisis parmi les groupes susmentionnés avec un effet stérique important.

6. Dispositif électroluminescent organique selon l'une quelconque des revendications 1 à 5, dans lequel le R désigne l'un parmi hydrogène, deutérium, tritium, atome de fluor, cyano, méthyle, méthyle deutéré, méthyle tritié, éthyle, éthyle deutéré, éthyle tritié, isopropyle, isopropyle deutéré, isopropyle tritié, tert-butyle, tert-butyle deutéré, tert-butyle tritié, cyclopentyle deutéré, cyclopentyle tritié, cyclohexyle, cyclopentyle, adamantyle, phényle, phényle deutéré, phényle tritié, biphényle, biphényle deutéré, biphényle tritié, terphényle deutéré, terphényle tritié, terphényle, naphtyle, anthryle, phénanthryle, pyridyle, quinolyle, furyle, thiényle, dibenzofuryle, dibenzothiényle, carbazolyle, N-phénylcarbazolyle, 9,9-diméthylfluorényle, 9,9-diphénylfluorényle, spirofluorényle, phényle à substitution méthyle, phényle à substitution éthyle, phényle à substitution isopropyle, phényle à substitution tert-butyle, biphényle à substitution méthyle, biphényle à substitution éthyle, biphényle à substitution isopropyle, biphényle à substitution tert-butyle, phényle à substitution méthyle deutéré, phényle à substitution éthyle deutéré, phényle à substitution isopropyle deutéré, phényle à substitution tert-butyle deutéré, biphényle à substitution méthyle deutéré, biphényle à substitution éthyle deutéré, biphényle à substitution isopropyle deutéré, diphényle à substitution tert-butyle deutéré, phényle à substitution méthyle tritié, phényle à substitution éthyle tritié, phényle à substitution isopropyle tritié, phényle à substitution tert-butyle tritié, diphényle à substitution méthyle tritié, diphényle à substitution éthyle tritié, diphényle à substitution isopropyle tritié, diphényle à substitution tert-butyle tritié, diphénylamido, di-biphénylamido et triphénylamino ;
dans lequel R₁ représente l'un parmi méthyle, méthyle deutéré, méthyle tritié, éthyle, éthyle deutéré, éthyle tritié, isopropyle, isopropyle deutéré, isopropyle tritié, tert-butyle, tert-butyle deutéré, tert-butyle tritié, cyclopentyle deutéré, cyclopentyle tritié, cyclopentyle, adamantyle, phényle, phényle deutéré, phényle tritié, biphényle, biphényle deutéré, biphényle tritié, terphényle deutéré, terphényle tritié, terphényle, naphtyle, anthryle, phénanthryle, pyridyle, quinolyle, furyle, thiényle, dibenzofuryle, dibenzothiényle, carbazolyle, N-phénylcarbazolyle, 9,9-diméthylfluorényle, 9,9-diphénylfluorényle, spirofluorényle, phényle à substitution méthyle, phényle à substitution éthyle, phényle à substitution isopropyle, phényle à substitution tert-butyle, diphényle à substitution méthyle, diphényle à substitution éthyle, diphényle à substitution isopropyle, diphényle à substitution tert-butyle, phényle à substitution méthyle deutéré, phényle à substitution éthyle deutéré, phényle à substitution isopropyle deutéré, phényle à substitution tert-butyle deutéré, diphényle à substitution méthyle deutéré, diphényle à substitution éthyle deutéré, diphényle à substitution isopropyle deutéré, diphényle à substitution tert-butyle deutéré, phényle à substitution méthyle tritié, phényle à substitution éthyle tritié, phényle à substitution isopropyle tritié, phényle à substitution tert-butyle tritié, diphényle à substitution méthyle tritié, diphényle à substitution éthyle tritié, diphényle à substitution isopropyle tritié et diphényle à substitution tert-butyle tritié ;
dans lequel le R₂ représente l'un parmi phényle, phényle deutéré, phényle tritié, biphényle, biphényle deutéré, biphényle tritié, terphényle deutéré, terphényle tritié, terphényle, naphtyle, anthryle, phénanthryle, pyridyle, quinolyle, dibenzofuryle, dibenzothiényle, N-phénylcarbazolyle, phényle à substitution méthyle, amidine, phényle à substitution éthyle, phényle à substitution isopropyle, phényle à substitution tert-butyle, biphényle à substitution méthyle, biphényle à substitution éthyle, biphényle à substitution isopropyle, biphényle à substitution tert-butyle, phényle à substitution méthyle deutéré, phényle à substitution éthyle deutéré, phényle à substitution isopropyle deutéré, phényle à substitution tert-butyle deutéré, biphényle à substitution méthyle deutéré, biphényle à substitution éthyle deutéré, biphényle à substitution isopropyle deutéré, biphényle à substitution tert-butyle deutéré, phényle à substitution méthyle tritié, phényle à substitution éthyle tritié, phényle à substitution isopropyle tritié, phényle à substitution tert-butyle tritié, diphényle à substitution méthyle tritié, diphényle à substitution éthyle tritié, diphényle à substitution isopropyle tritié et diphényle à substitution tert-butyle tritié.

7. Dispositif électroluminescent organique selon la revendication 6, dans lequel dans la Formule (1-2), (1-3), (1-6) ou (2-3), les Z₉ et Z₁₀ sont chacun CH.

8. Dispositif électroluminescent organique selon la revendication 6, dans lequel dans la Formule (1-2), (1-3), (1-6) ou (2-3), les Z₂ et Z₇ sont chacun CC(CH₃)₃, et Z1, Z₃-Z₆, et Z₈-Z₁₀ sont chacun CH.

9. Dispositif électroluminescent organique selon la revendication 1, dans lequel le colorant fluorescent est choisi parmi un composé représenté par les formules développées spécifiques suivantes :

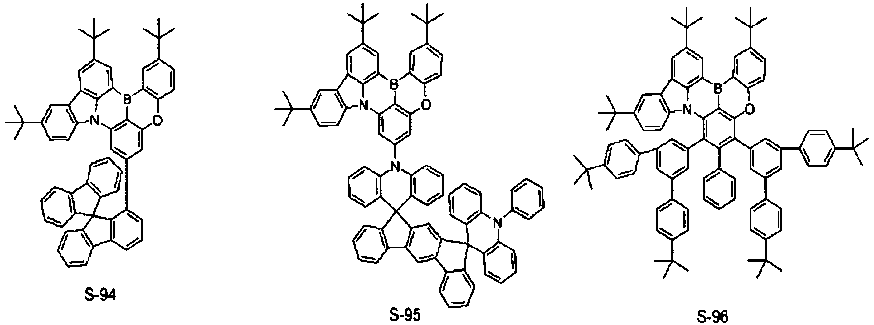

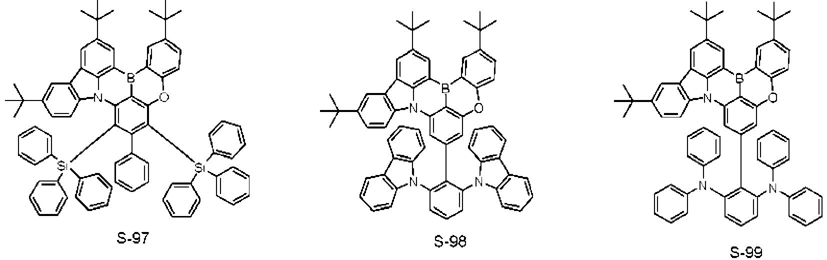

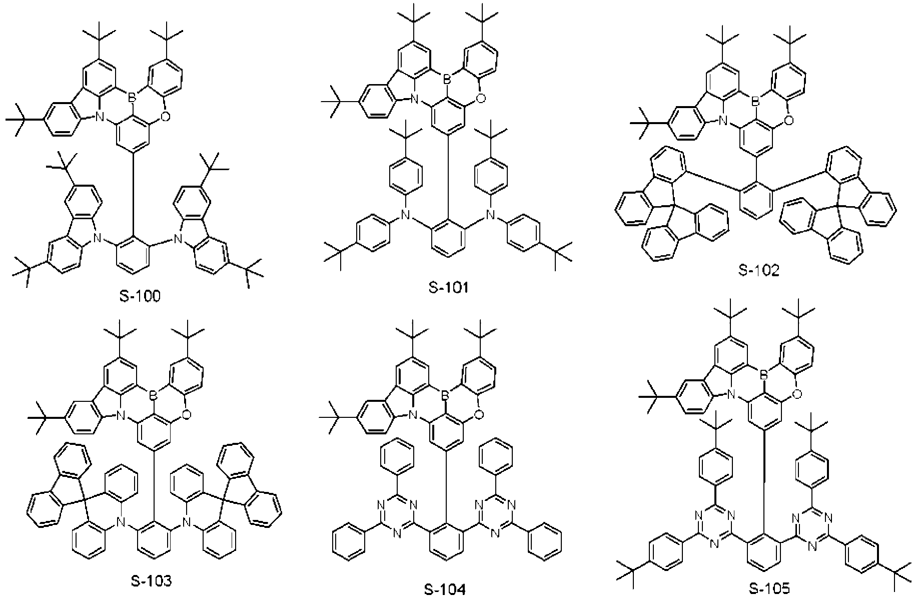

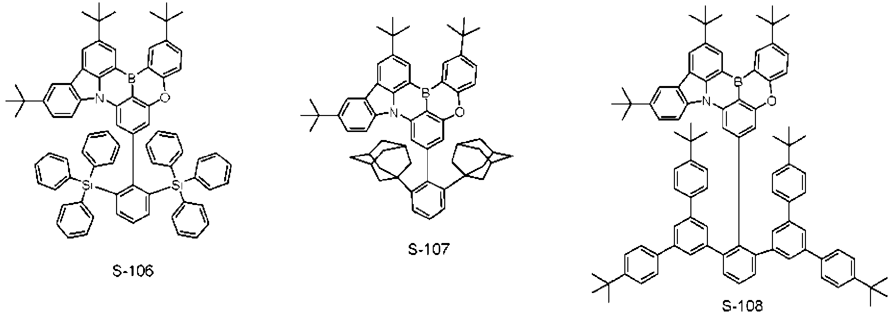

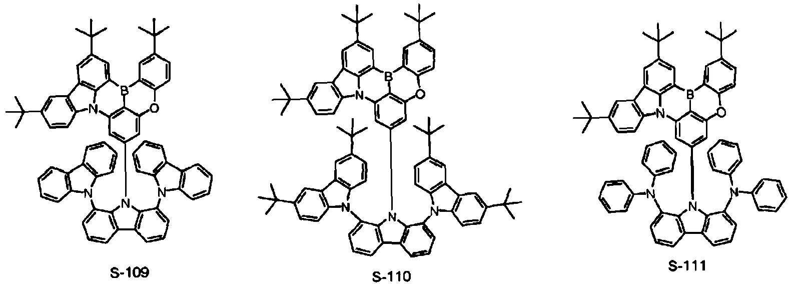

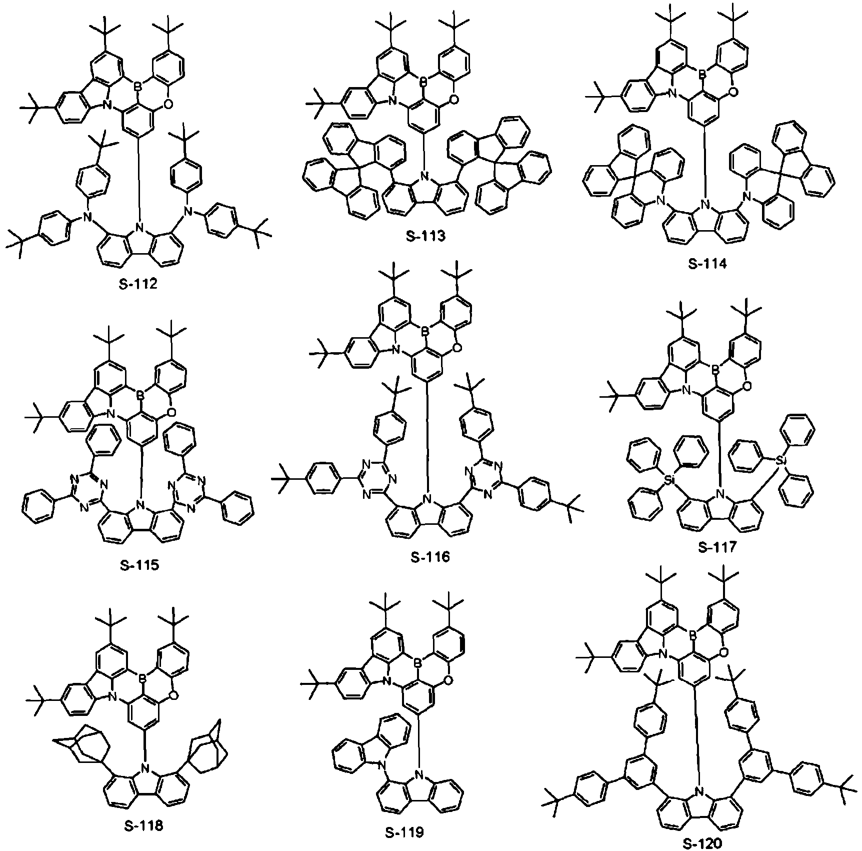

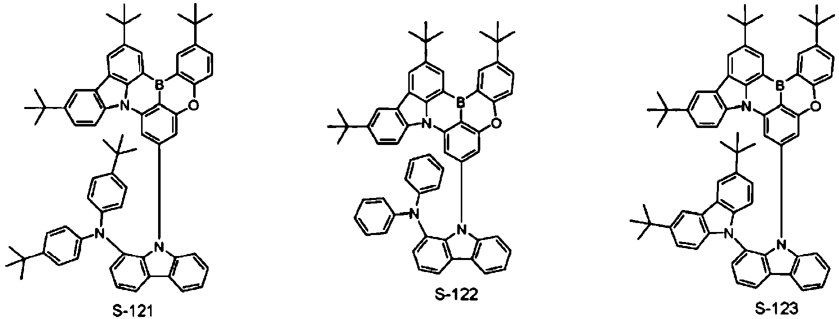

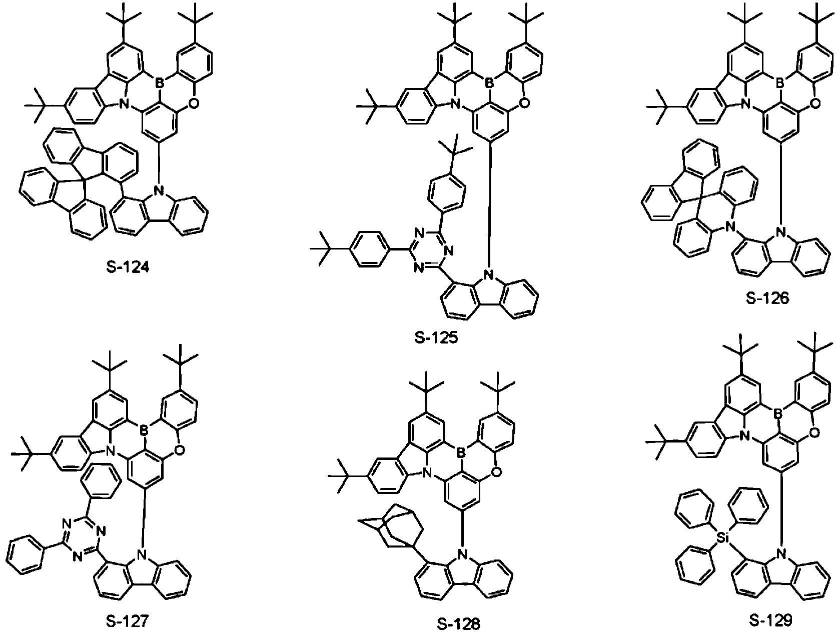

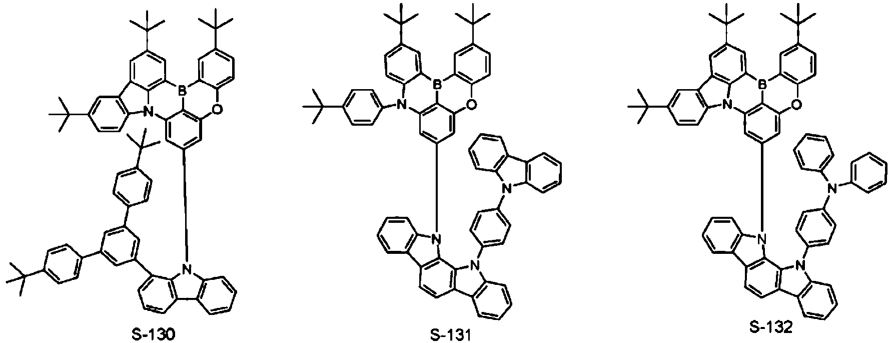

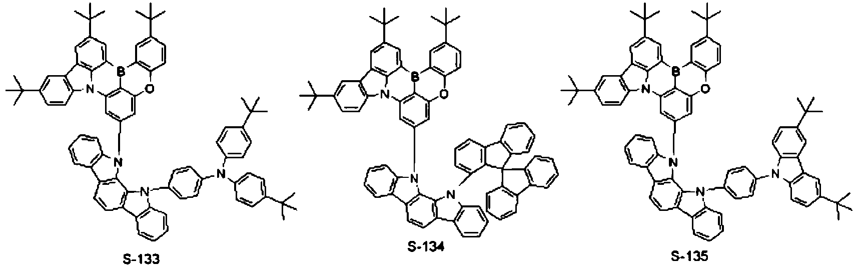

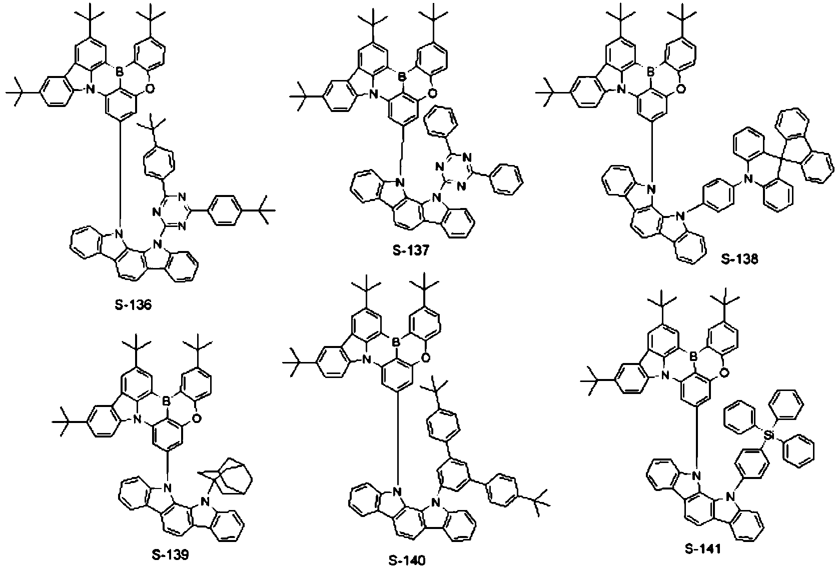

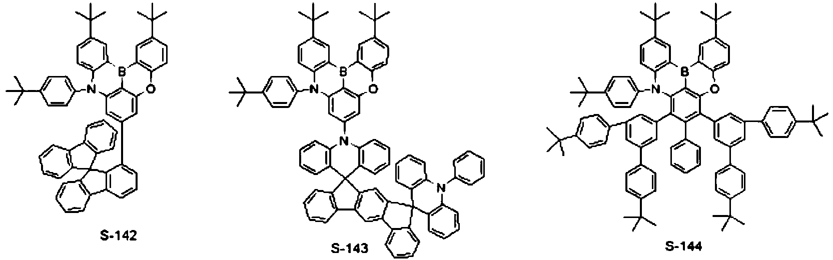

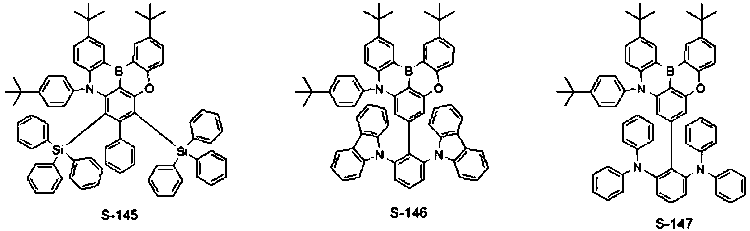

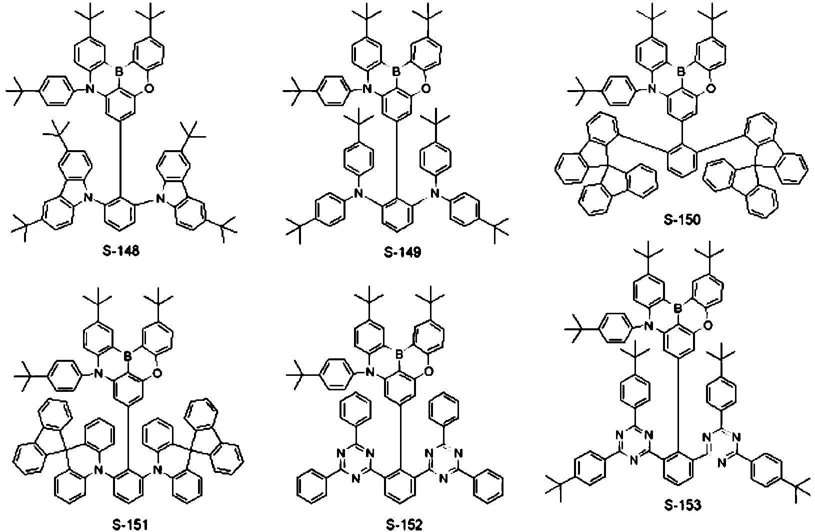

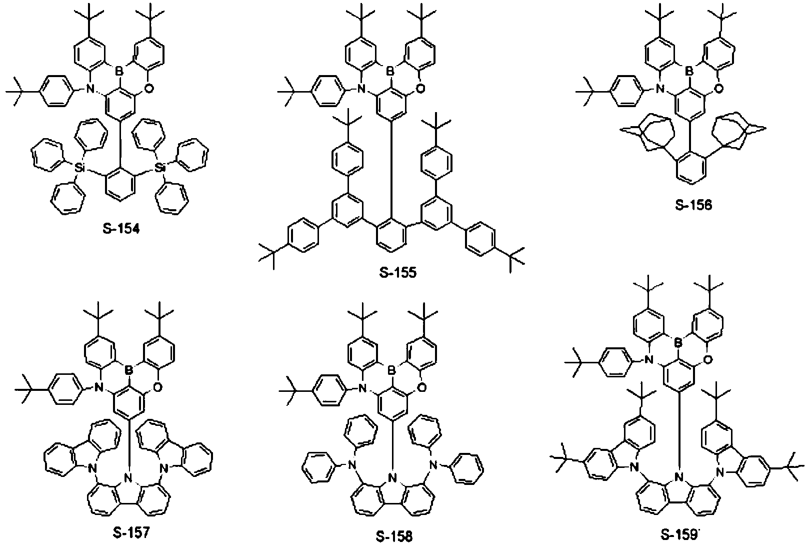

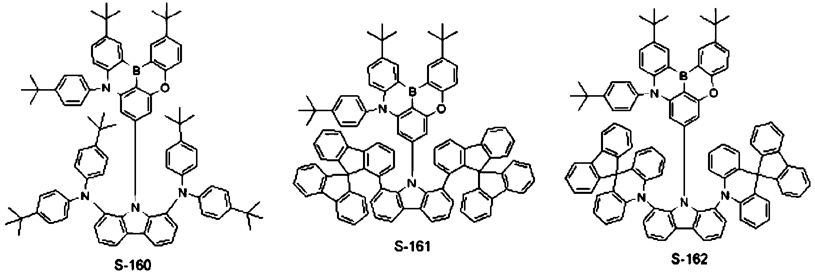

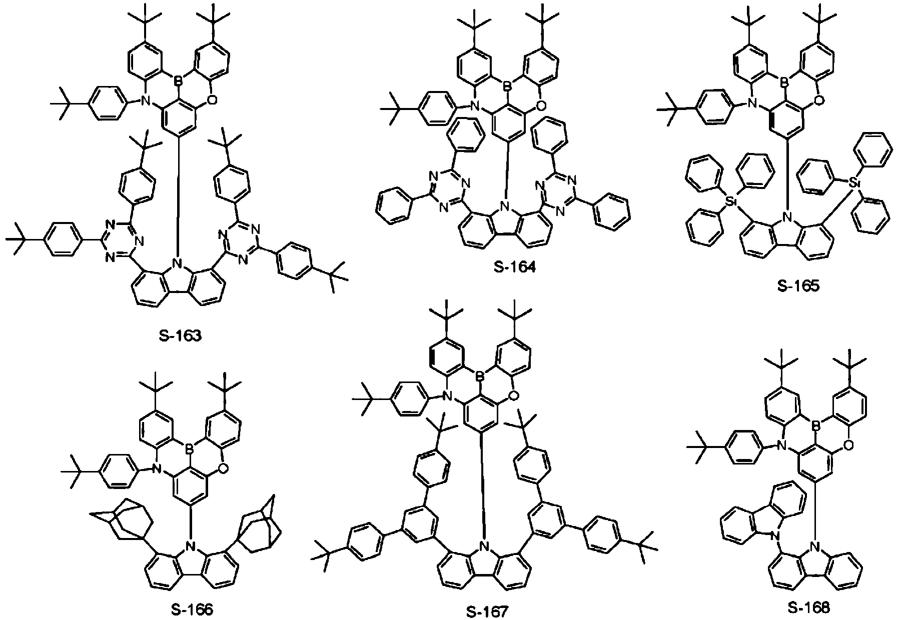

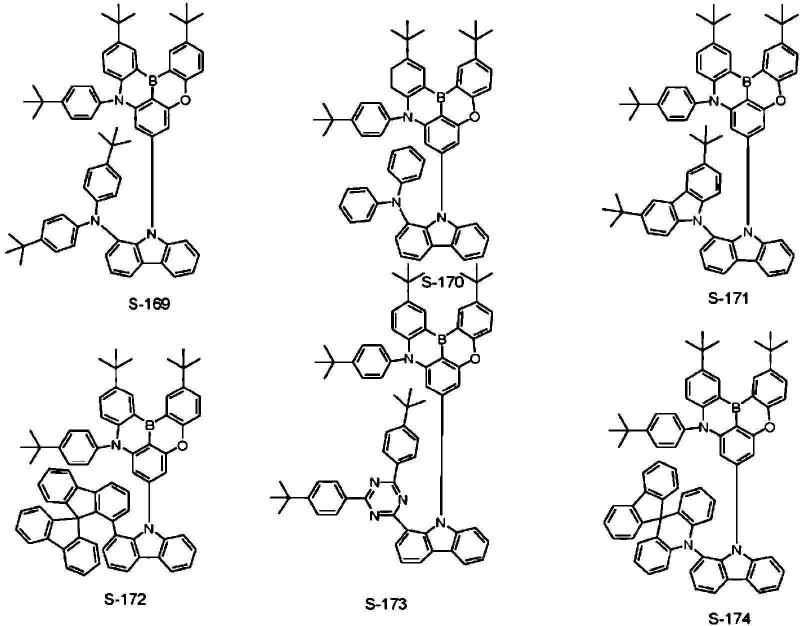

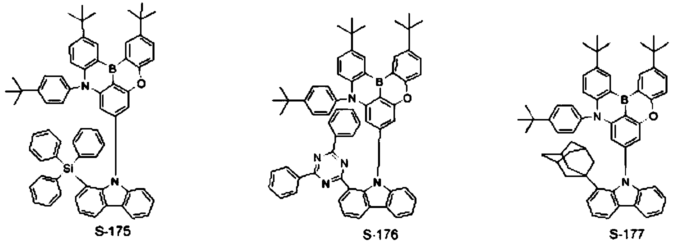

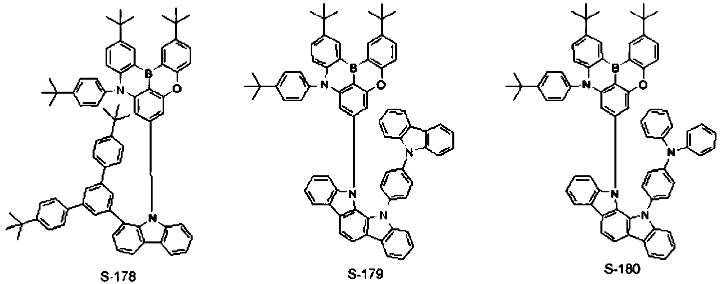

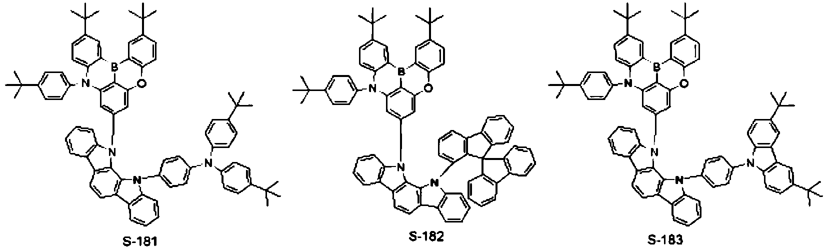

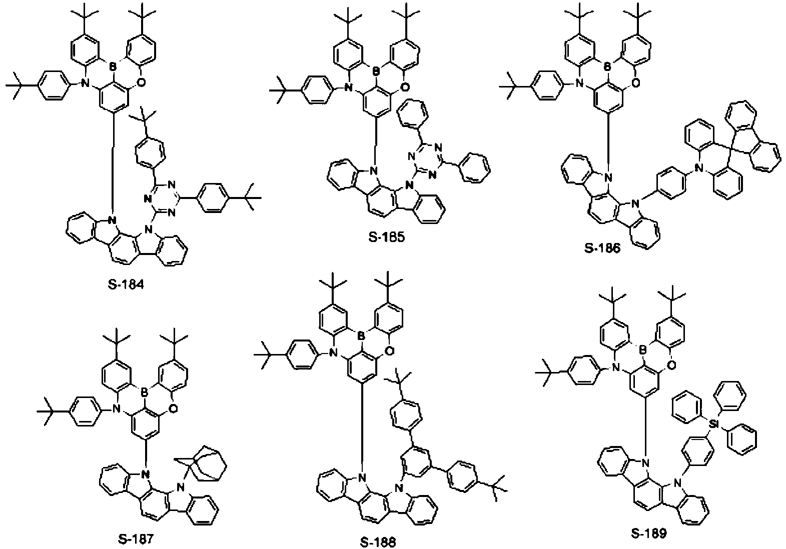

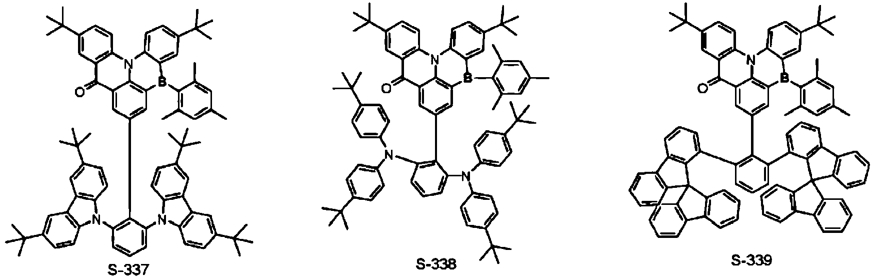

10. Dispositif électroluminescent organique selon l'une quelconque des revendications 1 à 9, dans lequel un rapport massique du colorant fluorescent à la couche luminescente va de 0,1 % à 50 %.

11. Dispositif électroluminescent organique selon la revendication 10, dans lequel le rapport massique du colorant fluorescent à la couche luminescente va de 0,5 % à 20 %.

12. Appareil d'affichage, comprenant le dispositif électroluminescent organique selon l'une quelconque des revendications 1 à 11.
